# EUROPÄISCHE PATENTSCHRIFT

(11) **EP 1 940 809 B1**
(45) Veröffentlichungstag und Bekanntmachung des Hinweises auf die Patenterteilung: **25.12.2013**
(21) Anmeldenummer: 06792405.0
(22) Anmeldetag: 09.10.2006
(51) Int. Cl.: C07D 257/04, A61K 31/41, A61P 9/00

(54) **TETRAZOL-DERIVATE UND IHRE VERWENDUNG ZUR BEHANDLUNG VON HERZ-KREISLAUF-ERKRANKUNGEN**
TETRAZOL DERIVATIVES AND THEIR USE FOR THE TREATMENT OF CARDIOVASCULAR DISEASES
DERIVES DE TETRAZOL ET LEUR UTILISATION POUR TRAITER DES MALADIES DU COEUR ET DE LA CIRCULATION SANGUINE

(30) Priorität: 21.10.2005 DE 102005050375
(43) Veröffentlichungstag der Anmeldung: 09.07.2008
(73) Patentinhaber: Bayer Intellectual Property GmbH, 40789 Monheim (DE)
(72) Erfinder: BARTEL, Stephan, 51515 Kürten (DE); HAHN, Michael, 40764 Langenfeld (DE); MORADI, Wahed, Ahmed, 40789 Monheim (DE); BECKER, Eva-Maria, 42117 Wuppertal (DE); RÖLLE, Thomas, 51381 Leverkusen (DE); STASCH, Johannes-Peter, 42651 Solingen (DE); SCHLEMMER, Karl-Heinz, 42113 Wuppertal (DE); WUNDER, Frank, 42117 Wuppertal (DE); KNORR, Andreas, 40699 Erkrath (DE)
(74) Vertreter: BIP Patents
(86) Internationale Anmeldenummer: PCT/EP2006/009727
(87) Internationale Veröffentlichungsnummer: WO 2007/045370

(56) Entgegenhaltungen:
- WO-A-01/19355
- WO-A-01/19778
- WO-A2-01/19776

## Beschreibung

Die vorliegende Anmeldung betrifft neue Tetrazol-Derivate, Verfahren zu ihrer Herstellung, ihre Verwendung zur Behandlung und/oder Prophylaxe von Krankheiten sowie ihre Verwendung zur Herstellung von Arzneimitteln zur Behandlung und/oder Prophylaxe von Krankheiten, insbesondere zur Behandlung und/oder Prävention kardiovaskulärer Erkrankungen.

Eines der wichtigsten zellulären Übertragungssysteme in Säugerzellen ist das cyclische Guanosinmonophosphat (cGMP). Zusammen mit Stickstoffmonoxid (NO), das aus dem Endothel freigesetzt wird und hormonelle und mechanische Signale überträgt, bildet es das NO/cGMP-System. Die Guanylatcyclasen katalysieren die Biosynthese .von cGMP aus Guanosintriphosphat (GTP). Die bisher bekannten Vertreter dieser Familie lassen sich sowohl nach strukturellen Merkmalen als auch nach der Art der Liganden in zwei Gruppen aufteilen: Die partikulären, durch natriuretische Peptide stimulierbaren Guanylatcyclasen und die löslichen, durch NO stimulierbaren Guanylatcyclasen. Die löslichen Guanylatcyclasen bestehen aus zwei Untereinheiten und enthalten höchstwahrscheinlich ein Häm pro Heterodimer, das ein Teil des regulatorischen Zentrums ist. Dieses hat eine zentrale Bedeutung für den Aktivierungsmechanismus. NO kann an das Eisenatom des Häms binden und so die Aktivität des Enzyms deutlich erhöhen. Hämfreie Präparationen lassen sich hingegen nicht durch NO stimulieren. Auch Kohlenmonoxid (CO) ist in der Lage, am Eisen-Zentralatom des Häms anzugreifen, wobei die Stimulierung durch CO deutlich geringer ist als die durch NO.

Durch die Bildung von cGMP und der daraus resultierenden Regulation von Phosphodiesterasen, Ionenkanälen und Proteinkinasen spielt die Guanylatcyclase eine entscheidende Rolle bei unterschiedlichen physiologischen Prozessen, insbesondere bei der Relaxation und Proliferation glatter Muskelzellen, der Plättchenaggregation und -adhäsion und der neuronalen Signalübertragung sowie bei Erkrankungen, welche auf einer Störung der vorstehend genannten Vorgänge beruhen. Unter pathophysiologischen Bedingungen kann das NO/cGMP-System supprimiert sein, was zum Beispiel zu Bluthochdruck, einer Plättchenaktivierung, einer vermehrten Zellproliferation, endothelialer Dysfunktion, Atherosklerose, Angina pectoris, Herzinsuffzienz, Thrombosen, Schlaganfall und Myokardinfarkt führen kann.

Eine auf die Beeinflussung des cGMP-Signalweges in Organismen abzielende NO-unabhängige Behandlungsmöglichkeit für derartige Erkrankungen ist aufgrund der zu erwartenden hohen Effizienz und geringen Nebenwirkungen ein vielversprechender Ansatz.

Zur therapeutischen Stimulation der löslichen Guanylatcyclase wurden bisher ausschließlich Verbindungen wie organische Nitrate verwendet, deren Wirkung auf NO beruht. Dieses wird durch Biokonversion gebildet und aktiviert die lösliche Guanylatcyclase durch Angriffe am Eisen-Zentralatom des Häms. Neben den Nebenwirkungen gehört die Toleranzentwicklung zu den entscheidenden Nachteilen dieser Behandlungsweise.

In den letzten Jahren wurden einige Substanzen beschrieben, die die lösliche Guanylatcyclase direkt, d.h. ohne vorherige Freisetzung von NO stimulieren, wie beispielsweise 3-(5'-Hydroxy-methyl-2'-furyl)-1-benzylindazol [YC-1, Wu et al., Blood 84 (1994), 4226; Mülsch et al., Br. J. Pharmacol. 120 (1997), 681], Fettsäuren [Goldberg et al., J. Biol. Chem. 252 (1977), 1279], Diphenyliodonium-hexafluorophosphat [Pettibone et al., Eur. J. Pharmacol. 116 (1985), 307], Isoliquiritigenin [Yu et al., Br. J. Pharmacol. 114 (1995), 1587], sowie verschiedene substituierte Pyrazolderivate (WO 98/16223, WO 98/16507 und WO 98/23619).

Die vorstehend beschriebenen Stimulatoren der löslichen Guanylatcyclase stimulieren das Enzym entweder direkt über die Häm-Gruppe (Kohlenmonoxid, Stickstoffmonoxid oder Diphenyliodoniumhexafluorophosphat) durch Interaktion mit dem Eisenzentrum der Häm-Gruppe und eine sich daraus ergebende, zur Erhöhung der Enzymaktivität führende Konformationsänderung [Gerzer et al., FEBS Lett. 132 (1981), 71] oder über einen Häm-abhängigen Mechanismus, der unabhängig von NO ist, aber zu einer Potenzierung der stimulierenden Wirkung von NO oder CO führt [z.B. YC-1, Hoenicka et al., J. Mol. Med. 77 (1999) 14; oder die in WO 98/16223, WO 98/16507 und WO 98/23619 beschriebenen Pyrazolderivate].

Die in der Literatur behauptete stimulierende Wirkung von Isoliquiritigenin und von Fettsäuren, wie z.B. von Arachidonsäure, Prostaglandin-Endoperoxiden und Fettsäure-Hydroperoxiden auf die lösliche Guanylatcyclase konnte nicht bestätigt werden [vgl. z.B. Hoenicka et al., J. Mol. Med. 77 (1999), 14].

Entfernt man von der löslichen Guanylatcyclase die Häm-Gruppe, zeigt das Enzym immer noch eine nachweisbare katalytische Basalaktivität, d.h. es wird nach wie vor cGMP gebildet. Die verbleibende katalytische Basalaktivität des Häm-freien Enzyms ist durch keinen der vorstehend genannten bekannten Stimulatoren stimulierbar.

Es wurde eine Stimulation von Häm-freier löslicher Guanylatcyclase durch Protoporphyrin IX beschrieben [Ignarro et al., Adv. Pharmacol. 26 (1994), 35]. Allerdings kann Protoporphyrin IX als Mimik für das NO-Häm-Addukt angesehen werden, weshalb die Zugabe von Protoporphyrin IX zur löslichen Guanylatcyclase zur Bildung einer der durch NO stimulierten Häm-haltigen löslichen Guanylatcyclase entsprechenden Struktur des Enzyms führen dürfte. Dies wird auch durch die Tatsache belegt, dass die stimulierende Wirkung von Protoporphyrin IX durch den vorstehend beschriebenen NO-unabhängigen, aber Häm-abhängigen Stimulator YC-1 erhöht wird [Mülsch et al., Naunyn Schmiedebergs Arch. Pharmacol. 355, R47].

Im Gegensatz zu den vorstehend beschriebenen Stimulatoren der löslichen Guanylatcyclase sind die Verbindungen der vorliegenden Erfindung in der Lage, sowohl die Häm-haltige als auch die Häm-freie Form der löslichen Guanylatcyclase zu aktivieren. Die Stimulierung des Enzyms verläuft bei diesen neuen Aktivatoren also über einen Häm-unabhängigen Weg, was auch dadurch belegt wird, dass die neuen Aktivatoren am Häm-haltigen Enzym einerseits keine synergistische Wirkung mit NO zeigen und andererseits sich die Wirkung dieser neuartigen Aktivatoren nicht durch den Häm-abhängigen Inhibitor der löslichen Guanylatcyclase, 1*H*,2,4-Oxadiazol-(4,3-a)-chinoxalin-1-on (ODQ), blockieren lässt.

In EP 0 341 551-A1 werden Alkensäure-Derivate als Leukotrien-Antagonisten für die Behandlung von Erkrankungen des Kreislauf- und Atmungssystems offenbart. In WO 01/19355, WO 01/19776, WO 01/19778, WO 01/19780, WO 02/070462 und WO 02/070510 werden Dicarbonsäure- bzw. Aminodicarbonsäure-Derivate als Stimulatoren der löslichen Guanylatcyclase zur Behandlung von Herz-Kreislauf-Erkrankungen beschrieben. Allerdings zeigte es sich, dass diese Verbindungen hinsichtlich ihrer pharmakokinetischen Eigenschaften Nachteile aufweisen, wie insbesondere eine geringe Bioverfügbarkeit und/oder eine nur kurze Wirkdauer nach oraler Gabe.

Aufgabe der vorliegenden Erfindung war daher die Bereitstellung neuer Verbindungen, welche als Aktivatoren der löslichen Guanylatcyclase wirken, jedoch nicht die vorstehend aufgeführten Nachteile der Verbindungen aus dem Stand der Technik aufweisen.

Diese Aufgabe wird durch die in der vorliegenden Erfindung beschriebenen Verbindungen gelöst. Diese Verbindungen zeichnen sich strukturell im Vergleich zu den Verbindungen aus dem Stand der Technik durch eine Tetrazol-Gruppierung in Verbindung mit einer 1,4-Diphenylbut-1-en-3-yl- oder 1,5-Diphenylpent-1-en-3-yl-Kernstruktur aus.

Im Einzelnen betrifft die vorliegende Erfindung Verbindungen der allgemeinen Formel (I) 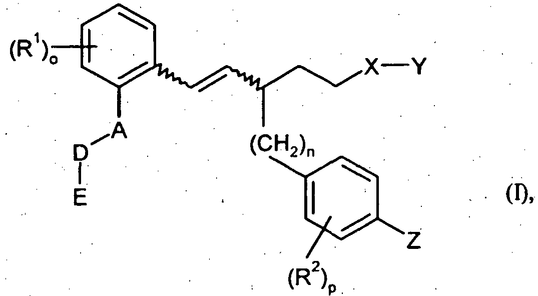 in welcher
- A: für O oder CH₂ steht,
- D: für eine Bindung oder für (C₁-C₇)-Alkandiyl, (C₂-C₇)-Alkendiyl oder (C₂-C₇)-Alkindiyl steht,
- E: für Wasserstoff, Trifluormethyl oder für eine Gruppe der Formel 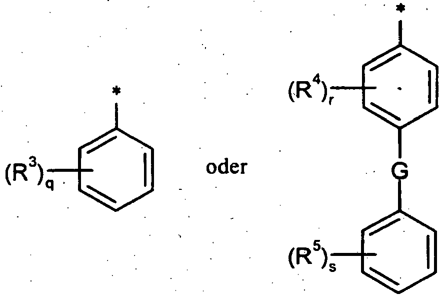 steht, worin * die Verknüpfungsstelle mit der Gruppe D und
- G: eine Bindung, CH₂, -CH₂-CH₂- oder -CH=CH- bedeutet,
- X: für -CH₂-CH₂- oder eine Gruppe der Formel 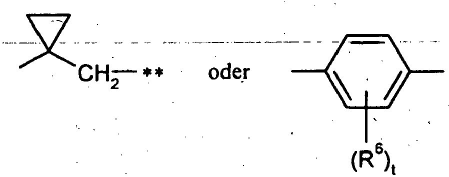 steht, worin ** die Verknüpfungsstelle mit der Gruppe Y bedeutet,
- Y: für Carboxyl
und
- Z: für eine Gruppe der Formel 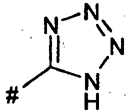
oder
- Y: für eine Gruppe der Formel 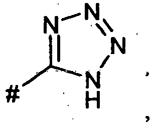
worin # jeweils die Verknüpfungsstelle bedeutet,
und
- Z: für Carboxyl stehen,
- n: für die Zahl 1 oder 2 steht,
- R¹, R², R³, R⁴, R⁵ und R⁶: unabhängig voneinander für einen Substituenten ausgewählt aus der Reihe Halogen, (C₁-C₆)-Alkyl, Trifluormethyl, (C₁-C₆)-Alkoxy, Trifluormethoxy, Cyano und Nitro stehen,
und
o, p, q, r, s und t unabhängig voreinander jeweils für die Zahl 0, 1, 2, 3 oder 4 stehen,
wobei für den Fall, dass R¹, R², R³, R⁴, R⁵ oder R⁶ mehrfach auftreten, ihre Bedeutungen jeweils gleich oder verschieden sein können,
sowie ihre Salze, Solvate und Solvate der Salze.

Erfindungsgemäße Verbindungen sind die Verbindungen der Formel (I) und deren Salze, Solvate und Solvate der Salze, die von Formel (I) umfassten Verbindungen der nachfolgend genannten Formeln und deren Salze, Solvate und Solvate der Salze sowie die von Formel (I) umfassen, nachfolgend als Ausführungsbeispiele genannten Verbindungen und deren Salze, Solvate und Solvate der Salze, soweit es sich bei den von Formel (I) umfassten, nachfolgend genannten Verbindungen nicht bereits um Salze, Solvate und Solvate der Salze handelt.

Die erfindungsgemäßen Verbindungen können in Abhängigkeit von ihrer Struktur in stereoisomeren Formen (Enantiomere, Diastereomere) existieren. Die Erfindung umfasst deshalb die Enantiomeren oder Diastereomeren und ihre jeweiligen Mischungen. Aus solchen Mischungen von Enantiomeren und/oder Diastereomeren lassen sich die stereoisomer einheitlichen Bestandteile in bekannter Weise isolieren.

Die Gruppierung 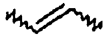 in Formel (I) bedeutet, dass diese CC-Doppelbindung in einer *cis-* oder in einer *trans*-Konfiguration vorliegen kann. Beide isomeren Formen werden von der vorliegenden Erfindung umfasst. Bevorzugt sind Verbindungen der Formel (I) mit einer *trans*-Anordnung dieser Doppelbindung.

Sofern die erfindungsgemäßen Verbindungen in tautomeren Formen vorkommen können, umfasst die vorliegende Erfindung sämtliche tautomere Formen.

Als Salze sind im Rahmen der vorliegenden Erfindung physiologisch unbedenkliche Salze der erfindungsgemäßen Verbindungen bevorzugt. Umfasst sind auch Salze, die für pharmazeutische Anwendungen selbst nicht geeignet sind, jedoch beispielsweise für die Isolierung oder Reinigung der erfindungsgemäßen Verbindungen verwendet werden können.

Physiologisch unbedenkliche Salze der erfindungsgemäßen Verbindungen umfassen Säureadditionssalze von Mineralsäuren, Carbonsäuren und Sulfonsäuren, z.B. Salze der Chlorwasserstoffsäure, Bromwasserstoffsäure, Schwefelsäure, Phosphorsäure, Methansulfonsäure, Ethansulfonsäure, Toluolsulfonsäure, Benzolsulfonsäure, Naphthalindisulfonsäure, Essigsäure, Trifluoressigsäure, Propionsäure, Milchsäure, Weinsäure, Äpfelsäure, Zitronensäure, Fumarsäure, Maleinsäure und Benzoesäure.

Physiologisch unbedenkliche Salze der erfindungsgemäßen Verbindungen umfassen auch Salze üblicher Basen, wie beispielhaft und vorzugsweise Alkalimetallsalze (z.B. Natrium- und Kaliumsalze), Erdalkalisalze (z.B. Calcium- und Magnesiumsalze) und Ammoniumsalze, abgeleitet von Ammoniak oder organischen Aminen mit 1 bis 16 C-Atomen, wie beispielhaft und vorzugsweise Ethylamin, Diethylamin, Triethylamin, Ethyldiisopropylamin, Monoethanolamin, Diethanolamin, Triethanolamin, Dicyclohexylamin, Dimethylaminoethanol, Prokain, Dibenzylamin, N-Methylmorpholin, Arginin, Lysin, Ethylendiamin und N-Methylpiperidin.

Als Solvate werden im Rahmen der Erfindung solche Formen der erfindungsgemäßen Verbindungen bezeichnet, welche in festem oder flüssigem Zustand durch Koordination mit Lösungsmittelmolekülen einen Komplex bilden. Hydrate sind eine spezielle Form der Solvate, bei denen die Koordination mit Wasser erfolgt. Als Solvate sind im Rahmen der vorliegenden Erfindung Hydrate bevorzugt.

Im Rahmen der vorliegenden Erfindung haben die Substituenten, soweit nicht anders spezifiziert, die folgende Bedeutung:
(C₁-C₄)-Alkyl und (C₁-C₄)-Alkyl stehen im Rahmen der Erfindung für einen geradkettigen oder verzweigten Alkylrest mit 1 bis 6 bzw. 1 bis 4 Kohlenstoffatomen. Bevorzugt ist ein geradkettiger oder verzweigter Alkylrest mit 1 bis 4 Kohlenstoffatomen. Beispielhaft und vorzugsweise seien genannt: Methyl, Ethyl, n-Propyl, Isopropyl, n-Butyl, *iso*-Butyl, *sec*.-Butyl, *tert*.-Butyl, 1-Ethylpropyl, n-Pentyl und n-Hexyl.
(C₁-C₇)-Alkandiyl steht im Rahmen der Erfindung für einen geradkettigen oder verzweigten divalenten Alkylrest mit 1 bis 7 Kohlenstoffatomen. Bevorzugt ist ein geradkettiger Alkandiylrest mit 1 bis 6 Kohlenstoffatomen. Beispielhaft und vorzugsweise seien genannt: Methylen, 1,2-Ethylen, Ethan-1,1-diyl, 1,3-Propylen, Propan-1,1-diyl, Propan-1,2-diyl, Propan-2,2-diyl, 1,4-Butylen, Butan-1,2-diyl, Butan-1,3-diyl, Butan-2,3-diyl, Pentan-1,5-diyl, Pentan-2,4-diyl, 3-Methyl-pentan-2,4-diyl und Hexan-1,6-diyl.
(C₂-C₇)-Alkendiyl steht im Rahmen der Erfindung für einen geradkettigen oder verzweigten divalenten Alkenylrest mit 2 bis 7 Kohlenstoffatomen und bis zu 3 Doppelbindungen. Bevorzugt ist ein geradkettiger Alkendiylrest mit 2 bis 6 Kohlenstoffatomen und bis zu 2 Doppelbindungen. Beispielhaft und vorzugsweise seien genannt: Ethen-1,1-diyl, Ethen-1,2-diyl, Propen-1,1-diyl, Propen-1,2-diyl, Propen-1,3-diyl, But-1-en-1,4-diyl, But-1-en-1,3-diyl, But-2-en-1,4-diyl, Buta-1,3-dien-1,4-diyl, Pent-2-en-1,5-diyl, Hex-3-en-1,6-diyl und Hexa-2,4-dien-1,6-diyl.
(C₂-C₇)-Alkindiyl steht im Rahmen der Erfindung für einen geradkettigen oder verzweigten divalenten Alkinylrest mit 2 bis 7 Kohlenstoffatomen und bis 3 Dreifachbindungen. Bevorzugt ist ein geradkettiger Alkindiylrest mit 2 bis 6 Kohlenstoffatomen und bis zu 2 Dreifachbindungen. Beispielhaft und vorzugsweise seien genannt: Ethin-1,2-diyl, Propin-1,3-diyl, But-1-in-1,4-diyl, But-1-in-1,3-diyl, But-2-in-1,4-diyl, Pent-2-in-1,5-diyl, Pent-2-in-1,4-diyl und Hex-3-in-1,6-diyl.
(C₁-C₆)-Alkoxy und (C₁-C₄)-Alkoxy stehen im Rahmen der Erfindung für einen geradkettigen oder verzweigten Alkoxyrest mit 1 bis 6 bzw. 1 bis 4 Kohlenstoffatomen. Bevorzugt ist ein geradkettiger oder verzweigter Alkoxyrest mit 1 bis 4 Kohlenstoffatomen. Beispielhaft und vorzugsweise seien genannt: Methoxy, Ethoxy, n-Propoxy, Isopropoxy, n-Butoxy, *tert*.-Butoxy, n-Pentoxy und n-Hexoxy.
Halogen schließt im Rahmen der Erfindung Fluor, Chlor, Brom und Iod ein. Bevorzugt sind Chlor oder Fluor.

Wenn Reste in den erfindungsgemäßen Verbindungen substituiert sind, können die Reste, soweit nicht anders spezifiziert, ein- oder mehrfach substituiert sein. Im Rahmen der vorliegenden Erfindung gilt, dass für alle Reste, die mehrfach auftreten, deren Bedeutung unabhängig voneinander ist. Eine Substitution mit ein, zwei oder drei gleichen oder verschiedenen Substituenten ist bevorzugt. Ganz besonders bevorzugt ist die Substitution mit einem Substituenten.

Bevorzugt im Rahmen der vorliegenden Erfindung sind Verbindungen der Formel (I), in welcher
- A: fur O steht,
- D: für (C₁-C₇)-Alkandiyl steht,
- E: für Wasserstoff, Trifluormethyl oder für eine Gruppe der Formel 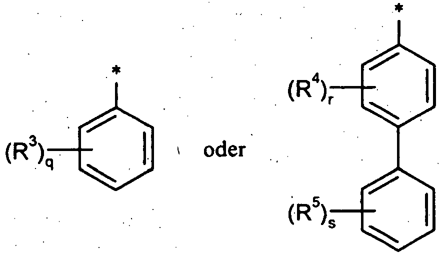 steht, worin * die Verknüpfungsstelle mit der Gruppe D bedeutet,
- X: für -CH₂-CH₂- oder eine Gruppe der Formel 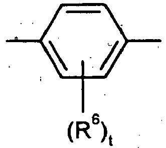 steht,
- Y: für Carboxyl
und
- Z: für eine Gruppe der Formel 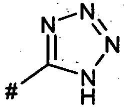
oder
- Y: für eine Gruppe der Formel 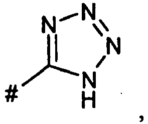
worin # jeweils die Verknüpfungsstelle bedeutet,
und
- Z: für Carboxyl stehen,
- n: für die Zahl 1 oder 2 steht,
- R¹, R³, R⁴ und R⁵: unabhängig voneinander für einen Substituenten ausgewählt aus der Reihe Fluor, Chlor, Brom, (C₁-C₄)-Alkyl, Trifluormethyl, (C₁-C₄)-Alkoxy und Trifluormethoxy stehen,
- o, q, r und s: unabhängig voneinander jeweils für die Zahl 0, 1 oder 2 stehen, wobei für den Fall, dass R¹, R³, R⁴ oder R⁵ mehrfach auftreten, ihre Bedeutungen jeweils gleich oder verschieden sein können,
- R² und R⁶: jeweils für Fluor stehen
und
p und t unabhängig voneinander jeweils für die Zahl 0 oder 1 stehen,
sowie ihre Salze, Solvate und Solvate der Salze.

Besonders bevorzugt im Rahmen der vorliegenden Erfindung sind Verbindungen der Formel (I-A) 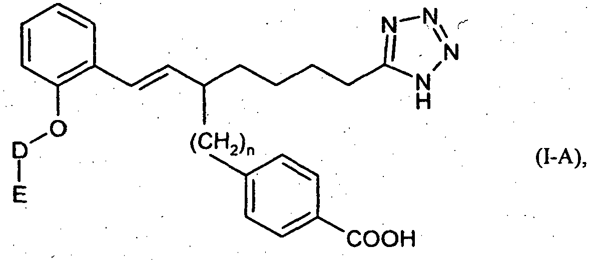 in welcher
- D: für (C₁-C₇)-Alkandiyl,
- E: für Wasserstoff oder eine Gruppe der Formel 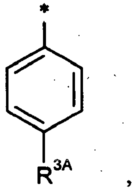
worin * die Verknüpfungsstelle mit der Gruppe D und
- R^{3A}: Wasserstoff, Fluor, Chlor, Methyl, *tert*.-Butyl, Trifluormethyl, Methoxy oder Trifluormethoxy bedeuten,
und
- n: für die Zahl 1 oder 2
stehen,
sowie ihre Salze, Solvate und Solvate der Salze.

Die in den jeweiligen Kombinationen bzw. bevorzugten Kombinationen von Resten im einzelnen angegebenen Reste-Definitionen werden unabhängig von den jeweiligen angegebenen Kombinationen der Reste beliebig auch durch Reste-Definitionen anderer Kombinationen ersetzt.

Ganz besonders bevorzugt sind Kombinationen von zwei oder mehreren der oben genannten Vorzugsbereiche.

Weiterer Gegenstand der Erfindung ist ein Verfahren zur Herstellung der erfindungsgemäßen Verbindungen der Formel (I), dadurch gekennzeichriet, dass man entweder
[A] Verbindungen der Formel (II-1) 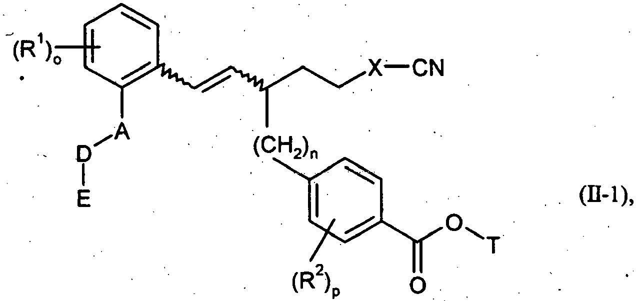 in welcher R¹, R², A, D, E, X, n, o und p jeweils die oben angegebenen Bedeutungen haben und
   T für (C₁-C₄)-Alkyl steht,
   in einem inerten Lösungsmittel mit einem Alkali-Azid in Gegenwart von Ammoniumchlorid oder mit Trimethylsilylazid gegebenenfalls in Gegenwart eines Katalysators zu Verbindungen der Formel (III-1) 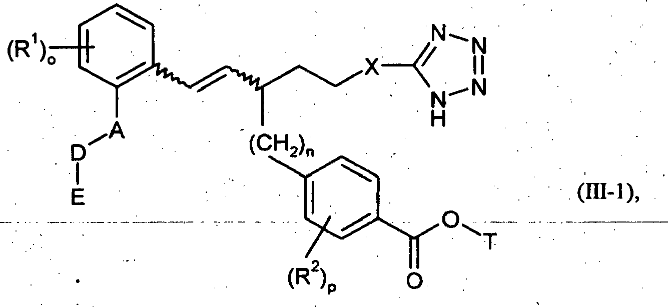 in welcher R¹, R², A, D, E, X, n, o, p und T jeweils die oben angegebenen Bedeutungen haben,
   oder
[B] Verbindungen der Formel (II-2) 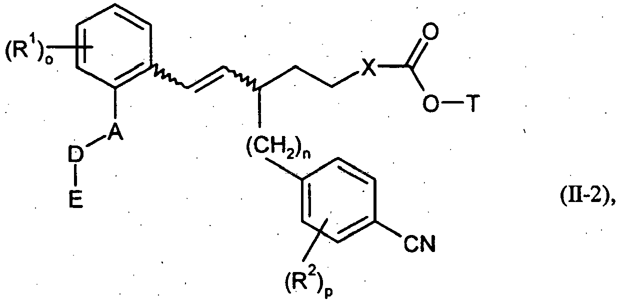 in welcher R¹, R², A, D, E, X, n, o, p und T jeweils die oben angegebenen Bedeutungen haben,
   in einem inerten Lösungsmittel mit einem Alkali-Azid in Gegenwart von Ammoniumchlorid oder mit Trimethylsilylazid gegebenenfalls in Gegenwart eines Katalysators zu Verbindungen der Formel (III-2) 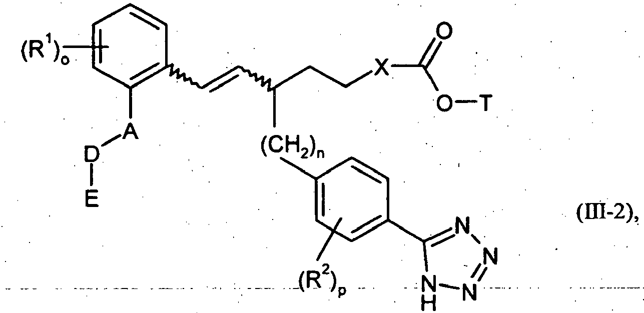
in welcher R¹, R², A, D, E, X, n, o, p und T jeweils die oben angegebenen Bedeutungen haben,
umsetzt
und die resultierenden Verbindungen der Formel (III-1) bzw. (III-2) durch Hydrolyse der Ester-Gruppierung -C(O)OT in die entsprechenden Carbonsäuren der Formel (I) überführt
und die Verbindungen der Formel (I) gegebenenfalls nach dem Fachmann bekannten Methoden in ihre Enantiomere und/oder Diastereomere trennt und/oder gegebenenfalls mit den entsprechenden (i) Lösungsmitteln und/oder (ii) Basen oder Säuren zu ihren Solvaten, Salzen und/oder Solvaten der Salze umsetzt.

Inerte Lösungsmittel für den Verfahrensschritt (II-1) → (III-1) bzw. (II-2) → (III-2) sind beispielsweise Ether wie Diethylether, Dioxan, Tetrahydrofuran, Glykoldimethylether oder Diethylenglykoldimethylether, Kohlenwasserstoffe wie Benzol, Toluol, Xylol, Hexan, Cyclohexan oder Erdölfraktionen, oder andere Lösungsmittel wie Dimethylsulfoxid, Dimethylformamid, *N,N*'-Dimethylpropylenharnstoff (DMPU) oder N-Methylpyrrolidon (NMP). Ebenso ist es möglich, Gemische der genannten Lösungsmittel einzusetzen. Bevorzugt wird Toluol verwendet.

Als Azid-Reagenz für diesen Verfahrensschritt ist insbesondere Natriumazid in Gegenwart von Ammoniumchlorid oder Trimethylsilylazid geeignet. Letztere Reaktion kann vorteilhafterweise in Gegenwart eines Katalysators durchgeführt werden. Hierfür eignen sich insbesondere Verbindungen wie Di-n-butylzinnoxid, Trimethylaluminium oder Zinkbromid. Bevorzugt wird Trimethylsilylazid in Kombination mit Di-n-butylzinnoxid verwendet.

Der Verfahrensschritt (II-1) → (III-1) bzw. (II-2) → (III-2) wird im Allgemeinen in einem Temperaturbereich von +50°C bis +150°C, bevorzugt bei +60°C bis +110°C durchgeführt. Die Umsetzung kann bei normalem, erhöhtem oder erniedrigtem Druck durchgeführt werden (z.B. von 0.5 bis 5 bar). Im Allgemeinen arbeitet man bei Normaldruck.

Die Hydrolyse der Carbonsäureester im Verfahrensschritt (III-1) bzw. (III-2) → (I) erfolgt nach üblichen Methoden, indem man die Ester in inerten Lösungsmitteln mit Säuren oder Basen behandelt, wobei bei letzterem die zunächst entstehenden Salze durch Behandeln mit Säure in die freien Carbonsäuren überführt werden. Im Falle der *tert*.-Butylester erfolgt die Esterspaltung bevorzugt mit Säuren.

Als inerte Lösungsmittel eignen sich für die Hydrolyse der Carbonsäureester Wasser oder die für eine Esterspaltung üblichen organischen Lösungsmittel. Hierzu gehören bevorzugt Alkohole wie Methanol, Ethanol, n-Propanol, Isopropanol, n-Butanol oder *tert*.-Butanol, oder Ether wie Diethylether, Tetrahydrofuran, Dioxan oder Glykoldimethylether, oder andere Lösungsmittel wie Aceton, Acetonitril, Dichlormethan, Dimethylformamid oder Dimethylsulfoxid. Ebenso ist es möglich, Gemische der genannten Lösungsmittel einzusetzen. Im Falle einer basischen Ester-Hydrolyse werden bevorzugt Gemische von Wasser mit Dioxan, Tetrahydrofuran, Methanol und/oder Ethanol eingesetzt. Im Falle der Umsetzung mit Trifluoressigsäure wird bevorzugt Dichlormethan und im Falle der Umsetzung mit Chlorwasserstoff bevorzugt Tetrahydrofuran, Diethylether, Dioxan oder Wasser verwendet.

Als Basen eignen sich für die Ester-Hydrolyse die üblichen anorganischen Basen. Hierzu gehören bevorzugt Alkali- oder Erdalkalihydroxide wie beispielsweise Natrium-, Lithium-, Kalium- oder Bariumhydroxid, oder Alkali- oder Erdalkalicarbonate wie Natrium-, Kalium- oder Calciumcarbonat. Besonders bevorzugt werden Natriumhydroxid oder Lithiumhydroxid eingesetzt.

Als Säuren eignen sich für die Esterspaltung im Allgemeinen Schwefelsäure, Chlorwasserstoff/ Salzsäure, Bromwasserstoff/Bromwasserstoffsäure, Phosphorsäure, Essigsäure, Trifluoressigsäure, Toluolsulfonsäure, Methansulfonsäure oder Trifluormethansulfonsäure oder deren Gemische gegebenenfalls unter Zusatz von Wasser. Bevorzugt sind Chlorwasserstoff oder Trifluoressigsäure im Falle der *tert*.-Butylester und Salzsäure im Falle der Methylester.

Die Esterspaltung erfolgt im Allgemeinen in einem Temperaturbereich von 0°C bis +100°C, bevorzugt bei +20°C bis +60°C. Die Umsetzungen können bei normalem, erhöhtem oder bei erniedrigtem Druck durchgeführt werden (z.B. von 0.5 bis 5 bar). Im Allgemeinen arbeitet man bei Normaldruck.

Die Verbindungen der Formeln (II-1) und (II-2) können nach den in EP 0 341 551-A1, WO 01/19355, WO 01/19776 und WO 01/19778 beschriebenen Verfahren hergestellt werden (vgl. auch die nachfolgenden Reaktionsschemata 1-14); der diesbezügliche Inhalt dieser Druckschriften wird hiermit ausdrücklich als Bestandteil der Offenbarung einbezogen.

Eine Trennung der erfindungsgemäßen Verbindungen in die entsprechenden Enantiomere und/oder Diastereomere kann gegebenenfalls, je nach Zweckmäßigkeit, auch bereits auf der Stufe der Verbindungen (II-1), (II-2), (III-1), (III-2) oder der in den Schemata 9 und 11-14 dargestellten phenolischen Vorstufen hierzu erfolgen, welche dann in separierter Form entsprechend der beschriebenen Verfahrenssequenz weiter umgesetzt werden. Eine solche Auftrennung der Stereoisomeren läßt sich nach üblichen, dem Fachmann bekannten Methoden durchrühren; vorzugsweise werden chromatographische Verfahren oder eine Trennung über diastereomere Salze verwendet.

Die Herstellung der erfindungsgemäßen Verbindungen kann durch die folgenden Syntheseschemata veranschaulicht werden:

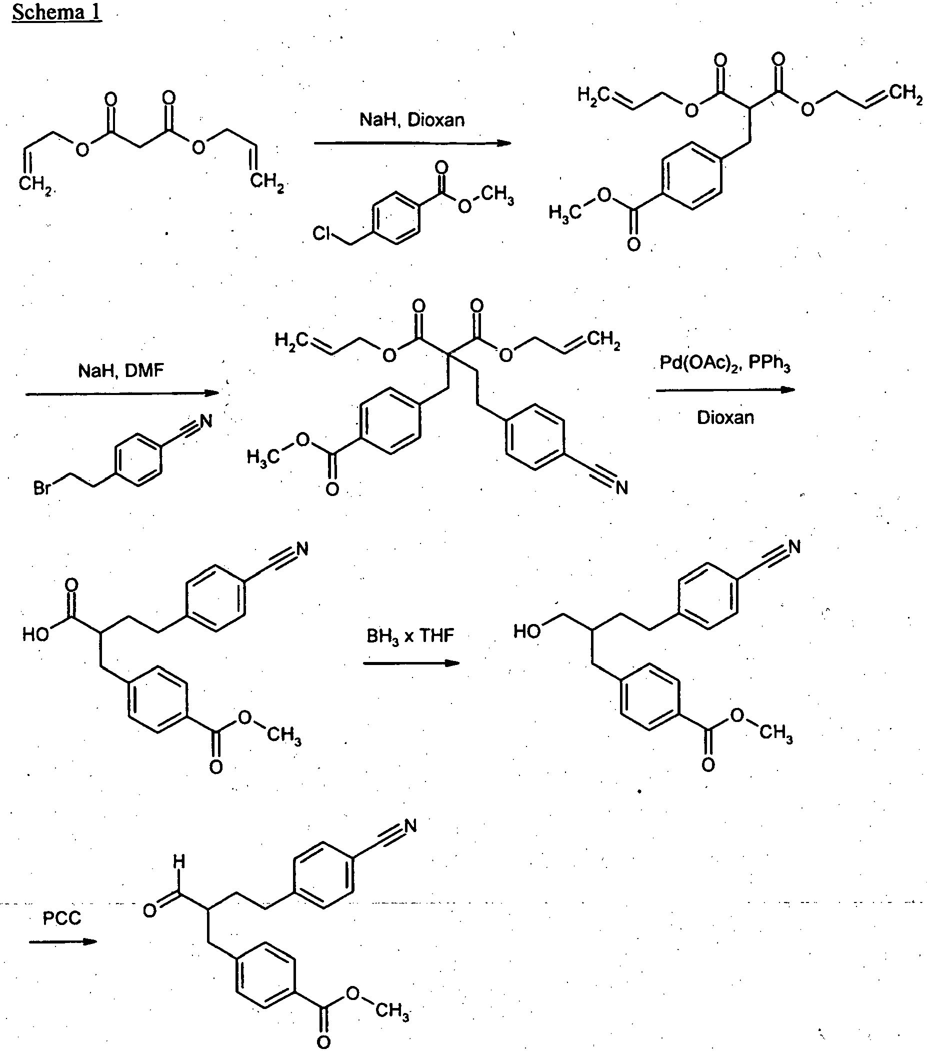

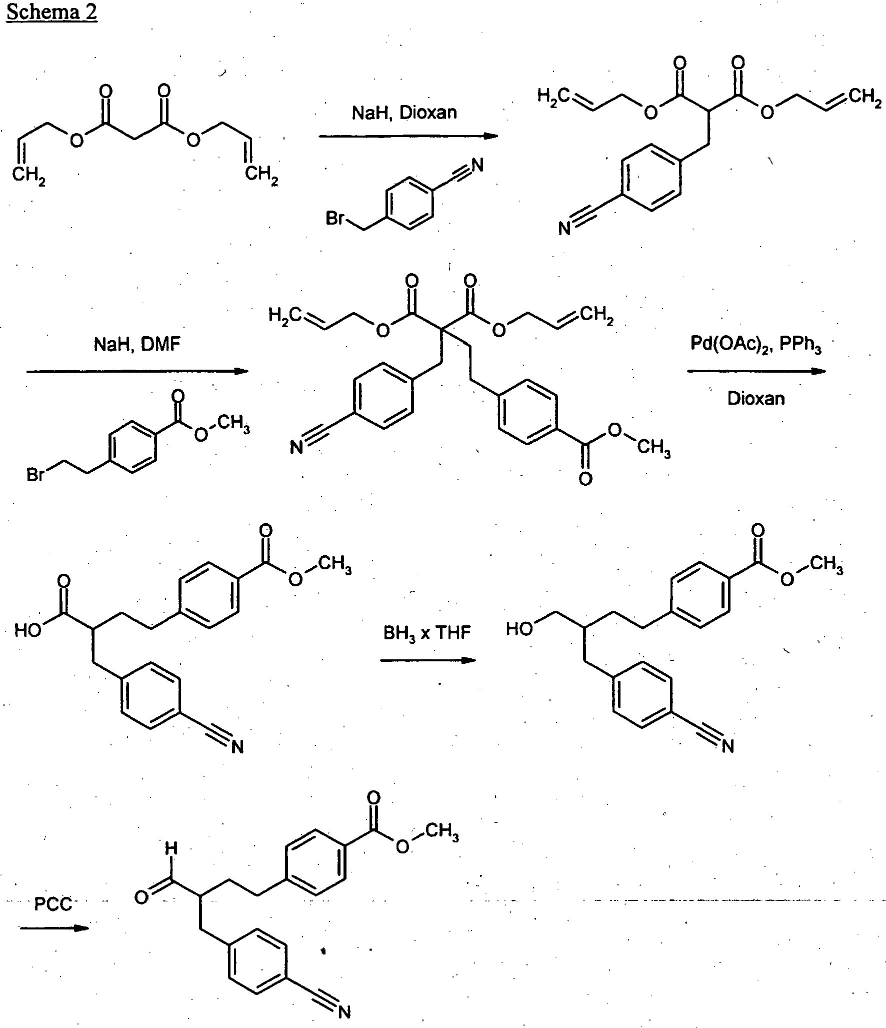

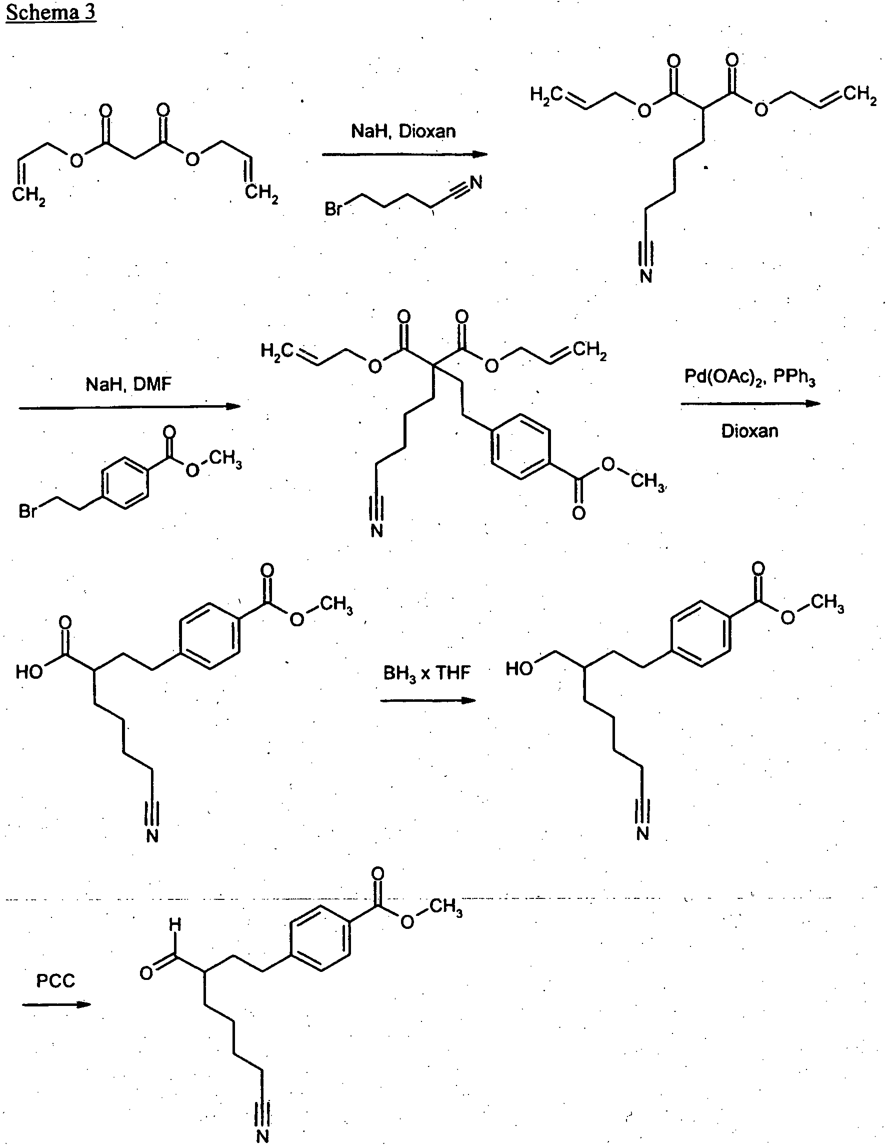

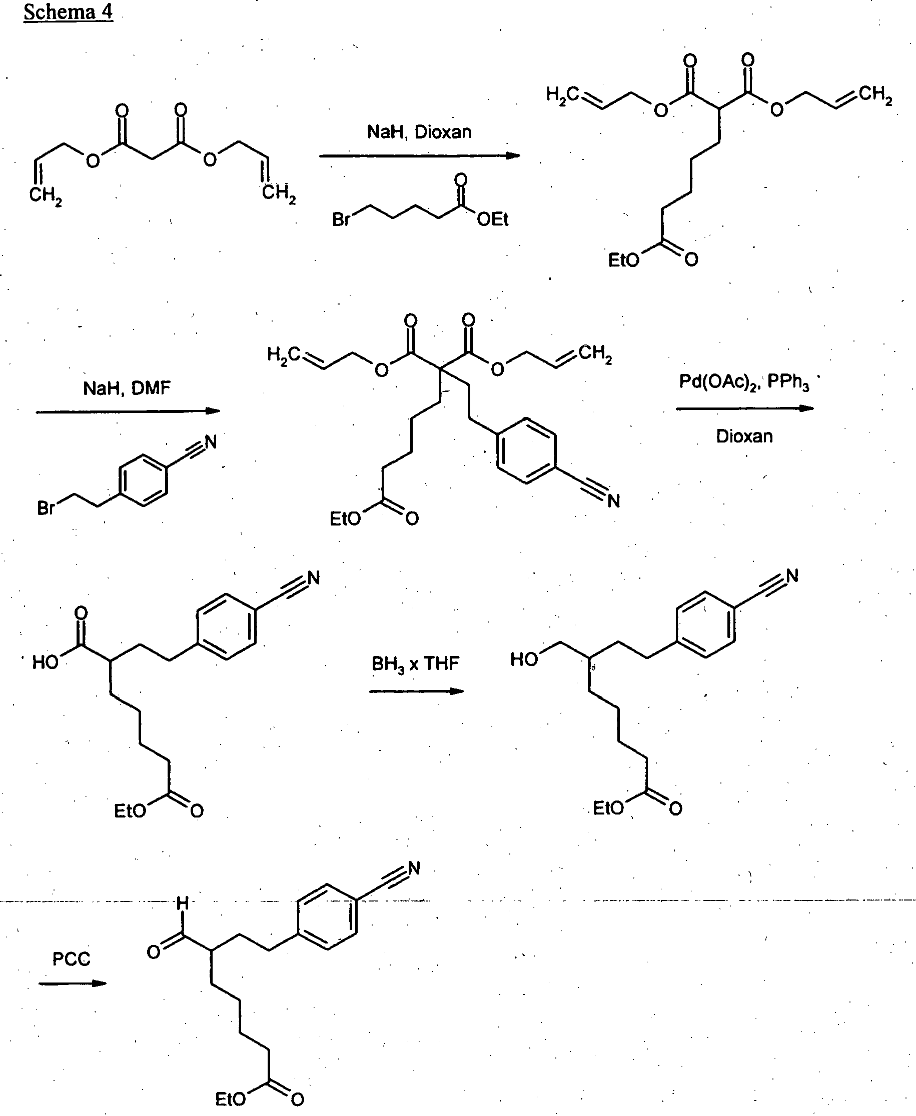

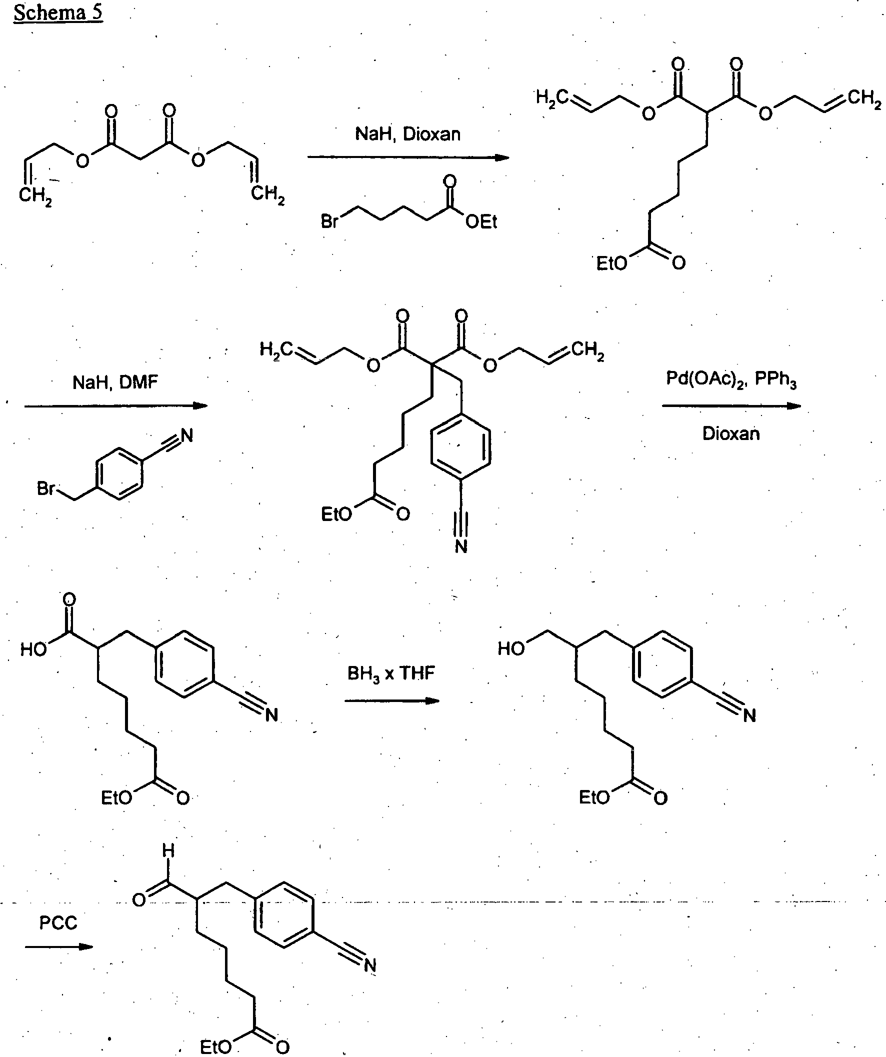

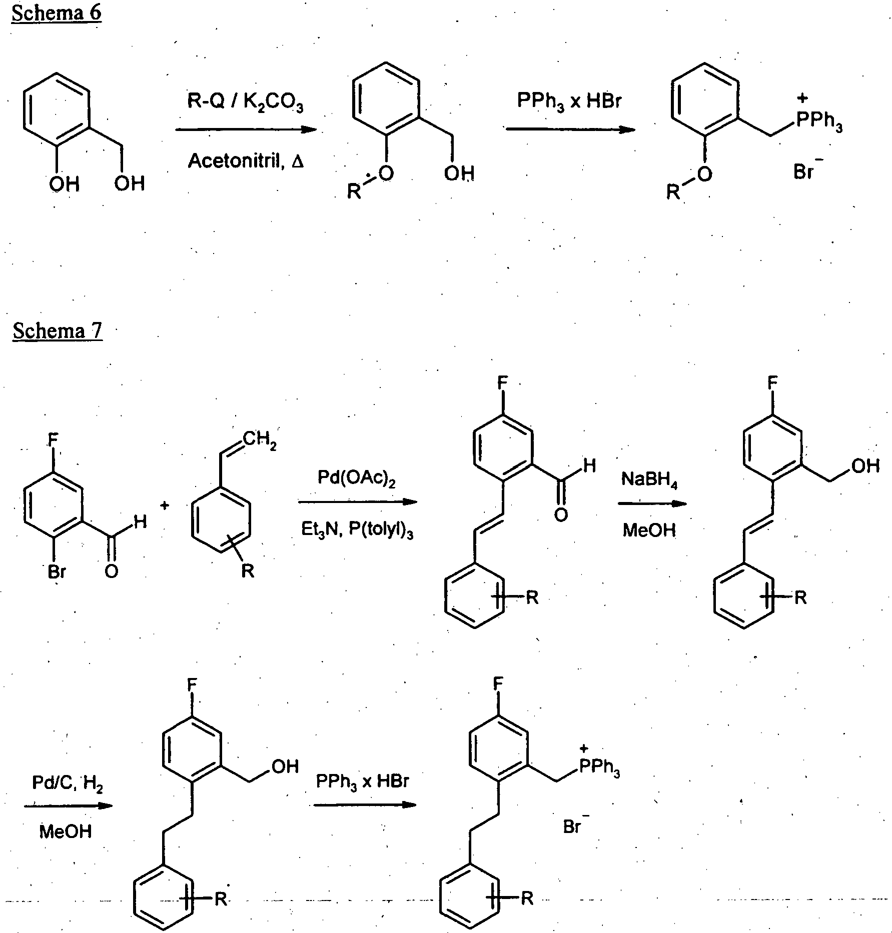

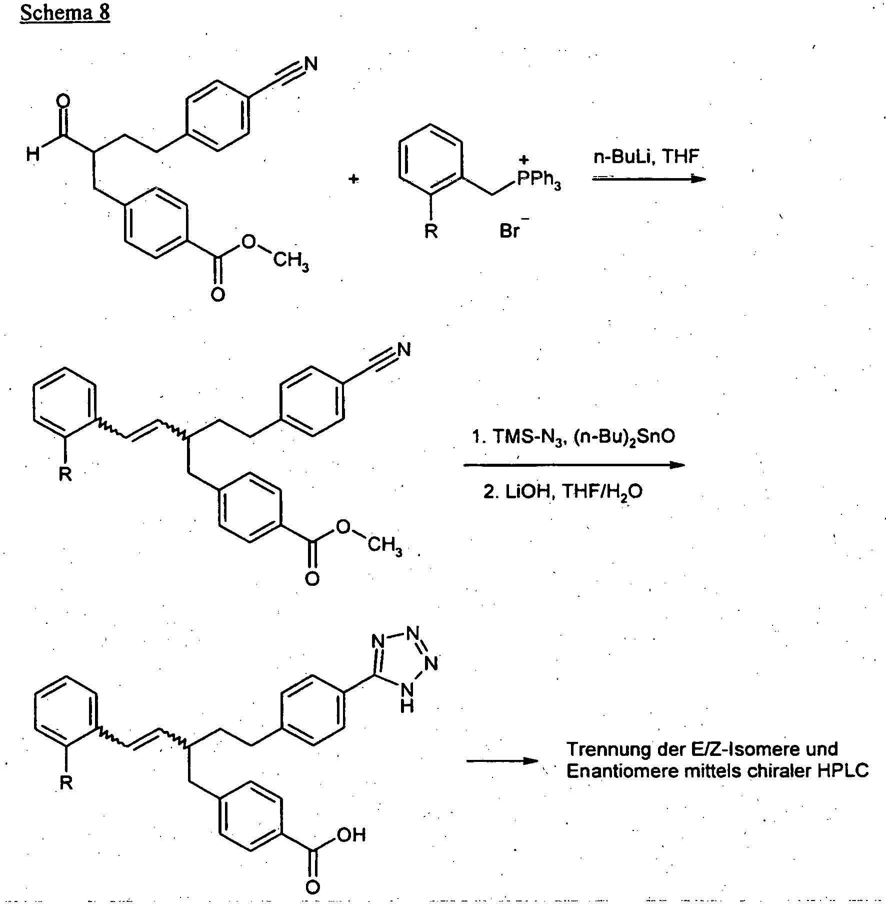

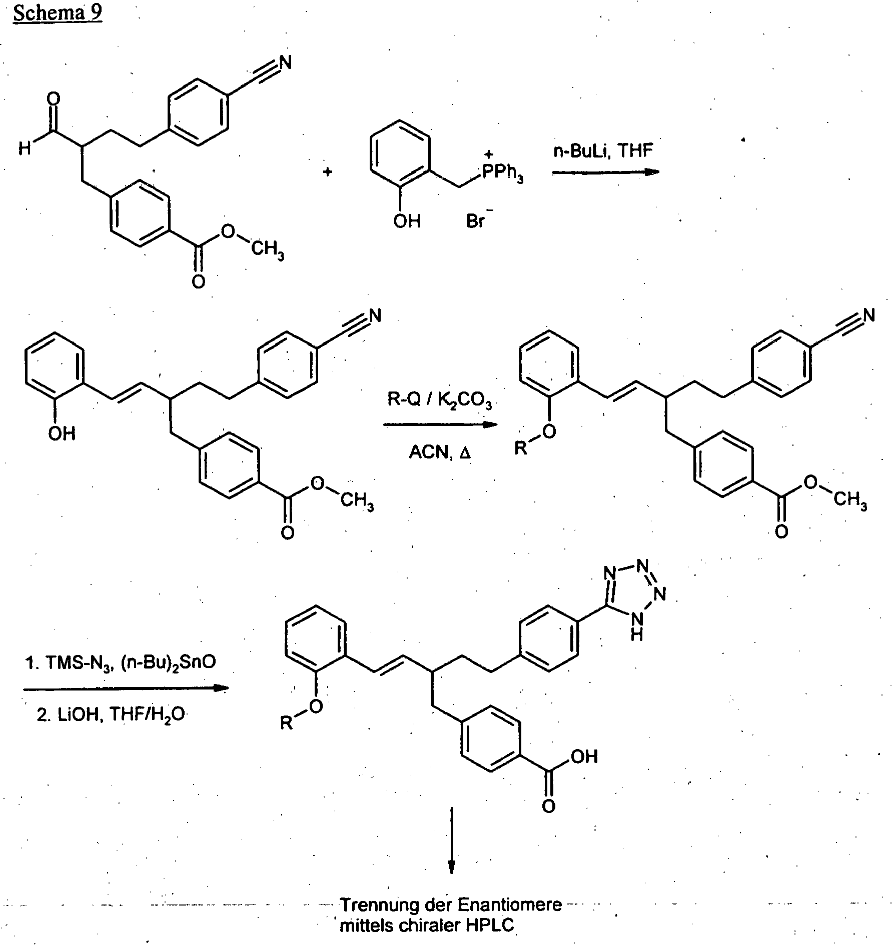

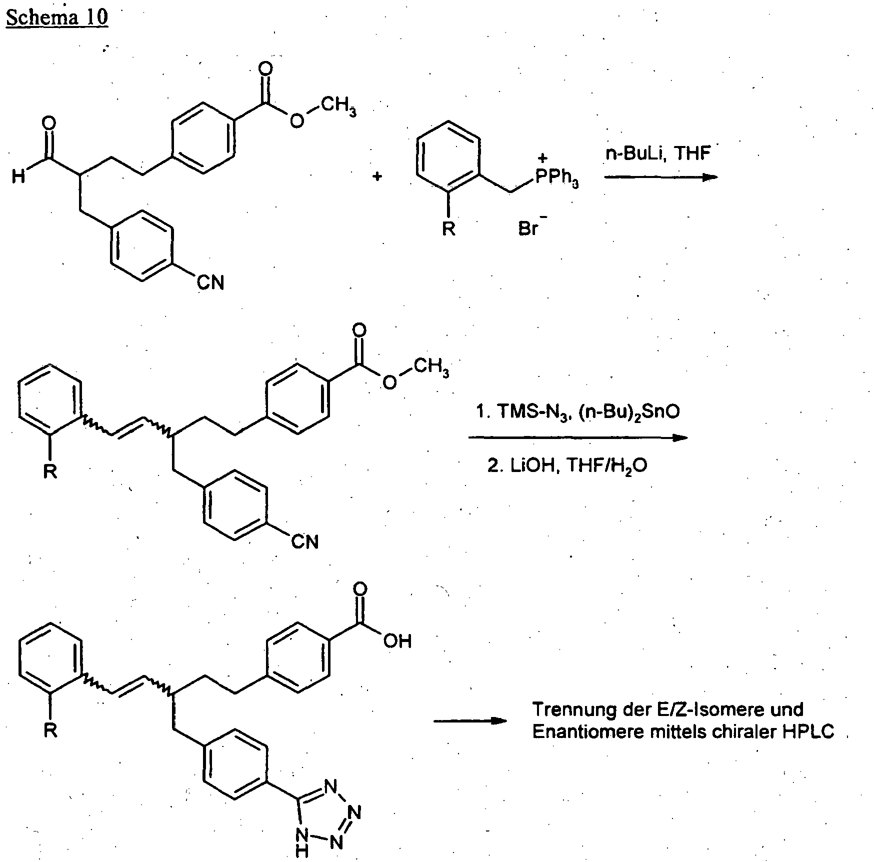

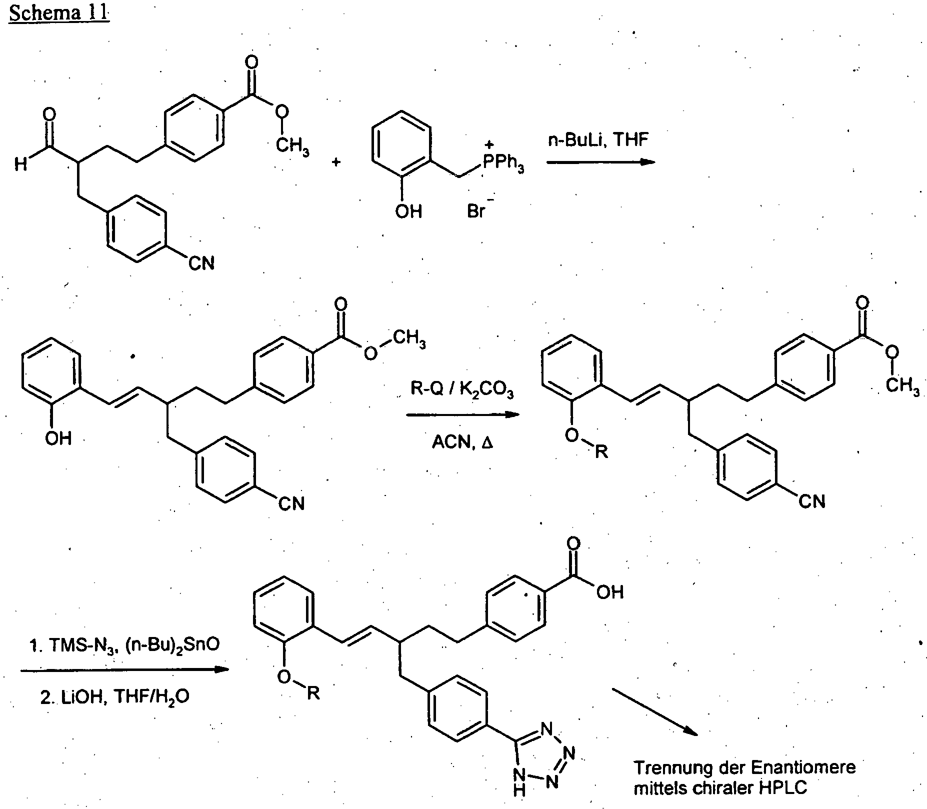

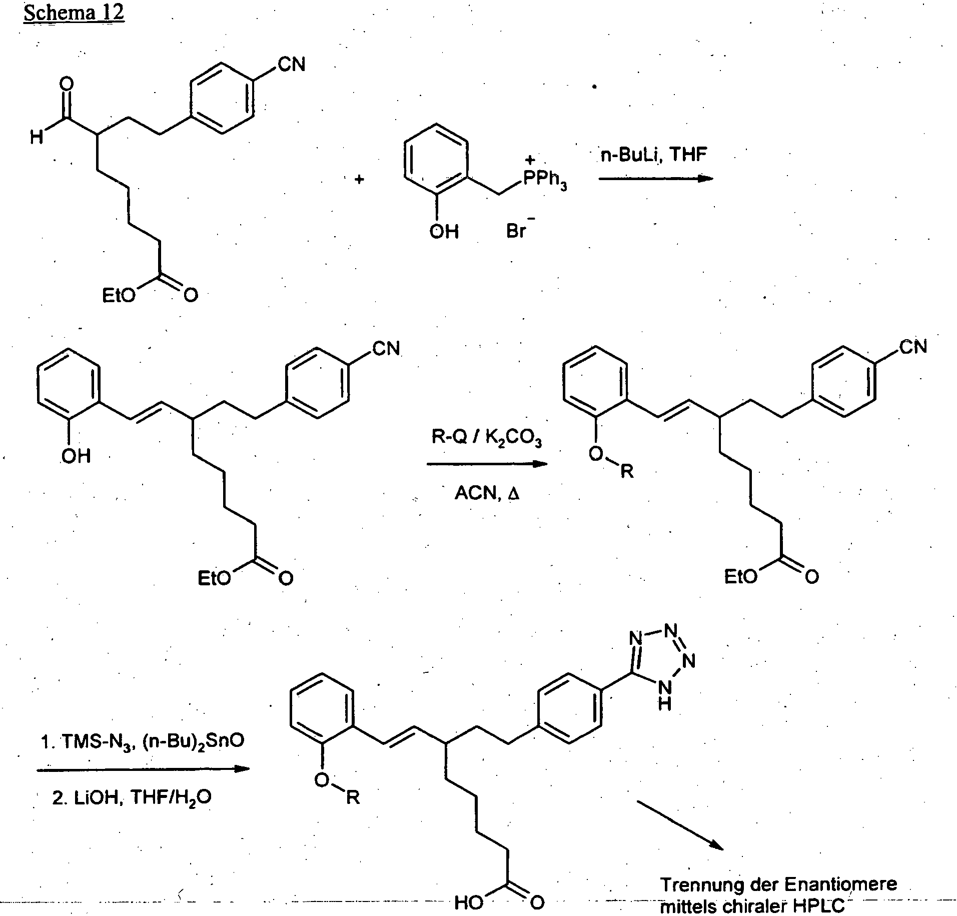

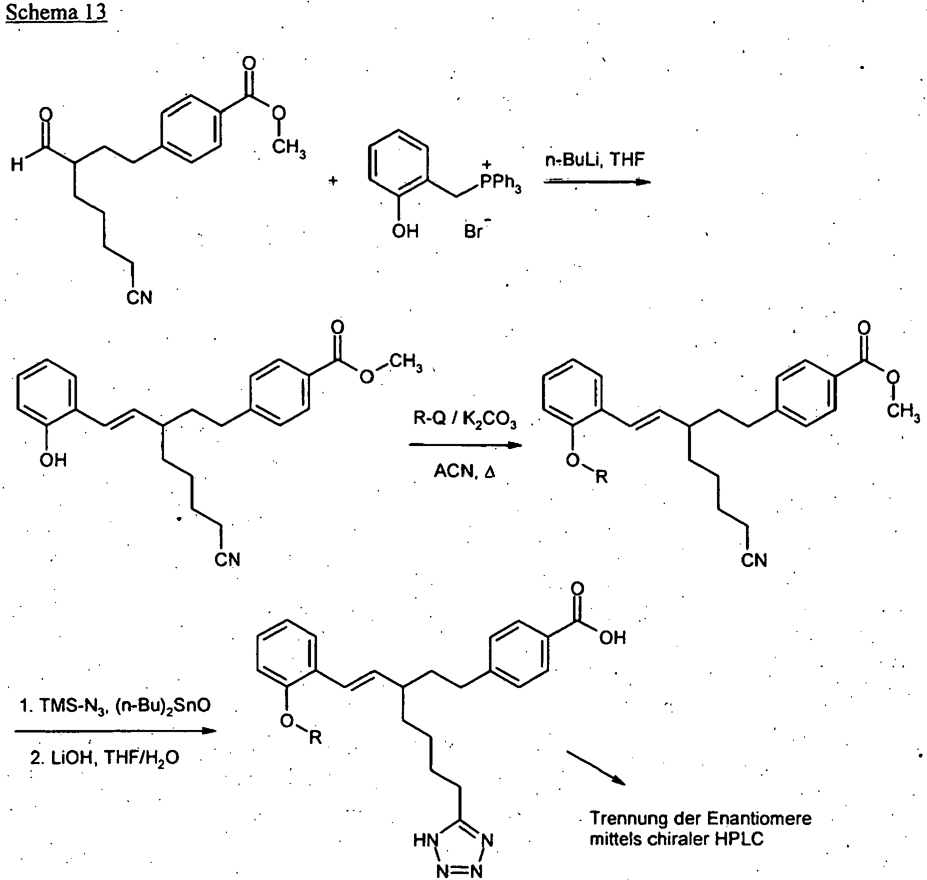

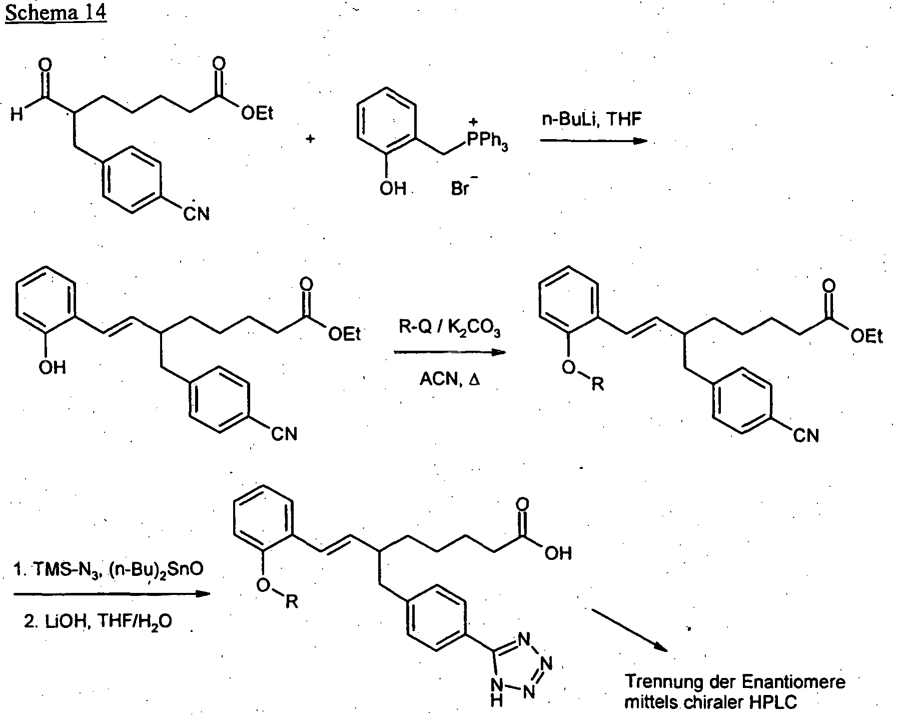

[Abkürzungen: Ac = Acetyl; ACN = Acetonitril; (Boc)₂O = Di-*tert*.-butylpyrocarbonat; Bu = Butyl; DME = 1,2-Dimethoxyethan; DMF = Dimethylformamid; DMSO = Dimethylsulfoxid; Et = Ethyl; Kat. = Katalysator; Me = Methyl; PCC = Pyridiniumchlorochromat; Ph = Phenyl; Q = Abgangsgruppe, z.B. Halogen; THF = Tetrahydrofuran; TMS = Trimethylsilyl].

Die erfindungsgemäßen Verbindungen besitzen wertvolle pharmakologische Eigenschaften und können zur Vorbeugung und Behandlung von Erkrankungen bei Menschen und Tieren verwendet werden.

Als besonderes und überraschendes Merkmal weisen die Verbindungen der vorliegenden Erfindung vorteilhafte pharmakokinetische Eigenschaften wie beispielsweise eine erhöhte Bioverfügbarkeit und/oder eine verlängerte Wirkdauer nach oraler Gabe auf.

Die erfindungsgemäßen Verbindungen führen zu einer Gefäßrelaxation, zu einer Thrombozytenaggregationshemmung und zu einer Blutdrucksenkung sowie zu einer Steigerung des koronaren Blutflusses. Diese Wirkungen sind über eine direkte Aktivierung der löslichen Guanylatcyclase und einen intrazellulären cGMP-Anstieg vermittelt.

Die erfindungsgemäßen Verbindungen können daher in Arzneimitteln zur Behandlung von kardiovaskulären Erkrankungen wie beispielsweise zur Behandlung des Bluthochdrucks und der Herzinsuffizienz, stabiler und instabiler Angina pectoris, pulmonaler Hypertonie, peripheren und kardialen Gefäßerkrankungen, Arrhythmien, zur Behandlung von thromboembolischen Erkrankungen und Ischämien wie Myokardinfarkt, Hirnschlag, transistorischen und ischämischen Attacken, peripheren Durchblutungsstörungen, zur Verhinderung von Restenosen wie nach Thrombolysetherapien, percutan-transluminalen Angioplastien (PTA), percutan-transluminalen Koronarangioplastien (PTCA) und Bypass sowie zur Behandlung von Arteriosklerose, asthmatischen Erkrankungen, Krankheiten des Urogenitalsystems wie beispielsweise Prostatahypertrophie, erektile Dysfunktion, weibliche sexuelle Dysfunktion und Inkontinenz, von Osteoporose, Glaukom und Gastroparese eingesetzt werden.

Außerdem können die erfindungsgemäßen Verbindungen zur Behandlung von primärem und sekundärem Raynaud-Phänomen, von Mikrozirkulationsstörungen, Claudicatio, peripheren und autonomen Neuropathien, diabetischen Mikroangiopathien, diabetischer Retinopathie, diabetischen Geschwüren an den Extremitäten, CREST-Syndrom, Erythematose, Onychomykose sowie von rheumatischen Erkrankungen verwendet werden.

Ferner eignen sich die erfindungsgemäßen Verbindungen zur Behandlung von Respiratory Distress-Syndromen und chronisch-obstruktiven Atemwegserkrankungen (COPD), von akuter und chronischer Niereninsuffizienz sowie zur Förderung der Wundheilung.

Die in der vorliegenden Erfindung beschriebenen Verbindungen stellen auch Wirkstoffe zur Bekämpfung von Krankheiten im Zentralnervensystem dar, die durch Störungen des NO/cGMP-Systems gekennzeichnet sind. Insbesondere sind sie geeignet zur Verbesserung der Wahrnehmung, Konzentrationsleistung, Lernleistung oder Gedächtnisleistung nach kognitiven Störungen, wie sie insbesondere bei Situationen/Krankheiten/Syndromen auftreten wie "Mild cognitive impairment", altersassoziierten Lern- und Gedächtnisstörungen, altersassoziierten Gedächtnisverlusten, vaskulärer Demenz, Schädel-Hirn-Trauma, Schlaganfall, Demenz, die nach Schlaganfällen auftritt ("post stroke dementia"), post-traumatischtim Schädel-Hirn-Trauma, allgemeinen Konzentrationsstörungen, Konzentrationsstörungen bei Kindern mit Lern- und Gedächtnisproblemen, Alzheimer'scher Krankheit, Demenz mit Lewy-Körperchen, Demenz mit Degeneration der Frontallappen einschliesslich des Pick's-Syndroms, Parkinson'scher Krankheit, progressiver nuclear palsy, Demenz mit corticobasaler Degeneration, Amyolateralsklerose (ALS), Huntington'scher Krankheit, Multipler Sklerose, Thalamischer Degeneration, Creutzfeld-Jacob-Demenz, HIV-Demenz, Schizophrenie mit Demenz oder Korsakoff-Psychose. Sie eignen sich auch zur Behandlung von Erkrankungen des Zentralnervensystems wie Angst-, Spannungs- und Depressionszuständen, zentral-nervös bedingten Sexualdysfunktionen und Schlafstörungen sowie zur Regulierung krankhafter Störungen der Nahrungs-, Genuss- und Suchtmittelaufnahme.

Weiterhin eignen sich die erfindungsgemäßen Verbindungen auch zur Regulation der cerebralen Durchblutung und stellen wirkungsvolle Mittel zur Bekämpfung von Migräne dar. Auch eignen sie sich zur Prophylaxe und Bekämpfung der Folgen cerebraler Infarktgeschehen (Apoplexia cerebri) wie Schlaganfall, cerebraler Ischämien und des Schädel-Hirn-Traumas. Ebenso können die erfindungsgemäßen Verbindungen zur Bekämpfung von Schmerzzuständen eingesetzt werden.

Zudem besitzen die erfindungsgemäßen Verbindungen antiinflammatorische Wirkung und können daher als entzündungshemmende Mittel eingesetzt werden.

Weiterer Gegenstand der vorliegenden Erfindung ist die Verwendung der erfindungsgemäßen Verbindungen zur Behandlung und/oder Prävention von Erkrankungen, insbesondere der zuvor genannten Erkrankungen.

Weiterer Gegenstand der vorliegenden Erfindung ist die Verwendung der erfindungsgemäßen Verbindungen zur Herstellung eines Arzneimittels zur Behandlung und/oder Prävention von Erkrankungen, insbesondere der zuvor genannten Erkrankungen.

Weiterer Gegenstand der vorliegenden Erfindung ist ein Verfahren zur Behandlung und/oder Prävention von Erkrankungen, insbesondere der zuvor genannten Erkrankungen, unter Verwendung einer wirksamen Menge von mindestens einer der erfindungsgemäßen Verbindungen.

Die erfindungsgemäßen Verbindungen können allein oder bei Bedarf in Kombination mit anderen Wirkstoffen eingesetzt werden. Weiterer Gegenstand der vorliegenden Erfindung sind Arzneimittel, enthaltend mindestens eine der erfindungsgemäßen Verbindungen und einen oder mehrere weitere Wirkstoffe, insbesondere zur Behandlung und/oder Prävention der zuvor genannten Erkrankungen. Als geeignete Kombinationswirkstoffe seien beispielhaft und vorzugsweise genannt:
- organische Nitrate und NO-Donatoren, wie beispielsweise Natriumnitroprussid, Nitroglycerin, Isosorbidmononitrat, Isosorbiddinitrat, Molsidomin oder SIN-1, sowie inhalatives NO;
- Verbindungen, die den Abbau von cyclischem Guanosinmonophosphat (cGMP) inhibieren, wie beispielsweise Inhibitoren der Phosphodiesterasen (PDE) 1, 2 und/oder 5, insbesondere PDE 5-Inhibitoren wie Sildenafil, Vardenafil und Tadalafil;
- NO-unabhängige, jedoch Häm-abhängige Stimulatoren der Guanylatcyclase, wie insbesondere die in WO 00/06568, WO 00/06569, WO 02/42301 und WO 03/095451 beschriebenen Verbindungen;
- antithrombotisch wirkende Mittel, beispielhaft und vorzugsweise aus der Gruppe der Thrombozytenaggregationshemmer, der Antikoagulantien oder der profibrinolytischen Substanzen;
- den Blutdruck senkende Wirkstoffe, beispielhaft und vorzugsweise aus der Gruppe der Calcium-Antagonisten, Angiotensin AII-Antagonisten, ACE-Hemmer, Endothelin-Antagonisten, Renin-Inhibitoren, alpha-Rezeptoren-Blocker, beta-Rezeptoren-Blocker, Mineralocorticoid-Rezeptor-Antagonisten sowie der Diuretika; und/oder
- den Fettstoffwechsel verändernde Wirkstoffe, beispielhaft und vorzugsweise aus der Gruppe der Thyroidrezeptor-Agonisten, Cholesterinsynthese-Inhibitoren wie beispielhaft und vorzugsweise HMG-CoA-Reduktase- oder Squalensynthese-Inhibitoren, der ACAT-Inhibitoren, CETP-Inhibitoren, MTP-Inhibitoren, PPAR-alpha-, PPAR-gamma- und/oder PPAR-delta-Agonisten, Cholesterin-Absorptionshemmer, Lipase-Inhibitoren, polymeren Gallensäureadsorber, Gallensäure-Reabsorptionshemmer und Lipoprotein(a)-Antagonisten.

Unter antithrombotisch wirkenden Mittel werden vorzugsweise Verbindungen aus der Gruppe der Thrombozytenaggregationshemmer, der Antikoagulantien oder der profibrinolytischen Substanzen verstanden.

Bei einer bevorzugten Ausführungsform der Erfindung werden die erfindungsgemäßen Verbindungen in Kombination mit einem Thrombozytenaggregationshemmer, wie beispielhaft und vorzugsweise Aspirin, Clopidogrel, Ticlopidin oder Dipyridamol, verabreicht.

Bei einer bevorzugten Ausführungsform der Erfindung werden die erfindungsgemäßen Verbindungen in Kombination mit einem Thrombin-Inhibitor, wie beispielhaft und vorzugsweise Ximelagatran, Melagatran, Bivalirudin oder Clexane, verabreicht.

Bei einer bevorzugten Ausführungsform der Erfindung werden die erfindungsgemäßen Verbindungen in Kombination mit einem GPIIb/IIIa-Antagonisten, wie beispielhaft und vorzugsweise Tirofiban oder Abciximab, verabreicht.

Bei einer bevorzugten Ausführungsform der Erfindung werden die erfindungsgemäßen Verbindungen in Kombination mit einem Faktor Xa-Inhibitor, wie beispielhaft und vorzugsweise BAY 59-7939, DU-176b, Fidexaban, Razaxaban, Fondaparinux, Idraparinux, PMD-3112, YM-150, KFA-1982, EMD-503982, MCM-17, MLN-1021, DX 9065a, DPC 906, JTV 803, SSR-126512 oder SSR-128428, verabreicht.

Bei einer bevorzugten Ausführungsform der Erfindung werden die erfindungsgemäßen Verbindungen in Kombination mit Heparin oder einem low molecular weight (LMW)-Heparin-Derivat verabreicht.

Bei einer bevorzugten Ausführungsform der Erfindung werden die erfindungsgemäßen Verbindungen in Kombination mit einem Vitamin K-Antagonisten, wie beispielhaft und vorzugsweise Coumarin, verabreicht.

Unter den Blutdruck senkenden Mitteln werden vorzugsweise Verbindungen aus der Gruppe der Calcium-Antagonisten, Angiotensin AII-Antagonisten, ACE-Hemmer, Endothelin-Antagonisten, Renin-Inhibitoren, alpha-Rezeptoren-Blocker, beta-Rezeptoren-Blocker, Mineralocorticoid-Rezeptor-Antagonisten sowie der Diuretika verstanden.

Bei einer bevorzugten Ausführungsform der Erfindung werden die erfindungsgemäßen Verbindungen in Kombination mit einem Calcium-Antagonisten, wie beispielhaft und vorzugsweise Nifedipin, Amlodipin, Verapamil oder Diltiazem, verabreicht.

Bei einer bevorzugten Ausführungsform der Erfindung werden die erfindungsgemäßen Verbindungen in Kombination mit einem alpha-1-Rezeptoren-Blocker, wie beispielhaft und vorzugsweise Prazosin, verabreicht.

Bei einer bevorzugten Ausführungsform der Erfindung werden die erfindungsgemäßen Verbindungen in Kombination mit einem beta-Rezeptoren-Blocker, wie beispielhaft und vorzugsweise Propranolol, Atenolol, Timolol, Pindolol, Alprenolol, Oxprenolol, Penbutolol, Bupranolol, Metipranolol, Nadolol, Mepindolol, Carazatol, Sotalol, Metoprolol, Betaxolol, Celiprolol, Bisoprolol, Carteolol, Esmolol, Labetalol, Carvedilol, Adaprolol, Landiolol, Nebivolol, Epanolol oder Bucindolol, verabreicht.

Bei einer bevorzugten Ausführungsform der Erfindung werden die erfindungsgemäßen Verbindungen in Kombination mit einem Angiotensin AII-Antagonisten, wie beispielhaft und vorzugsweise Losartan, Candesartan, Valsartan, Telmisartan oder Embursatan, verabreicht.

Bei einer bevorzugten Ausführungsform der Erfindung werden die erfindungsgemäßen Verbindungen in Kombination mit einem ACE-Hemmer, wie beispielhaft und vorzugsweise Enalapril, Captopril, Lisinopril, Ramipril, Delapril, Fosinopril, Quinopril, Perindopril oder Trandopril, verabreicht.

Bei einer bevorzugten Ausführungsform der Erfindung werden die erfindungsgemäßen Verbindungen in Kombination mit einem Endothelin-Antagonisten, wie beispielhaft und vorzugsweise Bosentan, Darusentan, Ambrisentan oder Sitaxsentan, verabreicht.

Bei einer bevorzugten Ausführungsform der Erfindung werden die erfindungsgemäßen Verbindungen in Kombination mit einem Renin-Inhibitor, wie beispielhaft und vorzugsweise Aliskiren, SPP-600 oder SPP-800, verabreicht.

Bei einer bevorzugten Ausführungsform der Erfindung werden die erfindungsgemäßen Verbindungen in Kombination mit einem Mineralocorticoid-Rezeptor-Antagonisten, wie beispielhaft und, vorzugsweise Spironolacton oder Eplerenon, verabreicht.

Bei einer bevorzugten Ausführungsform der Erfindung werden die erfindungsgemäßen Verbindungen in Kombination mit einem Diuretikum, wie beispielhaft und vorzugsweise Furosemid, verabreicht.

Unter den Fettstoffwechsel verändernden Mitteln werden vorzugsweise Verbindungen aus der Gruppe der CETP-Inhibitoren, Thyroidrezeptor-Agonisten, Cholesterinsynthese-Inhibitoren wie HMG-CoA-Reduktase- oder Squalensynthese-Inhibitoren, der ACAT-Inhibitoren, MTP-Inhibitoren, PPAR-alpha-, PPAR-gamma- und/oder PPAR-delta-Agonisten, Cholesterin-Absorptionshemmer, polymeren Gallensäureadsorber, Gallensäure-Reabsorptionshemmer, Lipase-Inhibitoren sowie der Lipoprotein(a)-Antagonisten verstanden.

Bei einer bevorzugten Ausführungsform der Erfindung werden die erfindungsgemäßen Verbindungen in Kombination mit einem CETP-Inhibitor, wie beispielhaft und vorzugsweise Torcetrapib (CP-529 414), JJT-705 oder CETP-vaccine (Avant), verabreicht.

Bei einer bevorzugten Ausführungsform der Erfindung werden die erfindungsgemäßen Verbindungen in Kombination mit einem Thyroidrezeptor-Agonisten, wie beispielhaft und vorzugsweise D-Thyroxin, 3,5,3'-Triiodothyronin (T3), CGS 23425 oder Axitirome (CGS 26214), verabreicht.

Bei einer bevorzugten Ausführungsform der Erfindung werden die erfindungsgemäßen Verbindungen in Kombination mit einem HMG-CoA-Reduktase-Inhibitor aus der Klasse der Statine, wie beispielhaft und vorzugsweise Lovastatin, Simvastatin, Pravastatin, Fluvastatin, Atorvastatin, Rosuvastatin, Cerivastatin oder Pitavastatin, verabreicht.

Bei einer bevorzugten Ausführungsform der Erfindung werden die erfindungsgemäßen Verbindungen in Kombination mit einem Squalensynthese-Inhibitor, wie beispielhaft und vorzugsweise BMS-188494 oder TAK-475, verabreicht.

Bei einer bevorzugten Ausführungsform der Erfindung werden die erfindungsgemäßen Verbindungen in Kombination mit einem ACAT-Inhibitor, wie beispielhaft und vorzugsweise Avasimibe, Melinamide, Pactimibe, Eflucimibe oder SMP-797, verabreicht.

Bei einer bevorzugten Ausführungsform der Erfindung werden die erfindungsgemäßen Verbindungen in Kombination mit einem MTP-Inhibitor, wie beispielhaft und vorzugsweise Implitapide, BMS-201038, R-103757 oder JTT-130, verabreicht.

Bei einer bevorzugten Ausführungsform der Erfindung werden die erfindungsgemäßen Verbindungen in Kombination mit einem PPAR-gamma-Agonisten, wie beispielhaft und vorzugsweise Pioglitazone oder Rosiglitazone, verabreicht.

Bei einer bevorzugten Ausführungsform der Erfindung werden die erfindungsgemäßen Verbindungen in Kombination mit einem PPAR-delta-Agonisten, wie beispielhaft und vorzugsweise GW 501516 oder BAY 68-5042, verabreicht.

Bei einer bevorzugten Ausführungsform der Erfindung werden die erfindungsgemäßen Verbindungen in Kombination mit einem Cholesterin-Absorptionshemmer, wie beispielhaft und vorzugsweise Ezetimibe, Tiqueside oder Pamaqueside, verabreicht.

Bei einer bevorzugten Ausführungsform der Erfindung werden die erfindungsgemäßen Verbindungen in Kombination mit einem Lipase-Inhibitor, wie beispielhaft und vorzugsweise Orlistat, verabreicht.

Bei einer bevorzugten Ausführungsform der Erfindung werden die erfindungsgemäßen Verbindungen in Kombination mit einem polymeren Gallensäureadsorber, wie beispielhaft und vorzugsweise Cholestyramin, Colestipol, Colesolvam, CholestaGel oder Colestimid, verabreicht.

Bei einer bevorzugten Ausführungsform der Erfindung werden die erfindungsgemäßen Verbindungen in Kombination mit einem Gallensäure-Reabsorptionshemmer, wie beispielhaft und vorzugsweise ASBT (= IBAT)-Inhibitoren wie z.B. AZD-7806, S-8921, AK-105, BARI-1741, SC-435 oder SC-635, verabreicht.

Bei einer bevorzugten Ausführungsform der Erfindung werden die erfindungsgemäßen Verbindungen in Kombination mit einem Lipoprotein(a)-Antagonisten, wie beispielhaft und vorzugsweise Gemcabene calcium (CI-1027) oder Nicotinsäure, verabreicht.

Weiterer Gegenstand der vorliegenden Erfindung sind Arzneimittel, die mindestens eine erfindungsgemäße Verbindung, üblicherweise zusammen mit einem oder mehreren inerten, nichttoxischen, pharmazeutisch geeigneten Hilfsstoffen enthalten, sowie deren Verwendung zu den zuvor genannten Zwecken.

Die erfindungsgemäßen Verbindungen können systemisch und/oder lokal wirken. Zu diesem Zweck können sie auf geeignete Weise appliziert werden, wie z.B. oral, parenteral, pulmonal, nasal, sublingual, lingual, buccal, rectal, dermal, transdermal, conjunctival, otisch oder als Implantat bzw. Stent.

Für diese Applikationswege können die erfindungsgemäßen Verbindungen in geeigneten Applikationsformen verabreicht werden.

Für die orale Applikation eignen sich nach dem Stand der Technik funktionierende, die erfindungsgemäßen Verbindungen schnell und/oder modifiziert abgebende Applikationsformen, die die erfindungsgemäßen Verbindungen in kristalliner und/oder amorphisierter und/oder gelöster Form enthalten, wie z.B. Tabletten (nicht-überzogene oder überzogene Tabletten, beispielsweise mit magensaftresistenten oder sich verzögert auflösenden oder unlöslichen Überzügen, die die Freisetzung der erfindungsgemäßen Verbindung kontrollieren), in der Mundhöhle schnell zerfallende Tabletten oder Filme/Oblaten, Filme/Lyophylisate, Kapseln (beispielsweise Hart- oder Weichgelatinekapseln), Dragees, Granulate, Pellets, Pulver, Emulsionen, Suspensionen, Aerosole oder Lösungen.

Die parenterale Applikation kann unter Umgehung eines Resorptionsschrittes geschehen (z.B. intravenös, intraarteriell, intrakardial, intraspinal oder intralumbal) oder unter Einschaltung einer Resorption (z.B. intramuskulär, subcutan, intracutan, percutan oder intraperitoneal). Für die parenterale Applikation eignen sich als Applikationsformen u.a. Injektions- und Infusionszubereitungen in Form von Lösungen, Suspensionen, Emulsionen, Lyophilisaten oder sterilen Pulvern.

Für die sonstigen Applikationswege eignen sich z.B. Inhalationsarzneiformen (u.a. Pulverinhalatoren, Nebulizer), Nasentropfen, -lösungen oder -sprays, lingual, sublingual oder buccal zu applizierende Tabletten, Filme/Oblaten oder Kapseln, Suppositorien, Ohren- oder Augenpräparationen, Vaginalkapseln, wäßrige Suspensionen (Lotionen, Schüttelmixturen), lipophile Suspensionen, Salben, Cremes, transdermale therapeutische Systeme (z.B. Pflaster), Milch, Pasten, Schäume, Streupuder, Implantate oder Stents.

Bevorzugt sind die orale oder parenterale Applikation, insbesondere die orale Applikation.

Die erfindungsgemäßen Verbindungen können in die angeführten Applikationsformen überführt werden. Dies kann in an sich bekannter Weise durch Mischen mit inerten, nichttoxischen, pharmazeutisch geeigneten Hilfsstoffen geschehen. Zu diesen Hilfsstoffen zählen u.a. Trägerstoffe (beispielsweise mikrokristalline Cellulose, Lactose, Mannitol), Lösungsmittel (z.B. flüssige Polyethylenglycole), Emulgatoren und Dispergier- oder Netzmittel (beispielsweise Natriumdodecylsulfat, Polyoxysorbitanoleat), Bindemittel (beispielsweise Polyvinylpyrrolidon), synthetische und natürliche Polymere (beispielsweise Albumin), Stabilisatoren (z.B. Antioxidantien wie beispielsweise Ascorbinsäure), Farbstoffe (z.B. anorganische Pigmente wie beispielsweise Eisenoxide) und Geschmacks- und/oder Geruchskorrigentien.

Im Allgemeinen hat es sich als vorteilhaft erwiesen, bei parenteraler Applikation Mengen von etwa 0.001 bis 1 mg/kg, vorzugsweise etwa 0.01 bis 0.5 mg/kg Körpergewicht zur Erzielung wirksamer Ergebnisse zu verabreichen. Bei oraler Applikation beträgt die Dosierung etwa 0.01 bis 100 mg/kg, vorzugsweise etwa 0.01 bis 20 mg/kg und ganz besonders bevorzugt 0.1 bis 10 mg/kg Körpergewicht.

Trotzdem kann es gegebenenfalls erforderlich sein, von den genannten Mengen abzuweichen, und zwar in Abhängigkeit von Körpergewicht, Applikationsweg, individuellem Verhalten gegenüber dem Wirkstoff, Art der Zubereitung und Zeitpunkt bzw. Intervall, zu welchem die Applikation erfolgt. So kann es in einigen Fällen ausreichend sein, mit weniger als der vorgenannten Mindestmenge auszukommen, während in anderen Fällen die genannte obere Grenze überschritten werden muss. Im Falle der Applikation größerer Mengen kann es empfehlenswert sein, diese in mehreren Einzelgaben über den Tag zu verteilen.

Die nachfolgenden Ausführungsbeispiele erläutern die Erfindung. Die Erfindung ist nicht auf die Beispiele beschränkt.

Die Prozentangaben in den folgenden Tests und Beispielen sind, sofern nicht anders angegeben, Gewichtsprozente; Teile sind Gewichtsteile. Lösungsmittelverhältnisse, Verdünnungsverhältnisse und Konzentrationsangaben von flüssig/flüssig-Lösungen beziehen sich jeweils auf das Volumen.

### A. Beispiele

### Abkürzungen:

- abs.: absolut
- aq.: wässrig
- Bsp.: Beispiel
- CI: chemische Ionisation (bei MS)
- DC: Dünnschichtchromatographie
- DCI: direkte chemische Ionisation (bei MS)
- DMF: Dimethylformamid
- DMSO: Dimethylsulfoxid
- d. Th.: der Theorie (bei Ausbeute)
- ee: Enantiomerenüberschuss
- EI: Elektronenstoß-Ionisation (bei MS)
- eq.: Äquivalent(e)
- ESI: Elektrospray-Ionisation (bei MS)
- GC: Gaschromatographie
- h: Stunde(n)
- HPLC: Hochdruck-, Hochleistungsflüssigchromatographie
- LC/MS: Flüssigchromatographie-gekoppelte Massenspektroskopie
- min: Minute(n)
- MS: Massenspektroskopie
- NMR: Kernresonanzspektroskopie
- R_{f}: Retentionsindex (bei DC)
- RT: Raumtemperatur
- Rₜ: Retentionszeit (bei HPLC)
- THF: Tetrahydrofuran
- UV: Ultraviolett-Spektroskopie
- v/v: Volumen zu Volumen-Verhältnis (einer Lösung)

### LC/MS-Methoden:

### Methode 1 (LC-MS)

Gerätetyp MS: Micromass ZQ; Gerätetyp HPLC: HP 1100 Series; UV DAD; Säule: Phenomenex Synergi 2µ Hydro-RP Mercury 20 mm x 4 mm; Eluent A: 11 Wasser + 0.5 ml 50%-ige Ameisensäure, Eluent B: 11 Acetonitril + 0.5 ml 50%-ige Ameisensäure; Gradient: 0.0 min 90% A → 2.5 min 30% A → 3.0 min 5% A → 4.5 min 5% A; Fluss: 0.0 min 1 ml/min → 2.5 min/3.0 min/4.5 min 2 ml/min; Ofen: 50°C; UV-Detektion: 210 nm.

### Methode 2 (LC-MS)

Gerätetyp MS: Micromass ZQ; Gerätetyp HPLC: Waters Alliance 2795; Säule: Phenomenex Synergi 2µ Hydro-RP Mercury 20 mm x 4 mm; Eluent A: 11 Wasser + 0.5 ml 50%-ige Ameisensäure, Eluent B: 11 Acetonitril + 0.5 ml 50%-ige Ameisensäure; Gradient: 0.0 min 90% A → 2.5 min 30% A → 3.0 min 5% A → 4.5 min 5% A; Fluss: 0.0 min 1 ml/min → 2.5 min/3.0 min/4.5 min 2 ml/min; Ofen: 50°C; UV-Detektion: 210 nm.

### Methode 3 (LC-MS)

Instrument: Micromass Platform LCZ mit HPLC Agilent Serie 1100; Säule: Phenomenex Synergi 2µ Hydro-RP Mercury 20 mm x 4 mm; Eluent A: 11 Wasser + 0.5 ml 50%-ige Ameisensäure, Eluent B: 11 Acetonitril + 0.5 ml 50%-ige Ameisensäure; Gradient: 0.0 min 90% A → 2.5 min 30% A → 3.0 min 5% A → 4.5 min 5% A; Fluss: 0.0 min 1 ml/min → 2.5 min/3.0 min/4.5 min 2 ml/min; Ofen: 50°C; UV-Detektion: 210 nm.

### Methode 4 (LC-MS)

Instrument: Micromass Quattro LCZ mit HPLC Agilent Serie 1100; Säule: Phenomenex Synergi 2µ Hydro-RP Mercury 20 mm x 4 mm; Eluent A: 11 Wasser + 0.5 ml 50%-ige Ameisensäure, Eluent B: 11 Acetonitril + 0.5 ml 50%-ige Ameisensäure; Gradient: 0.0 min 90% A→ 2.5 min 30% A → 3.0 min 5% A → 4.5 min 5% A; Fluss: 0.0 min 1 ml/min → 2.5 min/3.0 min/4.5 min 2 ml/min; Ofen: 50°C; UV-Detektion: 208-400 nm.

### Methode 5 (LC-MS)

Instrument: Micromass Platform LCZ mit HPLC Agilent Serie 1100; Säule: Thermo Hypersil GOLD 3µ 20 mm x 4 mm; Eluent A: 11 Wasser + 0.5 ml 50%-ige Ameisensäure, Eluent B: 11 Acetonitril + 0.5 ml 50%-ige Ameisensäure; Gradient: 0.0 min 100% A → 0.2 min 100% A → 2.9 min 30% A → 3.1 min 10% A → 5.5 min 10% A; Ofen: 50°C; Fluss: 0.8 ml/min; UV-Detektion: 210 nm.

### Methode 6 (LC-MS)

Gerätetyp MS: Micromass ZQ; Gerätetyp HPLC: Waters Alliance 2795; Säule: Merck Chromolith SpeedROD RP-18e 100 mm x 4.6 mm; Eluent A: Wasser + 500 µl 50%-ige Ameisensäure / l, Eluent B: Acetonitril + 500 µl 50%-ige Ameisensäure / l; Gradient: 0.0 min 10% B → 7.0 min 95% B → 9.0 min 95% B; Fluss: 0.0 min 1.0 ml/min → 7.0 min 2.0 ml/min → 9.0 min 2.0 ml/min; Ofen: 35°C; UV-Detektion: 210 nm.

### GC/MS-Methoden:

### Methode 1 (GC-MS)

Instrument: Micromass GCT, GC6890; Säule: Restek RTX-35MS, 30 m x 250 µm x 0.25 µm; konstanter Fluss mit Helium: 0.88 ml/min; Ofen: 60°C; Inlet: 250°C; Gradient: 60°C (0.30 min halten), 50°C/min → 120°C, 16°C/min → 250°C, 30°C/min → 300°C (1.7 min halten).

### Methode 2 (GC-MS)

Instrument: Micromass GCT, GC6890; Säule: Restek RTX-35MS, 30 m x 250 µm x 0.25 µm; konstanter Fluss mit Helium: 0.88 ml/min; Ofen: 60°C; Inlet: 250°C; Gradient: 60°C (0.30 min halten), 50°C/min → 120°C, 16°C/min → 250°C, 30°C/min → 300°C (8.7 min halten).

### HPLC-Methoden:

### Methode 1 (HPLC)

Instrument: HP 1100 mit DAD-Detektion; Säule: Kromasil 100 RP-18, 60 mm x 2.1 mm, 3.5 µm; Eluent A: 5 ml HClO₄ (70%-ig) / 1 Wasser, Eluent B: Acetonitril; Gradient: 0 min 2% B → 0.5 min 2% B → 4.5 min 90% B → 9 min 90% B → 9.2 min 2% B → 10 min 2% B; Fluss: 0.75 ml/min; Säulentemperatur: 30°C; UV-Detektion: 210 nm.

### Methode 2 (HPLC)

Instrument: HP 1100 mit DAD-Detektion; Säule: Kromasil 100 RP-18, 60 mm x 2.1 mm, 3.5 µm; Eluent A: 5 ml HClO₄ (70%-ig) / l Wasser, Eluent B: Acetonitril; Gradient: 0 min 2% B → 0.5 min 2% B → 4.5 min 90% B → 1 min 90% B → 15.2 min 2% B → 16 min 2% B; Fluss: 0.75 ml/min; Säulentemperatur: 30°C; UV-Detektion: 210 nm.

### Ausgangsverbindungen und Intermediate:

### Beispiel 1A

### (5-Brompentyl)benzol

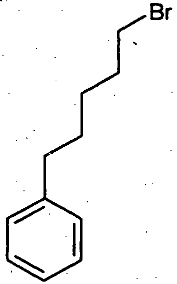

Eine Lösung von 416.7 ml (1.83 mol) 48%-iger Bromwasserstoffsäure wird mit 50 g (0.304 mol) 5-Phenylpentan-1-ol bei 0°C versetzt und 30 min bei 0°C nachgerührt. Anschließend wird die Reaktionslösung 12 Stunden bei 100°C gerührt. Nach vollständiger Umsetzung wird der Ansatz auf Raumtemperatur abgekühlt und mit 200 ml Essigsäureethylester versetzt. Nach Extraktion wird die organische Phase abgetrennt, mit gesättigter Natriumhydrogencarbonat-Lösung gewaschen und über Natriumsulfat getrocknet. Nach Filtration wird das Filtrat bis zur Trockene eingeengt. Das erhaltene Rohprodukt wird durch Flash-Chromatographie an Kieselgel (Laufmittel: Cyclohexan) gereinigt. Es werden 59.4 g (0.26 mol, 86% d. Th.) einer farblosen Flüssigkeit erhalten.
¹H-NMR (300 MHz, CDCl₃, δ/ppm): 7.32-7.22 (2H, m), 7.21-7.11 (3H, m), 3.40 (2H, t), 2.61 (2H, t), 1.97-1.81 (2H, m), 1.72-1.58 (2H, m), 1.56-1.39 (2H, m).
MS (CI): 226 (M⁺).

### Beispiel 2A

### [4-(2-Bromethyl)phenyl]methanol

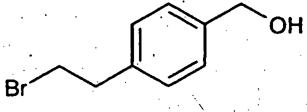

Eine Lösung von 2 g (8.73 mmol) 4-(2-Bromethyl)benzoesäure in 50 ml trockenem THF wird bei -10°C tropfenweise mit 13.1 ml (13.1 mmol) 1 M Boran-THF-Komplex versetzt. Nach Erwärmung auf Raumtemperatur wird der Ansatz eine Stunde nachgerührt. Nach vollständiger Umsetzung wird der Ansatz mit gesättigter Ammoniumchlorid-Lösung versetzt, in Essigsäureethylester aufgenommen, die organische Phase abgetrennt und über Natriumsulfat getrocknet. Nach Filtration wird das Lösungsmittel im Vakuum entfernt. Es werden 1.67 g (7.76 mmol, 79% d. Th.) eines farblosen Öls erhalten, das ohne weitere Reinigung in der nächsten Stufe eingesetzt wird.
¹H-NMR (300 MHz, DMSO-d₆, δ/ppm): 7.33-7.28 (4H, m), 5.14 (1H, t), 4.48 (2H, d), 3.77 (2H, t), 3.11 (2H,t).
MS (DCI, NH₃): 232 (M+NH₄⁺).

### Beispiel 3A

### 4-(2-Bromethyl)benzaldehyd

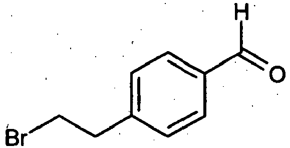

### Verfahren 1:

Eine Lösung von 200 mg (0.93 mmol) [4-(2-Bromethyl)phenyl]methanol in 20 ml Dichlormethan wird mit 240.5 mg (1.12 mmol) Pyridiniumchlorochromat (PCC) versetzt und 3 h bei Raumtemperatur gerührt. Anschließend wird die Reaktionslösung mit ca. 2 g Kieselgel versetzt und bis zur Trockene eingeengt. Der Rückstand wird durch Flash-Chromatographie an Kieselgel (Laufmittel: Cyclohexan/Essigsäureethylester 4:1) gereinigt. Es werden 183 mg (0.85 mmol, 82% d. Th.) eines farblosen Feststoffes erhalten.

### Verfahren 2:

Eine Lösung von 44.4 g (0.38 mol) Dichlormethyl-methylether in 230 ml Dichlormethan wird unter Kühlung (4-5°C) innerhalb von 10 min mit 42.26 ml Titantetrachlorid versetzt und 1 Stunde nachgerührt. Nachfolgend werden 64.89 g (0.34 mol) 2-Bromethylbenzol, gelöst in 24 ml Dichlormethan, bei 5-7°C innerhalb von 50 min zu der Reaktionslösung zudosiert. Anschließend erwärmt man die Reaktionslösung langsam auf Raumtemperatur und lässt den Ansatz über Nacht rühren. Nach vollständiger Umsetzung werden sehr vorsichtig innerhalb von 1 Stunde 140 ml Wasser zugetropft (Vorsicht: zunächst endotherme Reaktion durch Gasentwicklung, dann exotherme Reaktion bis 30°C, Kühlung erforderlich). Die Reaktionslösung wird dann dreimal mit Dichlormethan extrahiert, die vereinigten organischen Phasen mit 170 ml Wasser gewaschen, mit 115 ml Natriumhydrogencarbonat-Lösung neutralisiert und über Natriumsulfat getrocknet. Nach Filtration wird das Lösungsmittel im Vakuum entfernt. Der erhaltene Rückstand wird durch Flash-Chromatographie an Kieselgel (Laufmittel: Dichlormethan/Petrolether 1:2 → 1:1) gereinigt. Es werden 29.3 g (0.14 mol, 37% d. Th.) eines farblosen Feststoffes erhalten.
¹H-NMR (300 MHz, DMSO-d₆, δ/ppm): 9.99 (1H, s), 7.88 (2H, d), 7.52 (2H, d), 3.80 (2H, t), 3.24 (2H, t).
MS (EI): 212 (M⁺).

### Beispiel 4A

### 4-(2-Bromethyl)benzonitril

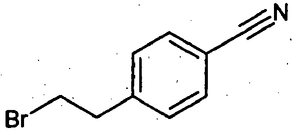

Eine Lösung von 29.3 g (0.14 mol) 4-(2-Bromethyl)benzaldehyd in 112.4 ml Ameisensäure wird mit 12.42 g (0.18 mol) Hydroxylamin-Hydrochlorid versetzt und 2 h unter Rückfluss erhitzt. Nach langsamem Abkühlen auf Raumtemperatur werden 670 ml Wasser zugesetzt und das Reaktionsgemisch langsam unter Kühlung mit 6 N Natronlauge neutralisiert. Anschließend wird dreimal mit Methyl-tert.-butylether extrahiert. Die vereinigten organischen Phasen werden über Magnesiumsulfat getrocknet und eingeengt. Der erhaltene Rückstand wird durch Flash-Chromatographie an Kieselgel (Laufmittel: Dichlormethan) gereinigt. Es werden 21.3 g (0.10 mol, 74% d. Th.) eines gelblichen Feststoffes erhalten.
¹H-NMR (400 MHz, DMSO-d₆, δ/ppm): 7.80 (2H, d), 7.51 (2H, d), 3.77 (2H, t), 3.22 (2H, t).
MS (DCI, NH₃): 227 (M+NH₄⁺).

### Beispiel 5A

### 2-(4-Methoxycarbonylbenzyl)malonsäurediallylester

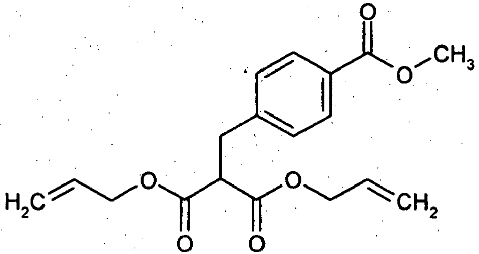

Eine Lösung von 56.7 g (0.3 mol) Malonsäurediallylester in 375 ml Dioxan und 75 ml THF wird bei 0°C portionsweise mit 14.42 g (0.36 mol) Natriumhydrid versetzt (Vorsicht: Wasserstoffentwicklung). Nach Erwärmen auf Raumtemperatur wird der Ansatz 1 h bei 40°C nachgerührt. Anschließend werden 111.88 g (0.6 mol) 4-Chlormethylbenzoesäuremethylester, gelöst in 375 ml Dioxan, langsam bei 40°C zugetropft und die Reaktionslösung dann über Nacht bei 110°C gerührt. Nach Abkühlen auf Raumtemperatur wird das Reaktionsgemisch auf 1200 ml Wasser gegeben. Dabei ist darauf zu achten, dass der pH-Wert < 7 ist (gegebenenfalls werden wenige ml 1 M Salzsäure bis ca. pH 2 zudosiert). Der Ansatz wird dann dreimal mit Essigsäureethylester extrahiert, die vereinigten organischen Phasen mit gesättigter Natriumchlorid-Lösung gewaschen und über Natriumsulfat getrocknet. Nach Filtration wird das Lösungsmittel im Vakuum bis zur Trockene eingeengt. Das erhaltene Rohprodukt wird durch Flash-Chromatographie (Laufmittel: Petrolether/ Essigsäureethylester 10:1; 3 kg Kieselgel) gereinigt. Es werden 85.4 g (0.26 mol 85% d. Th.) eines farblosen Feststoffs erhalten.
¹H-NMR (300 MHz, CDCl₃, δ/ppm): 7.96 (2H, d), 7.29 (2H, d), 5.91-5.74 (2H, m), 5.32-5.17 (4H, m), 4.59 (4H, d), 3.93 (3H, s), 3.74 (1H, t), 3.31 (2H, d).
MS (DCI): 349 (M+NH₄⁺).

### Beispiel 6A

### 2-[2-(4-Cyanophenyl)ethyl]-2-(4-methoxycarbonylbenzyl)malonsäurediallylester

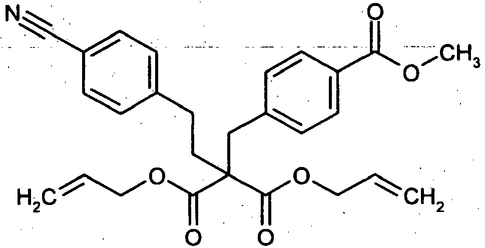

Eine Lösung von 55.71 g (0.17 mol) 2-(4-Methoxycarbonylbenzyl)malonsäurediallylester in 34 ml DMF wird bei 0°C portionsweise mit 6.70 g (0.17 mol) Natriumhydrid versetzt. Anschließen lässt man die Reaktionslösung auf Raumtemperatur kommen und rührt 1 Stunde nach. Danach wird die Reaktionslösung wieder auf 0°C abgekühlt, mit 42.98 g (0.20 mol) 4-(2-Bromethyl)benzonitril in 21 ml DMF versetzt und 30 min bei dieser Temperatur nachgerührt. Der Ansatz wird dann bei Raumtemperatur über Nacht gerührt. Das Reaktionsgemisch wird tropfenweise mit Wasser versetzt, dreimal mit Essigsäureethylester extrahiert, die vereinigten organischen Phasen mit gesättigter Kochsalz-Lösung gewaschen und über Natriumsulfat getrocknet. Nach Filtration wird das Lösungsmittel im Vakuum bis zur Trockene eingeengt. Das erhaltene Rohprodukt wird durch Flash-Chromatographie (Laufmittel: Petrolether/Essigsäureethylester 3:1; 3 kg Kieselgel) gereinigt. Es werden 36 g (78 mmol, 46% d. Th.) eines farblosen Feststoffes erhalten.
¹H-NMR (300 MHz, CDCl₃, δ/ppm): 7.95 (2H, d), 7.55 (2H, d), 7.21 (4H, t), 5.97-5.69 (2H, m), 5.40-5.23 (4H, m), 4.62 (4H, d), 3.92 (3H, s), 3.40 (2H, s), 2.72-2.61 (2H, m), 2.13-2.01 (2H, m).
MS (DCI): 479 (M+NH₄⁺).

### Beispiel 7A

### 4-[2-Carboxy-4-(4-cyanophenyl)butyl]benzoesäuremethylester

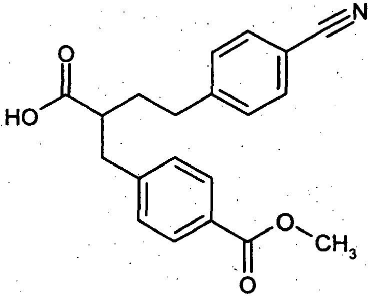

Eine Lösung von 43.5 g (0.09 mol) 2-[2-(4-Cyanophenyl)ethyl]-2-(4- methoxycarbonylbenzyl)malonsäurediallylester, 1.67 g (0.01 mol) Triphenylphosphin und 410 mg Palladiumacetat in 505 ml Dioxan wird bei Raumtemperatur mit einer Lösung von 41.8 ml (0.3 mol) Triethylamin und 8.6 ml (0.23 mol) Ameisensäure in 500 ml Dioxan versetzt. Das Reaktionsgemisch wird anschließend 2 h bei 100°C gerührt. Nach,vollständigem Umsatz wird die Reaktionslösung abgekühlt und das Lösungsmittel im Vakuum entfernt. Der erhaltene Rückstand wird durch Flash-Chromatographie an Kieselgel (Laufmittel: Dichlormethan/Methanol 50:1) gereinigt. Es werden 25 g (74 mmol, 82% d. Th.) eines farblosen Feststoffes erhalten.
¹H-NMR (400 MHz, DMSO-d₆, δ/ppm): 12.55-12.24 (1H, breit), 7.86 (2H, d), 7.72 (2H, d), 7.38 (2H, d), 7.32 (2H, d), 3.84 (3H, s), 2.99-2.81 (2H, m), 2.78-2.55 (3H, m), 1.90-1.67 (2H, m).
MS (ESI): 338 (M+H⁺).

### Beispiel 8A

### 4-[4-(4-Cyanophenyl)-2-hydroxymethyl-butyl]benzoesäuremethylester

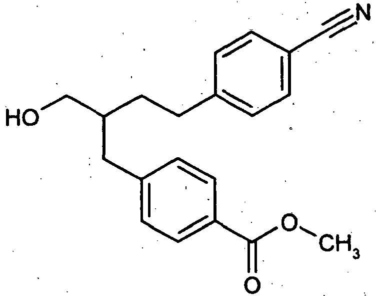

Zu einer Lösung von 4.2 g (12.98 mmol) 4-[2-Carboxy-4-(4-cyanophenyl)butyl]benzoesäuremethylester in 40 ml THF werden 26 ml (26 mmol) einer 1 M Boran-THF-Komplex-Lösung bei -15°C zugetropft und die Mischung 3 h bei dieser Temperatur nachgerührt. Danach werden nochmals 13 ml (13 mmol) 1 M Boran-THF-Komplex-Lösung zugetropft und weitere 30 min bei -15°C gerührt. Nach vollständiger Umsetzung wird das Reaktionsgemisch mit gesättigter Natriumhydrogencarbonat-Lösung versetzt und das Lösungsmittel bis zur Trockene eingeengt. Der Rückstand wird in Dichlormethan aufgenommen, über Natriumsulfat getrocknet und wiederum vom Lösungsmittel befreit. Das erhaltene Rohprodukt wird durch Flash-Chromatographie (Laufmittel: Essigsäureethylester/Petrolether 1:1; 150 g Kieselgel) gereinigt. Es werden 3.1 g (90% Reinheit, 83% d. Th.) eines farblosen Feststoffs erhalten.
¹H-NMR (300 MHz, DMSO-d₆, δ/ppm): 7.88 (2H, d), 7.71 (2H, d), 7.46 (4H, t), 4.54 (1H, t), 3.83 (3H, s), 3.41 (2H, t), 2.80-2.55 (4H, m), 1.79-1.39 (3H, m).
MS (ESI): 324 (M+H⁺).

### Beispiel 9A

### 4-[4-(4-Cyanophenyl)-2-formyl-butyl]benzoesäuremethylester

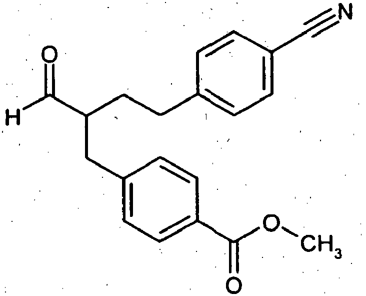

Eine Lösung von 5.7 g (17.63 mmol) 4-[4-(4-Cyanophenyl)-2-hydroxymethyl-butyl]benzoesäuremethylester in 250 ml Dichlormethan wird mit 4.56 g (21.15 mmol) Pyridiniumchlorochromat (PCC) versetzt und 5 Stunden bei Raumtemperatur gerührt. Nach vollständigem Umsatz werden ca. 10 g Kieselgel zugegeben und das Lösungsmittel im Vakuum bis zur Trockene entfernt. Der Rückstand wird durch Flash-Chromatographie an Kieselgel (Laufmittel: Cyclohexan/Essigsäureethylester 10:1 → 4:1) gereinigt. Es werden 4.16 g (12.94 mmol, 73% d. Th.) eines farblosen Feststoffs erhalten.
¹H-NMR (300 MHz, DMSO-d₆, δ/ppm): 9.68 (1H, s), 7.88 (2H, d), 7.73 (2H, d), 7.47 (4H, dd), 3.86 (3H, s), 3.14-3.02 (1H, m), 2.92-2.80 (1H, m), 2.78-2.54 (3H, m); 1.98-1.81 (1H, m), 1.76-1.60 (1H, m).
MS (DCI): 339 (M+NH₄⁺).

### Beispiel 10A

### E-4-[2-[2-(4-Cyanophenyl)ethyl]-4-(2-hydroxyphenyl)-but-3-enyl]benzoesäuremethylester

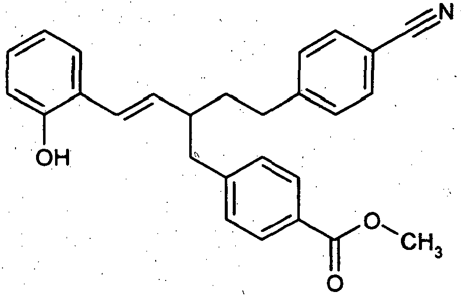

Zu einer Lösung von 1820 mg (4.05 mmol) (2-Hydroxybenzyl)-triphenylphosphoniumbromid in 50 ml wasserfreiem THF werden bei 0°C 5.9 ml (9.45 mmol) einer 1.6 M Lösung von n-Butyllithium in Hexan langsam hinzugefügt. Anschließend werden bei dieser Temperatur 1085 mg (3.38 mmol) 4-[4-(4-Cyanophenyl)-2-formyl-butyl]benzoesäuremethylester, gelöst in 40 ml THF, langsam zugegeben. Nach Erwärmung auf Raumtemperatur wird die Reaktionslösung 12 h nachgerührt, dann mit etwas Wasser versetzt und bis zur Trockene eingeengt. Der Rückstand wird in Essigsäureethylester aufgenommen, mit Wasser und gesättigter Natriumchlorid-Lösung gewaschen und über Natriumsulfat getrocknet. Nach Filtration wird das Lösungsmittel bis zur Trockene eingeengt. Das erhaltene Rohprodukt wird durch Flash-Chromatographie an Kieselgel (Laufmittel: Cyclohexan/ Essigsäureethylester 4:1 → 2:1) gereinigt. Es werden 1150 mg (2.79 mmol, 83% d. Th.) eines farblosen Feststoffs erhalten.
¹H-NMR (300 MHz, DMSO-d₆, δ/ppm): 9.39 (1H, s), 7.82 (2H, d), 7.60 (2H, d), 7.41-7.27 (5H, m), 7.01 (1H, t), 6.81-6.68 (2H, m), 6.45 (1H, d), 6.13-5.99 (1H, m), 3.81 (3H, s), 2.92-2.58 (5H, m), 1.86-1.56 (2H, m).
MS (DCI): 429 (M+NH₄⁺).

### Beispiel 11A

### 4-tert.-Butyl-2-chlor-1-methylbenzol

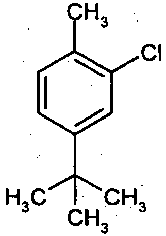

Eine Lösung von 2 g (13.49 mmol) 4-tert.-Butyltoluol wird mit 5.65 g (13.49 mmol) Benzyltri-methylammoniumtetrachloriodat versetzt und bei 70°C 24 Stunden lang gerührt. Nach Abkühlen wird der Niederschlag abgesaugt und das Filtrat eingeengt. Der erhaltene Rückstand wird über Kieselgel gereinigt (Laufmittel: Cyclohexan). Es werden 1.53 g (8.4 mmol) der Titelverbindung erhalten.
GC-MS (Methode 1): Rₜ = 5.27 min.
MS (EI): m/z = 182 (M)⁺.

### Beispiel 12A

### 2-Chlor-4-(tert.-butyl)benzylbromid

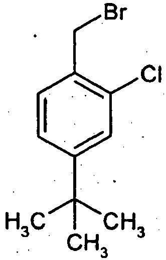

Zu einer Lösung von 2 g (10.95 mmol) 4-*tert*.-Butyl-2-chlor-1-methylbenzol in 10 ml Tetrachlormethan gibt man 1.75 g (9.85 mmol) *N*-Bromsuccinimid und 10.8 mg (0.006 mmol) 2,2'-Azobis-2-methylpropannitril und rührt die Mischung 4 Stunden unter Rückfluss nach. Nach Abkühlen wird der Ansatz eingeengt. Der Rückstand wird über Kieselgel-Flashchromatographie gereinigt (Laufmittel: Cyclohexan). Es werden 2.1 g (38% d. Th.) der Titelverbindung mit einer Reinheit von 52% erhalten.
GC-MS (Methode 1): Rₜ = 8.13 min.
MS (EI): m/z = 262 (M+H)⁺.

### Beispiel 13A

### 4-{(3E)-4-{2-[(4-tert.-Butyl-2-chlorbenzyl)oxy]phenyl}-2-[2-(4-cyanophenyl)ethyl]but-3-en-1-yl}benzoesäuremethylester

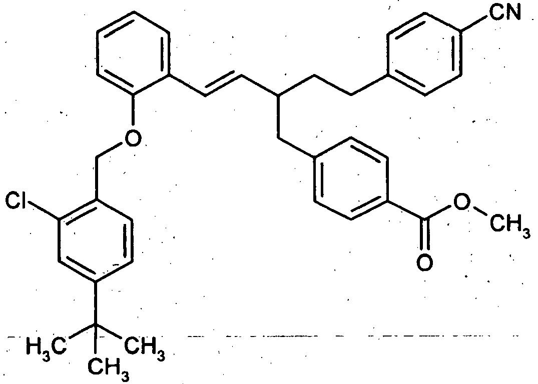

Eine Lösung von 450 mg (1.09 mmol) 4-[(3*E*)-2-[2-(4-Cyanophenyl)ethyl]-4-(2-hydroxyphenyl)-but-3-en-1-yl]benzoesäuremethylester in 10 ml trockenem Acetonitril wird mit 825.2 mg (1.64 mmol) 2-Chlor-4-(*tert*.-butyl)benzylbromid und 453.4 mg (3.28 mmol) wasserfreiem Kaliumcarbonat versetzt und 12 h unter Rückfluss erhitzt. Anschließend wird der Ansatz zur Trockene eingeengt. Der Rückstand wird in Essigsäureethylester aufgenommen, mit Wasser und gesättigter Natriumchlorid-Lösung gewaschen und über Natriumsulfat getrocknet. Die organische Phase wird eingeengt. Das erhaltene Rohprodukt wird über präparative HPLC gereinigt. Es werden 518 mg (0.87 mmol, 73.9% d. Th.) eines farblosen Schaums erhalten.
¹H-NMR (400 MHz, CDCl₃, δ/ppm): 7.91 (2H, d), 7.47 (2H, d), 7.43-7.34 (3H, m), 7.29-7.23 (1H, m), 7.23-7.14 (5H, m), 7.0-6.9 (2H, m), 6.67 (1H, d), 5.99 (1H, dd), 5.18-5.08 (2H, m), 3.88 (3H, s), 2.85-2.71 (3H, m), 2.66-2.54 (1H, m), 2.54-2.42 (1H, m), 1.85-1.75 (1H, m), 1.71-1.59 (1H, m), 1.31 (9H, s).
LC-MS (Methode 2): Rₜ = 3.46 min.
MS (ESIpos): m/z = 592 (M+H)⁺.

### Beispiel 14A

### 4-((3E)-4-{2-[(4-tert.-Butyl-2-chlorbenzyl)oxy]phenyl}-2-{2-[4-(1H-tetrazol-5-yl)phenyl]ethyl}-but-3-en-1-yl)benzoesäuremethylester

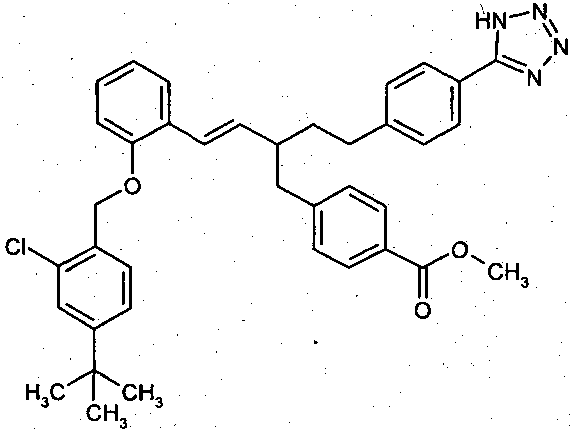

Eine Lösung von 478 mg (0.81 mmol) 4-{(3*E*)-4-{2-[(4-*tert*.-Butyl-2-chlorbenzyl)oxy]phenyl}-2-[2-(4-cyanophenyl)ethyl]but-3-en-1-yl}benzoesäuremethylester in 10 ml Toluol wird mit 1116 mg (9.69 mmol) Trimethylsilylazid und 351 mg (1.21 mmol) Di-n-butylzinnoxid versetzt und dann 12 h auf 80°C erhitzt. Nach Abkühlen auf Raumtemperatur wird der Ansatz mit gesättigter Natriumhydrogencarbonat-Lösung gewaschen. Die organische Phase wird abgetrennt, mit gesättigter Kochsalz-Lösung gewaschen und über Natriumsulfat getrocknet. Nach Filtration wird das Filtrat bis zur Trockene eingeengt. Das erhaltene Rohprodukt wird über eine Kieselgelsäule (Laufmittel: Cyclohexan/Essigsäureethylester 2:1 → 1:2) chromatographisch gereinigt. Es werden 144 mg (0.23 mmol, 28% d. Th.) eines weißen Schaums erhalten.
¹H-NMR (300 MHz, DMSO-d₆, δ/ppm): 7.9 (2H, d), 7.81 (2H, d), 7.48-7.24 (8H, m), 7.24-7.14 (1H, m), 7.06 (1H, m), 6.92 (1H, t), 6.48 (1H, d), 6.11 (1H, dd), 5.1 (2H, s), 3.79 (3H, s), 2.93-2.82 (1H, m), 2.80-2.57 (3H, m), 1.88-1.74 (1H, m), 1.74-1.57 (2H, m).
LC-MS (Methode 2): Rₜ = 3.28 min.
MS (ESIpos): m/z = 635 (M+H)⁺.

### Beispiel 15A

### 4-{(3E)-4-{2-[(4-tert.-Butylbenzyl)oxy]phenyl}-2-[2-(4-cyanophenyl)ethyl]but-3-en-1-yl}benzoe-säuremethylester

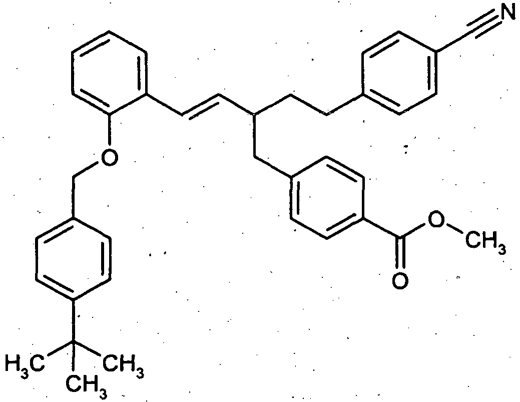

Eine Lösung von 2 g (3.6 mmol) *E*-4-[2-[2-(4-Cyanophenyl)ethyl]-4-(2-hydroxyphenyl)-but-3-en-yl]benzoesäuremethylester (Beispiel 17A) in 8 ml trockenem Acetonitril wird mit 2.20 g (9.71 mmol) 4-(tert.-Butyl)benzylbromid und 2.02 g (14.59 mmol) wasserfreiem Kaliumcarbonat versetzt und 12 Stunden unter Rückfluss erhitzt. Anschließend wird der Ansatz filtriert und das Filtrat zur Trockene eingeengt. Der Rückstand wird chromatographisch an Kieselgel (Laufmittel: Cyclo--hexan/Essigsäureethylester 10:1) gereinigt. Es werden 1.9 g (3.30 mmol, 97% Reinheit, 92% d. Th.) eines Öls isoliert.
¹H-NMR (400 MHz, CDCl₃, δ/ppm): 7.94-7.89 (2H, m), 7.49-7.30 (7H, m), 7.21-7.12 (5H, m), 6.97-6.90 (2H, m), 6.68-7.62 (1H, m), 5.06-5.02 (2H, m), 3.89 (3H, s), 2.83-2.69 (3H, m), 2.65-2.39 (2H, m), 1.86-1.59 (2H, m), 1.33 (9H, s).
LC-MS (Methode 2): Rₜ 3.37 min; m/z 575 (M+NH₄⁺), 557 (M⁺).

### Beispiel 16A

### 4-((3E)-4-{2-[(4-tert.-Butylbenzyl)oxy]phenyl}-2-{2-(4-(1H-tetrazol-5-yl)phenyl]ethyl}but-3-en-1-yl)benzoesäuremethylester

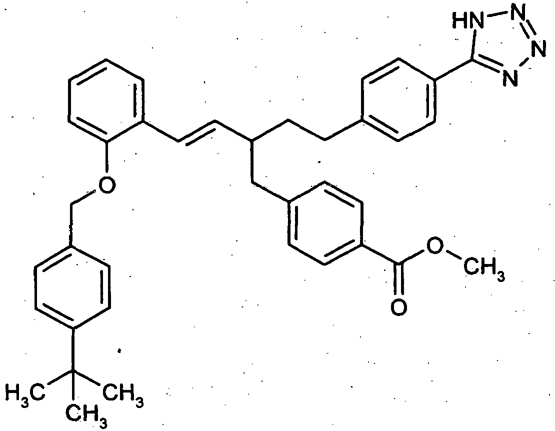

Eine Lösung von 1000 mg (1.79 mmol) 4-{(3*E*)-4-{2-[(4-*tert*.-Butylbenzyl)oxy]phenyl}-2-[2-(4-cyanophenyl)ethyl]but-3-en-1-yl}benzoesäuremethylester in 10 ml Toluol wird mit 2851 mg (24.7 mmol) Trimethylsilylazid und 618 mg (2.47 mmol) Di-*n*-butylzinnoxid versetzt und 12 h auf 80°C erhitzt. Nach Abkühlen auf Raumtemperatur wird der Ansatz mit gesättigter Natriumhydrogencarbonat-Lösung gewaschen. Die organische Phase wird abgetrennt, mit gesättigter Kochsalz-Lösung gewaschen und über Natriumsulfat getrocknet. Nach Filtration wird das Filtrat bis zur Trockene eingeengt. Das erhaltene Rohprodukt wird über eine Kieselgelsäule (Laufmittel: Cyclohexan/Essigsäureethylester 2:1 → 1:2) chromatographisch gereinigt. Es werden 648 mg (1.08 mmol, 60% d. Th.) eines farblosen Öls erhalten.
¹H-NMR (400 MHz, DMSO-d₆, δ/ppm): 16.75 (1H, breit), 7.92 (2H, d), 7.83 (2H, d), 7.42-7.25 (9H, m), 7.18 (1H, t), 7.04 (1H, d), 6.9 (1 H, t), 6.5 (1H, d), 6.12 (1H, dd), 5.05 (2H, s), 3.8 (3 H, s), 2.93-2.86 (1H, m), 2.8-2.7 (2H, m), 2.69-2.59 (1H, m), 1.89-1.78 (1H, m), 1.75-1.58 (2H, m), 1.22 (9H, s).

### Beispiel 17A

### 4-{(3E)-4-{2-[(2-Chlorbenzyl)oxy]phenyl}-2-[2-(4-cyanophenyl)ethyl]but-3-en-1-yl}benzoesäuremethylester

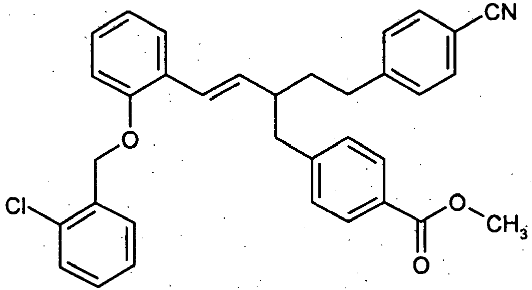

Eine Lösung von 200 mg (0.49 mmol) 4-[(3*E*)-2-[2-(4-Cyanophenyl)ethyl]-4-(2-hydröxyphenyl)-but-3-en-1-yl]benzoesäuremethylester in 10 ml trockenem Acetonitril wird mit 149.8 mg (0.73 mmol) 2-Chlorbenzylbromid und 201 mg (1.46 mmol) wasserfreiem Kaliumcarbonat versetzt und 12 Stunden unter Rückfluss erhitzt. Anschließend wird der Ansatz zur Trockene eingeengt. Der Rückstand wird in Essigsäureethylester aufgenommen, mit Wasser und gesättigter Natriumchlorid-Lösung gewaschen und über Natriumsulfat getrocknet. Die organische Phase wird eingeengt. Das erhaltene Rohprodukt wird über präparative HPLC gereinigt. Es werden 94 mg (0.17 mmol, 36% d. Th.) eines farblosen Schaums erhalten.
LC-MS (Methode 2): Rₜ = 3.28 min.
MS (ESIpos): m/z = 536 [M+H⁺].

### Beispiel 18A

### 4-((3E)-4-{2-[(2-Chlorbenzyl)oxy]phenyl}-2-{2-[4-(1H-tetrazol-5-yl)phenyl]ethyl}but-3-en-1-yl)-benzoesäuremethylester

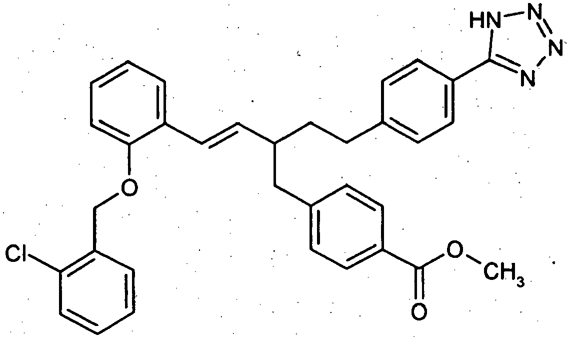

Eine Lösung von 94 mg (0.18 mmol) 4-{(3*E*)-4-{2-[(2-Chlorbenzyl)oxy]phenyl}-2-[2-(4-cyanophenyl)ethyl]but-3-en-1-yl}benzoesäuremethylester in 2 ml Toluol wird mit 303 mg (2.63 mmol) Trimethylsilylazid und 65.7 mg (0.26 mmol) Di-n-butylzinnoxid versetzt und 12 h auf 80°C erhitzt. Nach Abkühlen auf Raumtemperatur wird der Ansatz mit gesättigter Natriumhydrogencarbonat-Lösung gewaschen. Die organische Phase wird abgetrennt, mit gesättigter Kochsalz-Lösung gewaschen und über Natriumsulfat getrocknet. Nach Filtration wird das Filtrat bis zur Trockene eingeengt. Das erhaltene Rohprodukt wird über eine Kieselgelsäule (Laufmittel: Cyclohexan/Essigsäureethylester 1:1) chromatographisch gereinigt. Es werden 95 mg (0.16 mmol, 78.4% d. Th.) eines farblosen Schaums erhalten.
¹H-NMR (300 MHz, CDCl₃, δ/ppm): 7.83 (2H, d), 7.79 (2H, d), 7.52-7.38 (4H, m), 7.24-7.1 (6H, m), 7.0-6.9 (2H, m), 6.71 (1H, d), 5.06 (1H, dd), 5.18 (2H, s), 3.92 (3H, s), 2.87-2.51 (5H, m), 2.5-2.35 (1H, m).

### Beispiel 19A

### 4-[(3E)-2-[2-(4-Cyanophenyl)ethyl]-4-(2-{[2-(trifluormethyl)benzyl]oxy}phenyl)but-3-en-1-yl]-benzoesäuremethylester

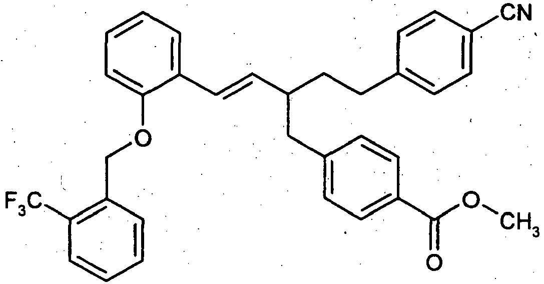

Eine Lösung von 438 mg (1.02 mmol) 4-[(3*E*)-2-[2-(4-Cyanophenyl)ethyl]-4-(2-hydroxyphenyl)-but-3-en-1-yl]benzoesäuremethylester in 20 ml trockenem Acetonitril wird mit 382 mg (1.6 mmol) 2-Trifluormethylbenzylbromid und 441.3 mg (3.19 mmol) wasserfreiem Kaliumcarbonat versetzt und 12 Stunden unter Rückfluss erhitzt. Anschließend wird der Ansatz zur Trockene eingeengt. Der Rückstand wird in Essigsäureethylester aufgenommen, mit Wasser und gesättigter Natriumchlorid-Lösung gewaschen und über Natriumsulfat getrocknet. Die organische Phase wird eingeengt. Das erhaltene Rohprodukt wird über präparative HPLC gereinigt. Es werden 181 mg (0.32 mmol, 31.3% d. Th.) eines farblosen Schaums erhalten.
¹H-NMR (300 MHz, CDCl₃, δ/ppm): 7.91 (2H, d), 7.71 (1H, d), 7.62 (1H, d), 7.53 (1H, t), 7.47 (3H, d), 7.4 (1H, d), 7.22-7.15 (5H, m), 6.98 (1H, t), 6.88 (1H, d), 6.66 (1H, d), 6.0 (1H, dd), 5.28 (2H, s), 3.88 (3H, s), 2.82-2.71 (3H, m), 2.68-2.42 (2H, m), 1.89-1.72 (1H, m), 1.72-1.62 (1H, m).

### Beispiel 20A

### 4-[(3E)-2-{2-[4-(1H-Tetrazol-5-yl)phenyl]ethyl}-4-(2-{[2-(trifluormethyl)benzyl]oxy}phenyl)but-3-en-1-yl]benzoesäuremethylester

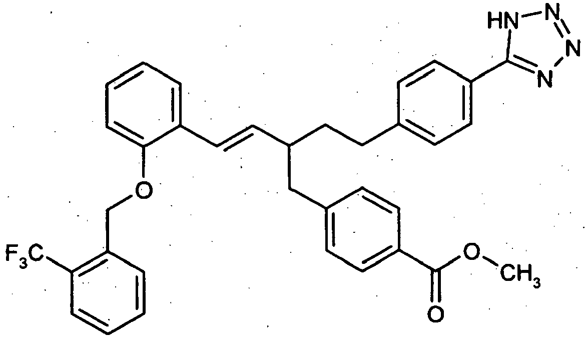

Eine Lösung von 212 mg (0.26 mmol) 4-[(3*E*)-2-[2-(4-Cyanophenyl)ethyl]-4-(2-{[2-(trifluormethyl)benzyl]oxy}phenyl)but-3-en-1-yl]benzoesäuremethylester in 3 ml Toluol wird mit 450 mg (3.91 mmol) Trimethylsilylazid und 97.3 mg (0.39 mmol) Di-n-butylzinnoxid versetzt und 12 Stunden auf 80°C erhitzt. Nach Abkühlen auf Raumtemperatur wird der Ansatz mit gesättigter Natriumhydrogencarbonat-Lösung gewaschen. Die organische Phase wird abgetrennt, mit gesättigter Kochsalz-Lösung gewaschen und über Natriumsulfat getrocknet. Nach Filtration wird das Filtrat bis zur Trockene eingeengt. Das erhaltene Rohprodukt wird über eine Kieselgelsäule (Laufmittel: Cyclohexan/Essigsäureethylester 1:1) chromatographisch gereinigt. Es werden 120 mg. (0.19 mmol, 75% d._Th.) eines farblosen Schaums erhalten.
LC-MS (Methode 1): Rₜ = 3.23 min.
MS (ESIpos): m/z = 613 (M+H)⁺.

### Beispiel 21A

### [2-(5-Phenylpentyloxy)phenyl]methanol

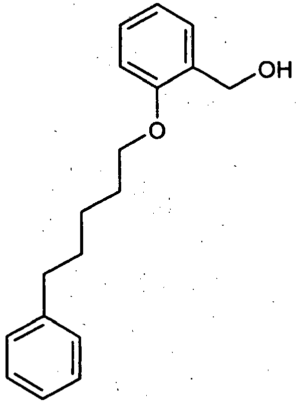

Eine Lösung von 10 g (80.56 mmol) 2-Hydroxybenzylalkohol in 200 ml trockenem Acetonitril wird mit 27.45 g (120.83 mmol) (5-Brompentyl)benzol und 12.25 g (88.61 mmol) wasserfreiem Kaliumcarbonat versetzt und 12 Stunden unter Rückfluss erhitzt. Anschließend wird der Ansatz zur Trockene eingeengt. Der Rückstand wird in Essigsäureethylester aufgenommen, mit Wasser und gesättigter Natriumchlorid-Lösung gewaschen und über Natriumsulfat getrocknet. Die organische Phase wird eingeengt. Das erhaltene Rohprodukt wird durch Flash-Chromatographie an Kieselgel (Laufmittel: Cyclohexan/Essigsäureethylester 10:1) gereinigt. Es werden 18.7 g (81% d. Th.) eines farblosen Feststoffs erhalten.
¹H-NMR (300 MHz, DMSO-d₆, δ/ppm): 7.38 (1H, d), 7.31-7.10 (6H, m), 6.91 (2H, t), 4.92 (1H, t), 4.50 (2H, d), 3.95 (2H, t), 2.59 (2H, t), 1.81-1.68 (2H, m), 1.67-1.55 (2H, m), 1.52-1.36 (2H, m).
MS (CI): 288 (M+NH₄⁺), 270 (M⁺).

### Beispiel 22A

### Triphenyl-[2-(5-phenylpentyloxy)benzyl]-phosphoniumbromid

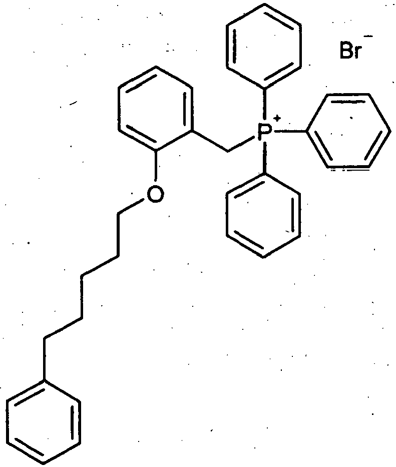

Eine Lösung von 18.7 g (69.16 mmol) [2-(5-Phenylpentyloxy)phenyl]methanol in 120 ml Acetonitril wird mit 22.55 g (65.71 mmol) Triphenylphosphoniumbromid versetzt und 3 Stunden unter Rückfluss erhitzt. Anschließend wird die Reaktionslösung bis zur Trockene eingeengt. Es werden 36.6 g (61.45 mmol, 83% d. Th.) kristallines Produkt erhalten, welches ohne weitere Reinigung umgesetzt wird.
¹H-NMR (400 MHz, DMSO-d₆, δ/ppm): 7.89 (3H, t), 7.78-7.66 (6H, m), 7.64-7.52 (6H, m), 7.32-7.24 (3H, m), 7.21-7.12 (3H, m), 7.01 (1H, d), 6.89-6.77 (2H, m), 4.90 (2H, d), 3.44 (2H, t), 2.56 (2H, t), 1.59-1.46 (2H, m), 1.38-1.25 (2H, m), 1.23-1.12 (2H, m).
MS (ESI): 515 (M⁺-Br).

### Beispiel 23A

### 4-((3E/Z)-2-[2-(4-Cyanophenyl)ethyl]-4-{2-[(5-phenylpentyl)oxy]phenyl}but-3-en-1-yl)benzoe-säuremethylester

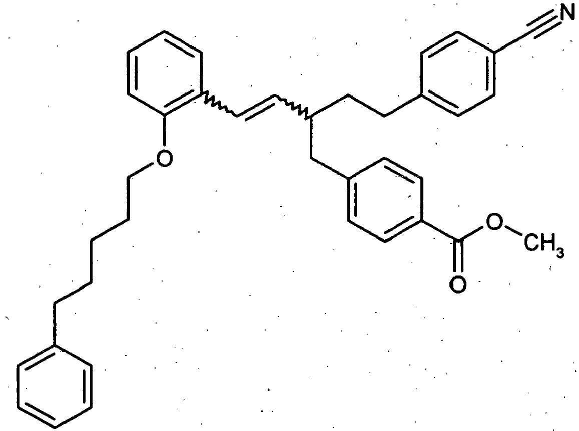

Zu einer Lösung von 1.8 g (3.02 mmol) Triphenyl-{2-[(5-phenylpentyl]xy]benzyl}-phosphoniumbromid in 30 ml THF werden bei 0°C 2.36 ml (3.78 mmol) einer 1.6 M Lösung von n-Butyllithium in Hexan langsam hinzugefügt. Anschließend werden bei dieser Temperatur 810 mg (2.52 mmol) 4-[4-(4-Cyanophenyl)-2-formyl-butyl]benzoesäuremethylester in 10 ml THF schnell zudosiert und die Mischung 1 h bei 0°C nachgerührt. Nach Erwärmung auf Raumtemperatur wird die Reaktionslösung weitere 3 h nachgerührt, dann mit Ammoniumchlorid-Lösung versetzt und bis zur Trockene eingeengt. Der Rückstand wird in Essigsäureethylester aufgenommen, mit Wasser und gesättigter Kochsalz-Lösung gewaschen und über Natriumsulfat getrocknet. Nach Filtration wird das Lösungsmittel bis zur Trockene eingeengt. Das erhaltene Rohprodukt wird durch Flash-Chromatographie (Laufmittel: Cyclohexan/Essigsäureethylester 10:1) gereinigt. Es werden 1.27 g (2.28 mmol, 90% d. Th.) der Titelverbindung in Form eines festen Schaums erhalten.
¹H-NMR (300 MHz, CDCl₃, δ/ppm): (*E*/*Z* = 3.6:1) 7.9 (1.6H; d), 7.8 (0.36H, d), .7.52 (1.6H, d), 7.44 (0.36H, d), 7.37-7.31 (1H, m), 7.31-7.02 (9H, m), 6.99-6.62 (3H, m), 6.54 (1H, d), 6.01-5.89 (1.6H, m), 5.45-5.36 (0.36H, m), 4.0-3.91 (2H, t), 3.9 (3H, s), 2.88-2.7 (3H, m), 2.7-2.55 (4H, m), 2.54-3.39 (1H, m), 1.9-1.6 (7H, m), 1.59-1.45 (2H, m).
MS (DCI): m/z = 575 (M+NH₄)⁺.

### Beispiel 24A

### 4-((3E/Z)-4-{2-[(5-Phenylpentyl)oxy]phenyl}-2-{2-[4-(1H-tetrazol-5-yl)phenyl]ethyl}but-3-en-1-yl)benzoesäuremethylester

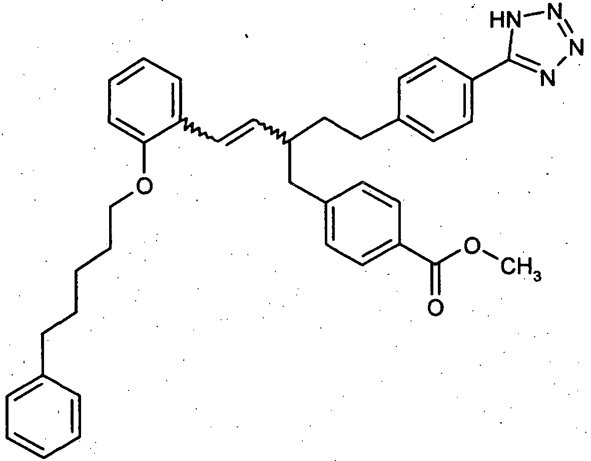

Eine Lösung von 160 mg (0.287 mmol) 4-((3*E*/*Z*)-2-[2-(4-Cyanophenyl)ethyl]-4-{2-[(5-phenyl-pentyl)oxy]phenyl)but-3-en-1-yl)benzoesäuremethylester in 5 ml Toluol wird mit 0.57 ml (4.3 mmol) Trimethylsilylazid und 107 mg (0.43 mmol) Di-n-butylzinnoxid versetzt und 12 Stunden auf 80°C erhitzt. Nach Abkühlen auf Raumtemperatur wird der Ansatz mit gesättigter Natritim-hydrogencarbonat-Lösung gewaschen. Die organische Phase wird abgetrennt, mit gesättigter Kochsalz-Lösung gewaschen und über Natriumsulfat getrocknet. Nach Filtration wird das Filtrat bis zur Trockene eingeengt. Das erhaltene Rohprodukt wird durch Flash-Chromatographie an Kieselgel (Laufmittel: Dichlormethan → Dichlormethan/Methanol 10:1) gereinigt. Es werden 138 mg (0.23 mmol, 73.6% d. Th.) eines farblosen Öls erhalten.
LC-MS (Methode 4): Rₜ = 3.37 min.
MS (ESIpos): m/z = 601 (M+H)⁺.

### Beispiel 25A

### 2-(4-Cyanobenzyl)malonsäurediallylester

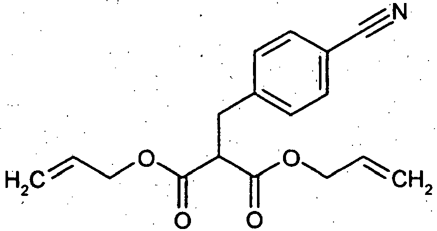

Eine Lösung von 121.5 g (659 mmol) Malonsäurediallylester in 1.5 Liter Dioxan wird bei 0°C portionsweise mit 19.79 g (494 mmol) Natriumhydrid versetzt (Vorsicht: Wasserstoffentwicklung). Nach Erwärmen auf Raumtemperatur wird der Ansatz 1 h bei 40°C nachgerührt. Anschließend werden 50 g (0.329 mol) 4-Chlormethylbenzonitril, gelöst in 500 ml Dioxan, langsam bei 40°C zugetropft und die Reaktionslösung dann über Nacht bei 110°C gerührt. Nach Abkühlen auf Raumtemperatur wird das Reaktionsgemisch auf 1200 ml Wasser gegeben. Dabei ist darauf zu achten, dass der pH-Wert < 7 ist (gegebenenfalls werden wenige ml 1 M Salzsäure bis ca. pH 2 zudosiert). Der Ansatz wird dann dreimal mit Essigsäureethylester extrahiert, die vereinigten organischen Phasen mit gesättigter Natriumchlorid-Lösung gewaschen und über Natriumsulfat getrocknet. Nach Filtration wird das Lösungsmittel im Vakuum bis zur Trockene entfernt. Anschließend wird durch Hochvakuum-Destillation überschüssiger Malonsäurediallylester entfernt (Siedepunkt: 57°C, 0.074 mbar). Der Destillationsrückstand wird durch Flash-Chromatographie an Kieselgel (Laufmittel: Petrolether/Essigsäureethylester 20:1) gereinigt. Es werden 67 g (0.22 mol, 67% d. Th.) eines farblosen Feststoffs erhalten.
¹H-NMR (300 MHz, DMSO-d₆, δ/ppm): 7.77 (2H, d), 7.48 (2H, d), 5.90-5.73 (2H, m), 5.29-5.13 (4H, m), 4.64-4.50 (4H, m), 4.09 (1H, t), 3.21 (2H, d).
MS (DCI): 317 (M+NH₄⁺).

### Beispiel 26A

### 2-(4-Cyanobenryl)-2-[2-(4-methoxycarbonylphenyl)ethyl]malonsäurediallylester

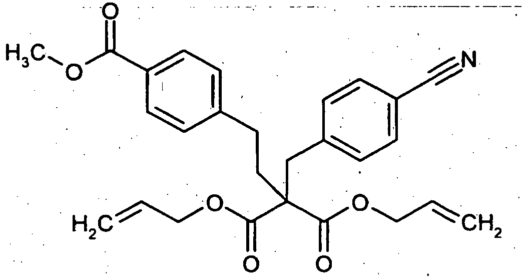

Eine Lösung von 48.53 g (162.14 mmol) 2-(4-Cyanobenzyl)malonsäurediallylester in 180 ml DMF wird bei 0°C portionsweise mit 7.13 g (178.36 mmol) Natriumhydrid versetzt. Anschließend lässt man die Reaktionslösung auf Raumtemperatur kommen und rührt 30 min nach. Danach wird die Reaktionslösung wieder auf 0°C abgekühlt, mit 55 g (194.6 mmol) 4-(2-Bromethyl)benzoesäure-methylester [CAS-Nr. 136333-97-6] in 195 ml DMF versetzt und 30 min bei dieser Temperatur nachgerührt. Der Ansatz wird dann bei Raumtemperatur über Nacht gerührt. Das Reaktionsgemisch wird tropfenweise mit Wasser versetzt, mit Essigsäureethylester dreimal extrahiert, die vereinigten organischen Phasen mit gesättigter Natriumchlorid-Lösung gewaschen und über Natrium-sulfat getrocknet. Nach Filtration wird das Lösungsmittel im Vakuum bis zur Trockene eingeengt. Das erhaltene Rohprodukt wird durch Flash-Chromatographie an Kieselgel (Laufmittel: Petrolether/Essigsäureethylester 10:1) gereinigt. Es werden 33.4 g (72.37 mol, 44% d. Th.) eines farblosen Feststoffs erhalten.
¹H-NMR (300 MHz, CDCl₃, δ/ppm): 7.89 (2H, d), 7.79 (2H, d), 7.38 (2H, d), 7.32 (2H, d), 5.97-5.81 (2H, m), 5.38-5.20 (4H, m), 4.61 (4H, d), 3.82 (3H, s), 3.39 (2H, s), 2.77-2.61 (2H, m), 1.99-1.84 (2H, m).
MS (DCI): 479 (M+NH₄⁺).

### Beispiel 27A

### 4-[3-Carboxy-4-(4-cyanophenyl)butyl]benzoesäuremethylester

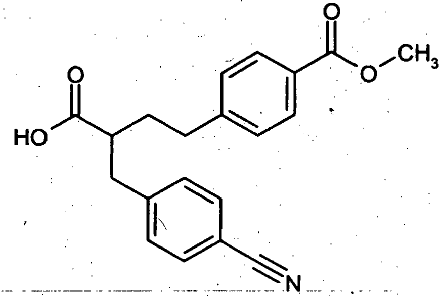

Eine Lösung von 7.5 g (16.25 mmol) 2-(4-Cyanobenzyl)-2-[2-(4-methoxycarbonyl-phenyl)ethyl]-malonsäurediallylester, 0.3 g (1.14 mmol) Triphenylphosphin und 70 mg Palladiumacetat in 170 ml Dioxan wird bei Raumtemperatur mit einer Lösung von 7.5 ml (53.6 mmol) Triethylamin und 1.5 ml (40.6 mmol) Ameisensäure in 170 ml Dioxan versetzt. Das Reaktionsgemisch wird anschließend 2 h bei 100°C gerührt. Nach vollständigem Umsatz wird die Reaktionslösung abgekühlt und das Lösungsmittel im Vakuum entfernt. Der Rückstand wird in Essigsäureethylester und Wasser aufgenommen, mit 1 N Salzsäure angesäuert und die organische Phase abgetrennt. Die wässrige Phase wird noch dreimal mit Essigsäureethylester extrahiert, die organischen Phasen werden anschließend vereinigt, mit gesättigter Natriumchlorid-Lösung gewaschen und über Natriumsulfat getrocknet. Nach Filtration wird die Lösung im Vakuum eingeengt. Es werden 5.48 g (89% d. Th., 90% Reinheit) eines farblosen Feststoffes erhalten.
¹H-NMR (400 MHz, DMSO-d₆, δ/ppm): 12.46-12.29 (1H, breit), 7.88 (2H, d), 7.74 (2H, d), 7.39 (2H, d), 7.31 (2H, d), 3.83 (3H, s), 2.99-2.83 (2H, m), 2.79-2.56 (3H, m), 1.93-1.67 (2H, m).
MS (DCI): 355 (M+NH₄⁺).

### Beispiel 28A

### 4-[3-(4-Cyanobenzyl)-4-hydroxybutyl]benzoesäuremethylester

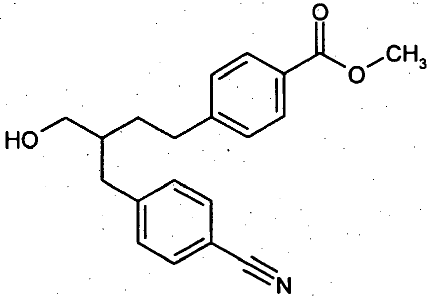

Zu einer Lösung von 8 g (23.71 mmol) 4-[3-Carboxy-4-(4-cyanophenyl)butyl]benzoesäuremethylester in 200 ml THF werden 47.43 ml (47.43 mmol) einer 1 M Boran-THF-Komplex-Lösung bei -10°C zugetropft. Nach Erwärmen auf -5°C wird bei dieser Temperatur noch 4 h nachgerührt. Nach vollständiger Umsetzung wird das Reaktionsgemisch mit gesättigter Natriumhydrogencarbonat-Lösung versetzt und das Lösungsmittel bis zur Trockene eingeengt. Der Rückstand wird in Dichlormethan aufgenommen, über Natriumsulfat getrocknet und wiederum vom Lösungsmittel -befreit. Das -erhaltene Rohprodukt wird durch Flash-Chromatographie an Kieselgel (Laufmittel: Essigsäureethylester/Cyclohexan 1:10) gereinigt. Es werden 5.8 g (98% Reinheit, 74% d. Th.) eines farblosen Feststoffs erhalten.
¹H-NMR (300 MHz, DMSO-d₆, δ/ppm): 7.86 (2H, d), 7.73 (2H, d), 7.38 (2H, d), 7.30 (2H, d), 4.60 (1H, t), 3.83 (3H, s), 3.32 (2H, t), 2.81-2.57, (4H, m), 1.79-1.56 (2H, m), 1.54-1.39 (1H, m).
MS (DCI): 341 (M+NH₄⁺).

### Beispiel 29A

### 4-[3-(4-Cyanobenzyl)-4-oxo-butyl]benzoesäuremethylester

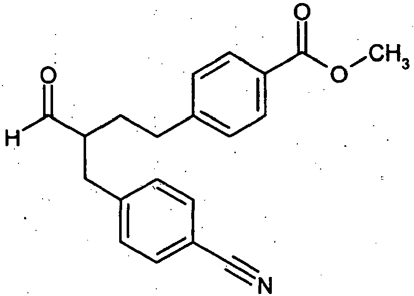

Eine Lösung von 400 mg (1.24 mmol) 4-[3-(4-Cyanobenzyl)-4-hydroxybutyl]benzoesäuremethylester in 7 ml Dichlormethan wird mit 320 mg (1.48 mmol) Pyridiniumchlorochromat (PCC) versetzt und 5 h bei Raumtemperatur gerührt. Nach vollständigem Umsatz wird ca. 1 g Kieselgel zugegeben und das Lösungsmittel im Vakuum bis zur Trockene entfernt. Der Rückstand wird durch Flash-Chromatographie an Kieselgel (Laufmittel: Cyclohexan/Essigsäureethylester 10:1 → 4:1) gereinigt. Es werden 302 mg (90% Reinheit, 69% d. Th.) eines farblosen Feststoffs erhalten.
¹H-NMR (300 MHz, DMSC-d₆, δ/ppm): 9.68 (1H, s), 7.87 (2H, d), 7.77 (2H, d), 7.43 (2H, d), 7.31 (2H, d), 3.86 (3H, s), 3.16-3.03 (1H, m), 2.94-2.81 (1H, m), 2.80-2.55 (3H, m), 1.99-1.81 (1H, m), 1:78-1.61 (1H, m).
MS (DCI): 339 (M+NH₄⁺).

### Beispiel 30A

### (4E)4-[3-(4-Cyanobenzyl)-5-(2-hydroxyphenyl)pent-4-enyl]benzoesäuremethylester

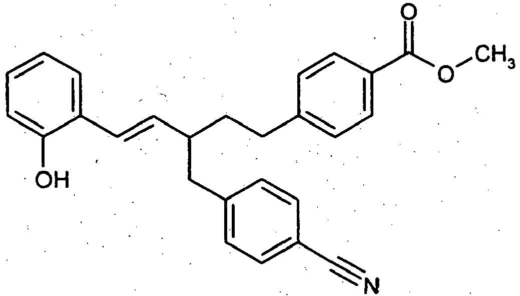

Zu einer Lösung von 1820 mg (4.05 mmol) (2-Hydroxybenzyl)-triphenylphosphoniumbromid in 10 ml wasserfreiem THF werden bei 0°C 5.91 ml (9.45 mmol) einer 1.6 M Lösung von n-Butyllithium in Hexan langsam hinzugefügt. Anschließend werden bei dieser Temperatur 1085 mg (3.38 mmol) 4-[3-(4-Cyanobenzyl)-4-oxobutyl]benzoesäuremethylester, gelöst in 10 ml THF, langsam zudosiert. Nach Erwärmung auf Raumtemperatur wird die Reaktionslösung 12 h nachgerührt, dann mit etwas Wasser versetzt und bis zur Trockene eingeengt. Der Rückstand wird in Essigsäureethylester aufgenommen, mit Wasser und gesättigter Natriumchlorid-Lösung gewaschen und über Natriumsulfat getrocknet. Nach Filtration wird das Lösungsmittel bis zur Trockene eingeengt. Das erhaltene Rohprodukt wird durch Flash-Chromatographie an Kieselgel (Laufmittel: Cyclohexan/ Essigsäureethylester 4:1 → 2:1) gereinigt. Es werden 1150 mg (2.79 mmol, 83% d. Th.) eines farblosen Feststoffs erhalten.
¹H-NMR (300 MHz, DMSO-d₆, δ/ppm): 9.47 (1H, s), 7.88 (2H, d), 7.71 (2H, d), 7.42-7.27 (5H, m), 7.01 (1H, t), 6.81-6.68 (2H, m), 6.45 (1H, d), 6.12-6.00 (1H, m), 3.84 (3H, s), 3.42 (2H, m), 2.95-2.56 (3H, m), 1.88-1.56 (2H, m).
MS (DCI): 429 (M+NH₄⁺).

### Beispiel 31A

### 4-((4E)-3-(4-Cyanobenryl)-5-{2-[phenylpentyl)oxy]phenyl}pent-4-en-1-yl)benzoesäuremethylester

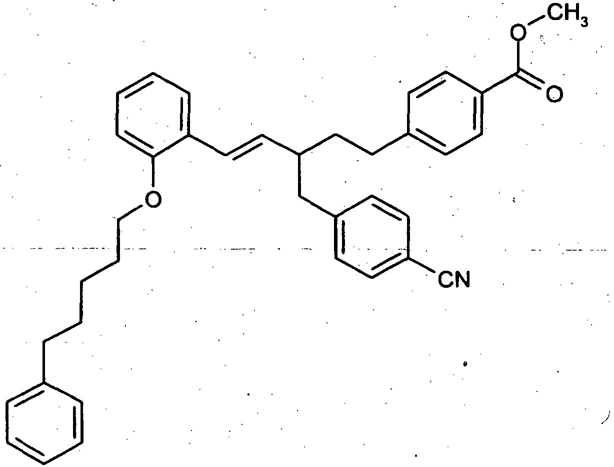

Eine Lösung von 300 mg (0.73 mmol) 4-[(4*E*)-3-(4-Cyanobenzyl)-5-(2-hydroxyphenyl)pent-4-en-1-yl]benzoesäuremethylester in 30 ml trockenem Acetonitril wird mit 198.72 mg (0.87 mmol) (5-Brompentyl)benzol und 151 mg (1.09 mmol) wasserfreiem Kaliumcarbonat versetzt und 12 h unter Rückfluss erhitzt. Anschließend wird der Ansatz zur Trockene eingeengt. Der Rückstand, wird in Essigsäureethylester aufgenommen, mit Wasser und gesättigter Natriumchlorid-Lösung gewaschen und über Natriumsulfat getrocknet. Die organische Phase wird eingeengt. Das erhaltene Rohprodukt wird durch Flash-Chromatographie an Kieselgel (Laufmittel: Cyclohexan/Essigsäureethylester 7:3) gereinigt. Es werden 295 mg (0.53 mmol, 72.6% d. Th.) eines Öls erhalten.
¹H-NMR (400 MHz, DMSO-d₆, δ/ppm): 7.83 (2H, d), 7.71 (2H, d), 7.4-7.33 (2H, m), 7.29 (2H, d), 7.27-7.21 (2H, m), 7.2-7.1 (5H, m), 6.95-6.83 (2H, m), 6.44-6.35 (1H, m), 6.11-6.03 (1H, m), 3.95-3.83 (2H, m), 3.79 (3H, s), 2.91-2.79 (1H, m), 2.79-2.57 (3H, m), 2.57-2.39 (4H, m), 1.84-1.73 (1H, m), 1.72-1.5 (6H, m).
LC-MS (Methode 1): Rₜ= 3.57 min.
MS (ESIpos): m/z = 558 (M+H)⁺.

### Beispiel 32A

### 4-{(4E-5-{2-[(5-Phenylpentyl)oxy]phenyl}-3-[4-(1H-tetrazol-5-yl)benzyl]pent-4-en-1-yl}benzoe-säuremethylester

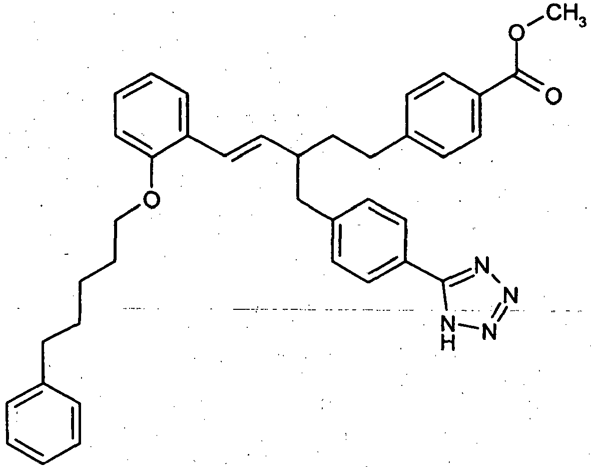

Eine Lösung von 295 mg (0.53 mmol) 4-((4*E*)-3-(4-Cyanobenzyl)-5-{2-[(5-phenylpentyl)oxy]-phenyl}pent-4-en-1-yl)benzoesäuremethylester in 20 ml Toluol wird mit 1.05 ml (7.93 mmol) Trimethylsilylazid und 198 mg (0.79 mmol) Di-*n*-butylzinnoxid versetzt und 12 Stunden auf 80°C erhitzt. Nach Abkühlen auf Raumtemperatur wird der Ansatz mit gesättigter Natriumhydrogencarbonat-Lösung gewaschen. Die organische Phase wird abgetrennt, mit gesättigter Kochsalz-Lösung gewaschen und über Natriumsulfat getrocknet. Nach Filtration wird das Filtrat bis zur Trockene eingeengt. Das erhaltene Rohprodukt wird durch Flash-Chromatographie an Kieselgel (Laufmittel: Dichlormethan → Dichlormethan/Methanol 10:1) gereinigt. Es werden 284 mg (0.47 mmol, 89% d. Th.) eines weißen Schaums erhalten.
LC-MS (Methode 1): Rₜ = 3.40 min.
MS (ESIpos): m/z = 601 (M+H)⁺.

### Beispiel 33A

### E-5-Fluor-2-[2-(4-methoxyphenyl)vinyl]benzaldehyd

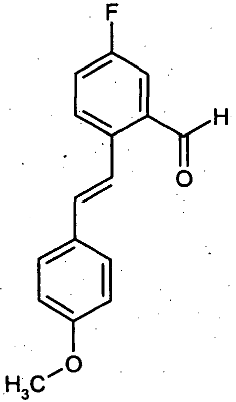

Eine Lösung von 10 g (49.26 mmol) 2-Brom-5-fluorbenzaldehyd in 200 ml trockenem DMF wird unter Argon mit 7.27 g (54.18 mmol) 4-Methoxystyrol, 1.5 g (4.93 mmol) Tri-2-tolylphosphin, 330 mg (1.48 mmol) Palladium(II)acetat und 10.3 ml (73.89 mmol) Triethylamin versetzt und 12 h bei 100°C gerührt. Nach vollständiger Umsetzung wird die Reaktionslösung auf Raumtemperatur abgekühlt und bis zur Trockene eingeengt. Der Rückstand wird in 100 ml Wasser aufgenommen und dreimal mit jeweils 50 ml Diethylether extrahiert. Die vereinigten organischen Phasen werden über Natriumsulfat getrocknet. Nach Filtration wird die Lösung bis zur Trockene eingeengt und der Rückstand durch Flash-Chromatographie an Kieselgel (Laufmittel: Cyclohexan/Essigsäureethylester 10:1) gereinigt. Es werden 7.79 g (55% d. Th.) eines farblosen Feststoffs erhalten.
¹H-NMR (400 MHz, DMSO-d₆, δ/ppm): 10.40 (1H, s), 8.01-7.89 (2H, m), 7.68-7.49 (4H, m), 7.22 (1H, d), 6.99 (2H, d), 3.80 (3H, s).
MS (DCI): 274 (M+NH₄⁺).

### Beispiel 34A

### E-{5-Fluor-2-[2-(4-methoxyphenyl)vinyl]phenyl}methanol

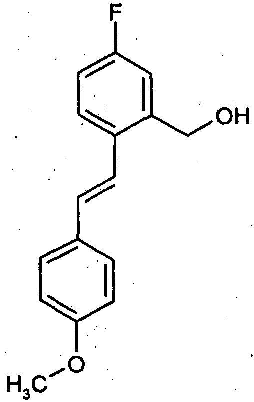

Eine Lösung von 7.79 g (30.40 mmol) *E*-5-Fluor-2-[2-(4-methoxyphenyl)vinyl]benzaldehyd in 500 ml Methanol wird bei 0°C portionsweise mit 1.73 g (45.60 mmol) Natriumborhydrid versetzt und anschließend 2 Stunden bei Raumtemperatur nachgerührt. Nach vollständiger Umsetzung wird der Ansatz bis zur Trockene eingeengt und der Rückstand in Wasser und Dichlormethan aufgenommen. Die wässrige Phase wird noch zweimal mit Dichlormethan extrahiert. Die vereinigten organischen Phasen werden über Natriumsulfat getrocknet, filtriert und bis zur Trockene eingeengt. Der erhaltene Rückstand wird durch Flash-Chromatographie an Kieselgel (Laufmittel: Cyclohexan/Essigsäureethylester 4:1 → 1:1) gereinigt. Es werden 6.8 g (85% d. Th.) eines farblosen Feststoffs erhalten.
¹H-NMR (400 MHz, DMSO-d₆, δ/ppm): 7.71 (1H, m), 7.54 (2H, d), 7.27-7.12 (2H, m), 7.11-6.99 (2H, m), 6.97 (2H, d), 5.47 (1H, t), 4.67 (2H, d), 3.79 (3H, s).
LC-MS (Methode 1): Rₜ 2.49 min; m/z 259 (M+H⁺).

### Beispiel 35A

### {5-Fluor-2-[2-(4-methoxyphenyl)ethyl]phenyl}methanol

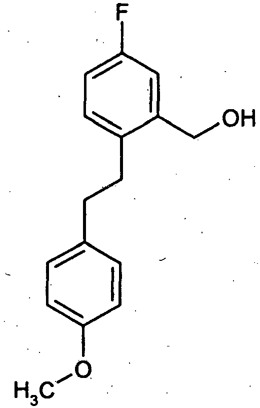

Eine Lösung von 6.8 g (26.33 mmol) *E*-{5-Fluor-2-[2-(4-methoxyphenyl)vinyl]phenyl}methanol und 0.5 g 10% Palladium auf Kohle in 50 ml Methanol und 250 ml Ethanol wird bei Raumtemperatur 1 h lang unter Normaldruck hydriert. Nach Beendigung der Umsetzung wird der Ansatz über Kieselgur filtriert und das Filtrat anschließend bis zur Trockene eingeengt. Der erhaltene Rückstand wird durch Flash-Chromatographie an Kieselgel (Laufmittel: Cyclohexan/Essigsäureethylester 10:1 → 4:1 → 1:1) gereinigt. Es werden 5.95 g (87% d. Th.) eines farblosen Feststoffs erhalten.
¹H-NMR (400 MHz, DMSO-d₆, δ/ppm): 7.21-7.09 (4H, m), 6.97 (1H, t), 6.83 (2H, d), 5.24 (1H, t), 4.51 (2H, d), 3.72 (3H, s), 2.81-2.68 (4H, m).
LC-MS (Methode 1): Rₜ 2.49 min; m/z 278 (M+NH₄⁺).

### Beispiel 36A

### {5-Fluor-2-[2-(4-methoxyphenyl)ethyl]benzyl}triphenylphosphoniumbromid

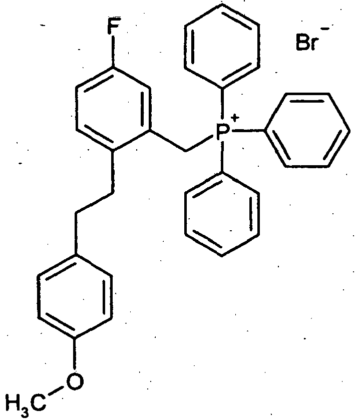

-Eine Lösung von 5.95 g (22.86 mmol) {5-Fluor-2-[2-(4-methoxyphenyl)ethyl]phenyl}methanol in 130 ml Acetonitril wird mit 7.45 g (21.71 mmol) Triphenylphosphoniumbromid versetzt und 3 h unter Rückfluss erhitzt. Anschließend wird die Reaktionslösung bis zur Trockene eingeengt und das erhaltene Öl in Diethylether verrieben. Dabei kristallisiert das Produkt als weißer Feststoff aus. Nach Filtration wird der Feststoff bei 50°C im Trockenofen über Nacht getrocknet. Es werden 11.5 g (77% d. Th.) der Titelverbindung erhalten.
¹H-NMR (300 MHz, DMSO-d₆, δ/ppm): 7.92 (3H, t), 7.81-7.69 (6H, m), 7.68-7.57 (6H, m), 7.30-7.21 (1H, m), 7.19-7.08 (1H, m), 6.94 (2H, d), 6.81 (2H, d), 6.70-6.58 (1H, m), 4.94 (2H, d), 3 .71 (3H, s), 2.57-2.46 (2H, t), 2.18 (2H, t).

### Beispiel 37A

### 4-((4E/Z)-3-(4-Cyanobenzyl)-5-{5-fluor-2-[2-(4-methoxyphenyl)ethyl]phenyl}pent-4-en-1-yl)-benzoesäuremethylester

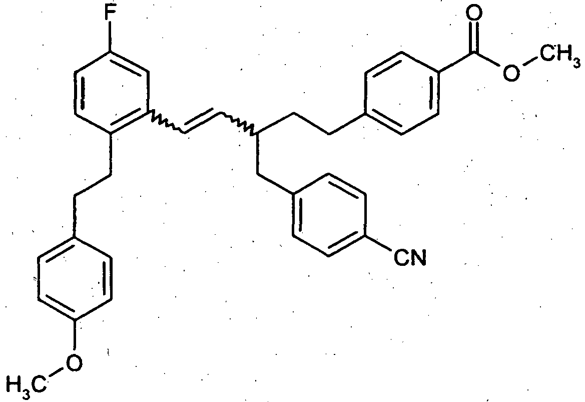

Zu einer Lösung von 1.5 g (4.67 mmol) {5-Fluor-2-[2-(4-methoxyphenyl)ethyl]benzyl}(triphenyl)-phosphoniumbromid werden bei 0°C 4.38 ml (7 mmol) einer 1.6 M Lösung von n-Butyllithium in Hexan langsam hinzugefügt. Anschließend werden bei dieser Temperatur 3.280 g (5.6 mmol) 4-[3-(4-Cyanobenzyl)-4-oxobutyl]benzoesäuremethylester langsam zudosiert. Nach Erwärmung auf Raumtemperatur wird die Reaktionslösung 12 h nachgerührt, dann mit etwas Wasser versetzt und bis zur Trockene eingeengt. Der Rückstand wird in Essigsäureethylester aufgenommen, mit Wasser und gesättigter Natriumchlorid-Lösung gewaschen und über Natriumsulfat getrocknet. Nach Filtration wird das Lösungsmittel bis zur Trockene eingeengt. Das erhaltene Rohprodukt wird durch Flash-Chromatographie an Kieselgel (Laufmittel: Cyclohexan/Essigsäureethylester 50:1 → 20:1) gereinigt. Es werden 2.17 g (3.9 mmol, 84% d. Th.) eines weißen Schaums erhalten.
MS (ESIpos): m/z = 548 (M+H)⁺.

### Beispiel 38A

### 4-{(4E/Z)-5-{5-Fluor-2-[2-(4-methoxyphenyl)ethyl]phenyl}-3-[4-(1H-tetrazol-5-yl)benzyl]pent-4-en-1-yl}benzoesäuremethylester

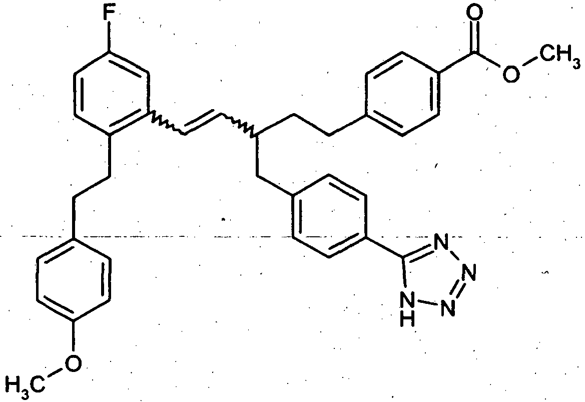

Eine Lösung von 600 mg (1.1 mmol) 4-((4*E*/*Z*)-3-(4-Cyanobenzyl)-5-{5-fluor-2-[2-(4-methoxyphenyl)ethyl]phenyl}pent-4-en-1-yl)benzoesäuremethylester in 30 ml Toluol wird mit 2.18 ml (16.43 mmol) Trimethylsilylazid und 409 mg (1.64 mmol) Di-n-butylzinnoxid versetzt und 12 h auf 80°C erhitzt. Nach Abkühlen auf Raumtemperatur wird der Ansatz mit gesättigter Natriumhydrogencarbonat-Lösung gewaschen. Die organische Phase wird abgetrennt, mit gesättigter Kochsalz-Lösung gewaschen und über Natriumsulfat getrocknet. Nach Filtration wird das Filtrat bis zur Trockene eingeengt. Es werden 642 mg (1.01 mmol, 99% d. Th.) eines weißen Schaums erhalten.
LC-MS (Methode 1): Rₜ = 3.19 min.
MS (ESIpos): m/z = 591 (M+H)⁺.

### Beispiel 39A

### 4'-Trifluormethyl-biphenyl-4-carbonsäureethylester

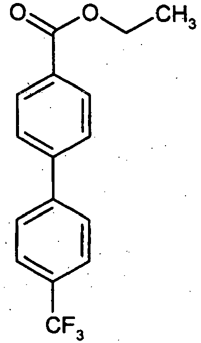

7 g (30.56 mmol) 4-Brombenzoesäureethylester werden in 60 ml 1,2-Dimethoxyethan gelöst und unter Argon mit 6.96 g (36.67 mmol) 4-Trifluormethylphenylboronsäure, 271 mg Bis(triphenyl-phosphin)palladium(II)chlorid und 40.7 ml einer 2 M Natriumcarbonat-Lösung in Wasser versetzt. Das Reaktionsgemisch wird anschließend 12 h unter Rückfluss gerührt. Danach wird der. Ansatz abgekühlt, über 1 g Extrelute filtriert, mit Dichlormethan gewaschen und eingeengt. Das erhaltene Rohprodukt wird durch Flash-Chromatographie an Kieselgel (Laufmittel: Cyclohexan/Dichlormethan 2:1) gereinigt. Es werden 6.31 g (21.4 mmol, 70% d. Th.) eines farblosen Feststoffs erhalten.
¹H-NMR (300 MHz, CDCl₃, δ/ppm): 8.17 (2H, d), 7.72 (4H, s), 7.67 (2H, d), 4.41 (2H, q), 1.43 (3H, t).
MS (EI): 294 (M⁺).

### Beispiel 40A

### (4'-Trifluormethyl-biphenyl-4-yl)methanol

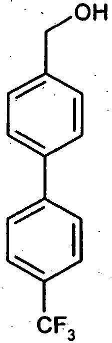

Eine Lösung von 6.24 g (21.21 mmol) 4'-Trifluormethyl-biphenyl-4-carbonsäureethylester in 60 ml trockenem THF wird bei 0°C tropfenweise mit 12.73 ml (12.73 mmol) einer 1 M Lösung von Lithiumaluminiumhydrid in THF versetzt. Nach vollständiger Umsetzung wird der Ansatz mit gesättigter Ammoniumchlorid-Lösung versetzt, in Essigsäureethylester aufgenommen, die organische Phase abgetrennt und über Natriumsulfat getrocknet. Nach Filtration wird das Lösungsmittel im Vakuum entfernt. Das erhaltene Rohprodukt wird durch Flash-Chromatographie an Kieselgel (Laufmittel: Cyclohexan/Essigsäureethylester 5:1) gereinigt. Es werden 5.1 g (20.21 mmol, 95% d. Th.) eines farblosen Feststoffs erhalten.
¹H-NMR (300 MHz, DMSO-d₆, δ/ppm): 7.88 (2H, d), 7.82 (2H, d), 7.71 (2H, d), 7.46 (2H, d), 5.23 (1H, t), 4.58 (2H, d).
MS (EI): 252 (M⁺).

### Beispiel 41A

### 4-Chlormethyl-4'-trifluormethyl-biphenyl

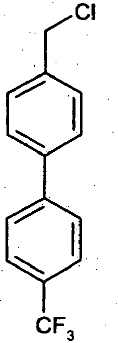

Eine Lösung von 5.0 g (19.82 mmol) (4'-Trifluormethyl-biphenyl-4-yl)methanol in 40 ml Chloroform wird mit 2.89 ml (39.65 mmol) Thionylchlorid, gelöst in 10 ml Chloroform, versetzt und über 12 Stunden bei Raumtemperatur gerührt. Nach vollständiger Umsetzung wird das Reaktionsgemisch bis zur Trockene eingeengt, der Rückstand in Essigsäureethylester aufgenommen und mit gesättigter Natriumcarbonat-Lösung gewaschen. Die organische Phase wird anschließend abgetrennt, über Natriumsulfat getrocknet und nach Filtration eingeengt. Das erhaltene Rohprodukt wird durch Flash-Chromatographie an Kieselgel (Laufmittel: Cyclohexan/Essigsäureethylester 9: 1) gereinigt. Es werden 5.26 g (19.43 mmol, 98% d. Th.) eines farblosen Feststoffs erhalten.
¹H-NMR (300 MHz, DMSO-d₆, δ/ppm): 7.91 (2H, d), 7.82 (2H, d), 7.78 (2H, d), 7.58 (2H, d), 4.83 (2H, s).
MS (EI): 270 (M⁺).

### Beispiel 42A

### 4-[(4E)-3-(4-Cyanobenzyl)-5-(2-{[4'-(trifluormethyl)biphenyl-4-yl]methoxy}phenyl)pent-4-en-1-yl]benzoesäuremethylester

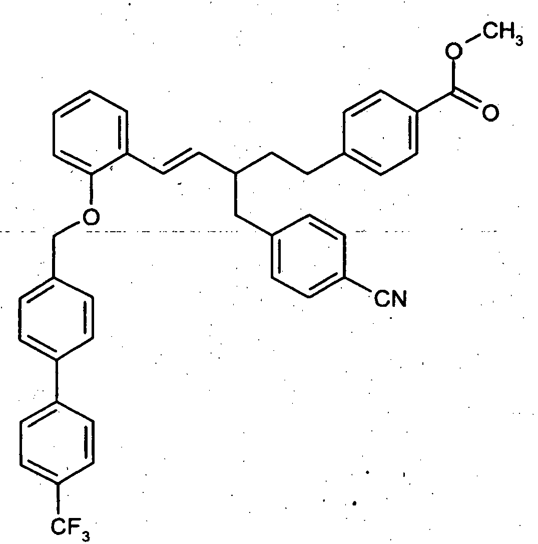

Eine Lösung von 300 mg (0.73 mmol) 4-[(4*E*)-3-(4-Cyanobenzyl)-5-(2-hydroxyphenyl)pent-4-en-1-yl]benzoesäuremethylester in 30 ml trockenem Acetonitril wird mit 236.8 mg (0.87 mmol) 4-(Chlormethyl)-4'-(trifluormethyl)biphenyl und 151 mg (1.09 mmol) wasserfreiem Kaliumcarbonat versetzt und 12 Stunden unter Rückfluss erhitzt. Anschließend wird der Ansatz zur Trockene eingeengt. Der Rückstand wird in Essigsäureethylester aufgenommen, mit Wasser und gesättigter Natriumchlorid-Lösung gewaschen und über Natriumsulfat getrocknet. Die organische Phase wird eingeengt. Das erhaltene Rohprodukt wird durch Flash-Chromatographie an Kieselgel (Laufmittel: Cyclohexan/Essigsäureethylester 7:3) gereinigt. Es werden 331 mg (0.51 mmol, 70% d. Th.) eines Feststoffs erhalten.
LC-MS (Methode 1): Rₜ = 3.57 min.
MS (ESIpos): m/z = 646 (M+H)⁺.

### Beispiel 43A

### 4-[(4E)-3-[4-(1H-Tetrazol-5-yl)benzyl]-5-(2-{[4'-(trifluormethyl)biphenyl-4-yl]methoxy}phenyl)-pent-4-en-1-yl]benzoesäuremethylester

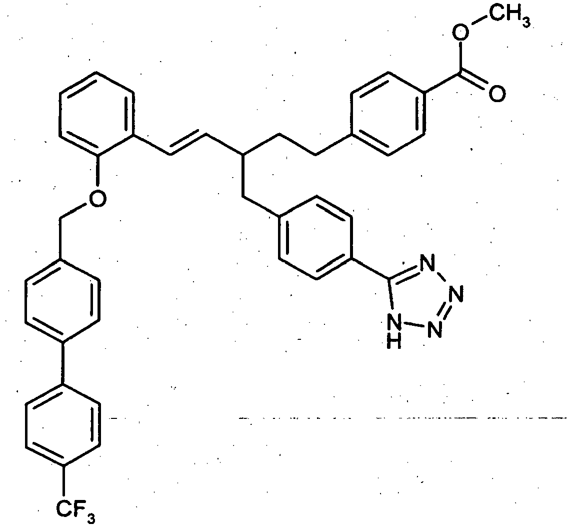

Eine Lösung von 295 mg (0.53 mmol) 4-[(4*E*)-3-4-Cyanobenzyl)-5-(2-{[4'-(trifluormethyl)-biphenyl-4-yl]methoxy}phenyl)pent-4-en-1-yl]benzoesäuremethylester in 20 ml Toluol wird mit 1.33 ml (9.99 mmol) Trimethylsilylazid und 249 mg (1 mmol) Di-*n*-butylzinnoxid versetzt und 12 Stunden auf 80°C erhitzt. Nach Abkühlen auf Raumtemperatur wird der Ansatz mit gesättigter Natriumhydrogencarbonat-Lösung gewaschen. Die organische Phase wird abgetrennt, mit gesättigter Kochsalz-Lösung gewaschen und über Natriumsulfat getrocknet. Nach Filtration wird das Filtrat bis zur Trockene eingeengt. Das erhaltene Rohprodukt wird durch Flash-Chromatographie an Kieselgel (Laufmittel: Dichlormethan → Dichlormethan/Methanol 10:1) gereinigt. Es werden 387 mg (0.56 mmol, 64% d. Th.) eines weißen Schaums erhalten.
LC-MS (Methode 1): Rₜ = 3.42 min.
MS (ESIpos): m/z = 689 (M+H)⁺.

### Beispiel 44A

### 4-[(4E)-5-{2-[(4-tert.-Butylbenzyl)oxy]phenyl}-3-(4-cyanobenzyl)pent-4-en-1-yl]benzoesäure-methylester

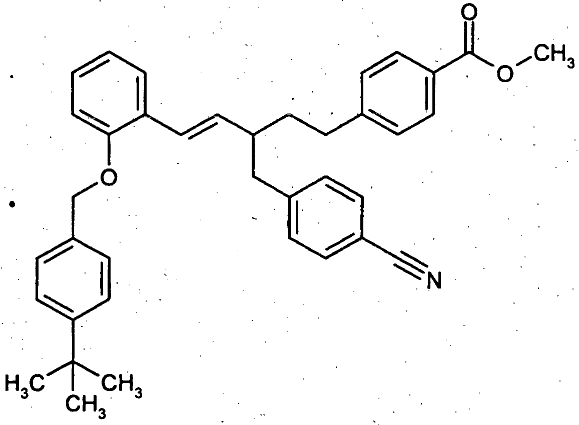

Eine Lösung von 1.4 g (3.13 mmol, 92%-ig) *E*-4-[3-(4-Cyanobenzyl)-5-(2-hydroxyphenyl)pent-4-enyl]benzoesäuremethylester in 7 ml trockenem Acetonitril wird mit 1.42 g (6.26 mmol) 4-(*tert*.-Butyl)benzylbromid und 1.30 g (9.39 mmol) wasserfreiem Kaliumcarbonat versetzt und 12 h unter Rückfluß erhitzt. Anschließend wird der Ansatz filtriert und zur Trockene eingeengt. Der Rückstand wird direkt chromatographisch an Kieselgel (Laufmittel: Cyclohexan/Essigsäureethylester 10:1, dann 1:5) aufgereinigt. Es werden 1.85 g (3.32 mmol, 93% Reinheit, 98% d. Th.) eines Öls erhalten.
¹H-NMR (300 MHz, CDCl₃, δ/ppm): 7.94-7.88 (2H, m), 7.52-7.14 (12H, m), 6.97-6.89 (2H, m), 6.67-6.57 (1H, m), 6.02-5.92 (1H, m), 5.05-5.02 (2H, m), 3.90 (3H, s), 2.85-2.67 (3H, m), 2.67-2.55 (1H, m), 2.54-2.42 (1H, m), 1.88-1.61 (2H, m), 1.33 (9H, s).
LC-MS (Methode 4): Rₜ 3.53 min; m/z 557 (M⁺).

### Beispiel 45A

### 4-{(4E)-5-{2-[(4-tert.-Butylbenzyl)oxy]phenyl}-3-[4-(1H-tetrazol-5-yl)benzyl]pent-4-en-1-yl}-benzoesäuremethylester

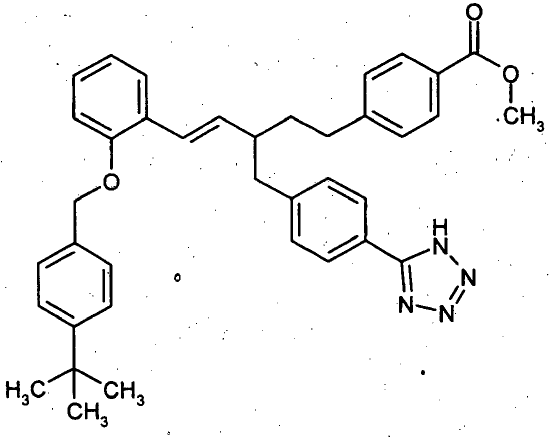

Eine Lösung von 180 mg (0.32 mmol) 4-[(4*E*)-5-{2-[(4-*tert*.-Butylbenzyl)oxy]phenyl}-3-(4-cyanobenzyl)pent-4-en-1-yl]benzoesäuremethylester in 10 ml Toluol wird mit 557.3 mg (4.84 mmol) Trimethylsilylazid und 120.51 mg (0.48 mmol) Di-*n*-butylzinnoxid versetzt und 12 Stunden auf 80°C erhitzt. Nach Abkühlen auf Raumtemperatur wird der Ansatz mit gesättigter Natriumhydrogencarbonat-Lösung gewaschen. Die organische Phase wird abgetrennt, mit gesättigter Kochsalz-Lösung gewaschen und über Natriumsulfat getrocknet. Nach Filtration wird das Filtrat bis zur Trockene eingeengt. Das erhaltene Rohprodukt wird über eine Kieselgelsäule (Laufmittel: Cyclohexan/Essigsäureethylester 2:1 → 1:2) gereinigt. Es werden 88 mg (0.14 mmol, 43% d. Th.) eines weißen Schaums erhalten.
¹H-NMR (300 MHz, CDCl₃, δ/ppm): 13.15 (1H, breit), 7.87-7.73 (4H, m), 7.49-7.31 (6H, m), 7.18 (2H, d), 7.09 (2H, d), 6.99-6.9 (2H, m), 6.7 (1H, d), 6.05 (1H, dd), 5.03 (2H, s), 3.94 (3H, s), 2.86-2.66 (3H, m), 2.66-2.51 (1H, m), 2.49-2.34 (1H, m), 1.85-1.62 (2H, m), 1.32 (9H, s).

### Beispiel 46A

### 2-(4-Cyanobutyl)malonsäurediallylester

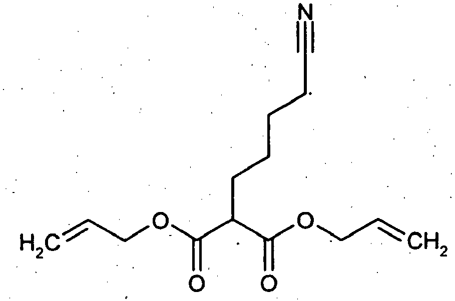

Zu einer Lösung von 100 g (542.9 mmol) Malonsäurediallylester in 700 ml trockenem Dioxan werden bei 0°C 21.71 g (542.9 mmol, 60%-ig) Natriumhydrid portionsweise zugegeben. Nach Beendigung der Gasentwicklung wird das Reaktionsgemisch auf 40°C erwärmt und 1 Stunde nachgerührt. Anschließend werden 43.98 g (271.5 mmol) 5-Bromvaleriansäurenitril in 350 ml trockenem Dioxan zugetropft und das Gemisch 12 Stunden bei 110°C gerührt. Nach Beendigung der Reaktion wird der Ansatz auf Raumtemperatur abgekühlt, mit 400 ml gesättigter Ammoniumchlorid-Lösung versetzt und mit Essigsäureethylester extrahiert. Nach Phasentrennung wird die wässrige Phase noch dreimal mit je 250 ml Essigsäureethylester extrahiert. Nach Vereinigung der organischen Phasen wird mit gesättigter Natriumchlorid-Lösung gewaschen, über Natriumsulfat getrocknet und anschließend das Lösungsmittel im Vakuum abgezogen. Darauffolgend wird durch Hochvakuum-Destillation überschüssiger Malonsäurediallylester entfernt (Siedepunkt: 57°C, 0.074 mbar). Der erhaltene Destillationsrückstand wird durch Flash-Chromatographie an Kieselgel (Laufmittel: Cyclohexan/Essigsäureethylester 2:1) gereinigt. Es werden 105 g (233 mmol, ca. 60% Reinheit, 43% d. Th.) der Titelverbindung erhalten, welche ohne weitere Aufreinigung in der Folgestufe eingesetzt wird. Eine kleine Menge wird mittels präparativer HPLC für die analytische Charakterisierung aufgereinigt.
¹H-NMR (300 MHz, DMSO-d₆, δ/ppm): 5.96-5.83 (2H, m), 5.35-5.19 (4H, m), 4.64-4.59 (4H, m), 3.66-3.61 (1H, t), 2.54-2.47 (2H, t), 1.86-1.75 (2H, m), 1.62-1.50 (2H, m), 1.42-1.29 (2H, m).
LC-MS (Methode 1): Rₜ 2.44 min; m/z 266 (M+H)⁺.

### Beispiel 47A

### 2-(4-Cyanobutyl)-2-[2-(4-methoxycarbonyl-phenyl)ethyl]malonsäurediallylester

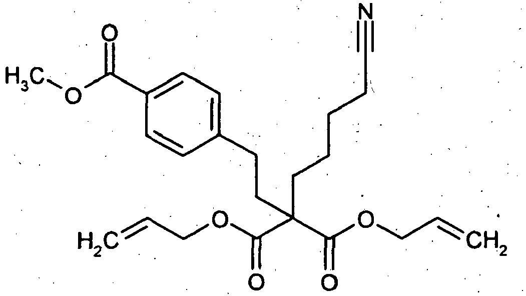

Eine Lösung von 48.64 g (127.73 mmol) 2-(4-Cyanobutyl)malonsäurediallylester in 160 ml trockenem DMF wird bei 0°C portionsweise mit 5.62 g (140 mmol, 60%-ig) Natriumhydrid versetzt. Anschließend lässt man die Reaktionslösung auf Raumtemperatur kommen und rührt 90 min nach. Danach wird die Reaktionslösung wieder auf 0°C abgekühlt, mit 45.72 g (153.3 mmol) 4-(2-Bromethyl)benzoesäuremethylester in 80 ml trockenem DMF versetzt und 45 min bei dieser Temperatur nachgerührt. Der Ansatz wird dann bei Raumtemperatur über Nacht gerührt. Das Reaktionsgemisch wird mit Wasser versetzt und mit Essigsäureethylester extrahiert. Nach Phasentrennung wird die wässrige Phase noch dreimal mit je 200 ml Essigsäureethylester extrahiert. Nach Vereinigung der organischen Phasen wird mit gesättigter Natriumchlorid-Lösung gewaschen, über Natriumsulfat getrocknet und anschließend das Lösungsmittel im Vakuum abgezogen. Das erhaltene Rohprodukt wird mittels Kieselgel-Chromatographie (Laufmittel; Cyclohexan/Essigsäureethylester 10:1 → 100% Essigsäureethylester) aufgereinigt. Es werden 18.53 g (43.3 mmol, 34% d. Th.) einer farblosen Flüssigkeit erhalten.
¹H-NMR (300 MHz, DMSO-d₆, δ/ppm): 7.90-7.88 (2H, d), 7.36-7.34 (2H, d), 5.97-5.83 (2H, m), 5.35-5.21 (4H, m), 4.64-4.59 (4H, m), 3.84 (3H, s), 2.57-2.48 (4H, t), 2.15-2.09 (2H, m), 1.98-1.89 (2H, m), 1.63-1.52 (2H, m), 1.34-1.21 (2H, m).
LC-MS (Methode 2): Rₜ 2.59 min; m/z 428 (M+H)⁺.

### Beispiel 48A

### 4-(3-Carboxy-7-cyanoheptyl)benzoesäuremethylester

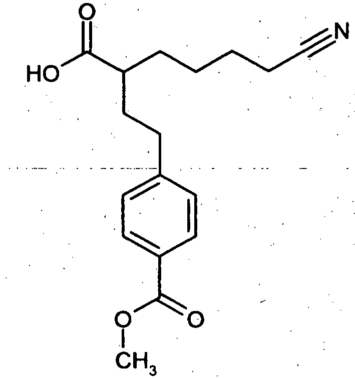

Eine Lösung von 6.92 g (16.19 mmol) 2-(4-Cyanobutyl)-2-[2-(4-methoxycarbonylphenyl)ethyl]-malonsäurediallylester, 594 mg (2.26 mmol) Triphenylphosphin und 145 mg (0.64 mmol) Palladiumacetat in 67 ml Dioxan wird bei Raumtemperatur mit einer Lösung von 7.45 ml (53.42 mmol) Triethylamin und 1.53 ml (40.47 mmol) Ameisensäure in 67 ml Dioxan versetzt. Das Reaktionsgemisch wird anschließend 12 h bei 100°C gerührt. Nach vollständigem Umsatz wird die Reaktionslösung abgekühlt und das Lösungsmittel im Vakuum entfernt. Der Rückstand wird in Essigsäureethylester und Wasser aufgenommen, mit 1 N Salzsäure angesäuert und die organische Phase abgetrennt. Die wässrige Phase wird noch dreimal mit Essigsäureethylester extrahiert, die organischen Phasen werden vereinigt, mit gesättigter Natriumchlorid-Lösung gewaschen und über Natriumsulfat getrocknet. Nach Filtration wird die Lösung im Vakuum eingeengt. Das erhaltene Rohprodukt wird durch Flash-Chromatographie an Kieselgel [Laufmittel: Cyclohexan/Essigsäure-ethylester (mit 1% Ameisensäure) 2:1 → 1:3] gereinigt. Es werden 2.1 g (43% d. Th.) eines farblosen Feststoffs erhalten.
LC-MS (Methode 2): Rₜ 1.88 min; m/z 303 (M⁺).

### Beispiel 49A

### 4-(7-Cyano-3-hydroxymethyl-heptyl)benzoesäuremethylester

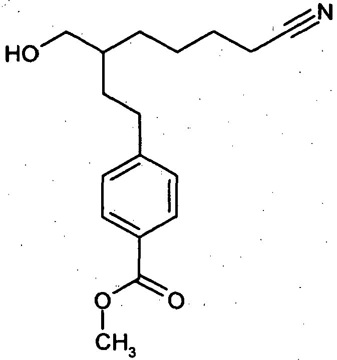

Zu einer Lösung von 5 g (16.48 mmol) 4-(3-Carboxy-7-cyanoheptyl)benzoesäuremethylester in 62 ml THF werden 33 ml (33 mmol) einer 1 M Boran-THF-Komplex-Lösung bei -15°C zugetropft und die Lösung 2 h bei dieser Temperatur nachgerührt. Anschließend werden weitere 16 ml (16 mmol) 1 M Boran-THF-Komplex-Lösung bei -15°C zugetropft und nochmals 45 min nachgerührt. Danach wird die Reaktionsmischung auf 0°C erwärmt und bei dieser Temperatur noch 1 h gerührt. Nach vollständiger Umsetzung wird das Reaktionsgemisch mit 100 ml gesättigter Natriumhydrogencarbonat-Lösung versetzt und das Lösungsmittel bis zur Trockene eingeengt. Der Rückstand wird in Essigsäureethylester und Wasser aufgenommen und die wässrige Phase nochmals mit Essigsäureethylester extrahiert. Die vereinigten organischen Phasen werden über Natriumsulfat getrocknet und wiederum vom Lösungsmittel befreit. Das erhaltene Rohprodukt wird durch Flash-Chromatographie an Kieselgel (Laufmittel:. Cyclohexan/Essigsäureethylester 2:1 → 1:3 → 100% Essigsäureethylester) gereinigt. Es werden 2.4 g (93% Reinheit, 47% d. Th.) eines farblosen Feststoffs erhalten.
¹H-NMR (400 MHz, DMSO-d₆, δ/ppm): 7.88-7.86 (2H, d), 7.36-7.34 (2H, d), 4.43-4.40 (t, 1H), 3.83 (s, 3H), 3.37-3.34 (2H, t), 2.68-2.64 (2H, t), 2.49-2.46 (2H, t), 1.67-1.57 (1H, m), 1.56-1.45 (3H, m), 1.42-1.25 (5H, m).
LC-MS (Methode 2): Rₜ 1.93 min; m/z 290 (M+H)⁺.

### Beispiel 50A

### 4-(7-Cyano-3-formyl-heptyl)benzoesäuremethylester

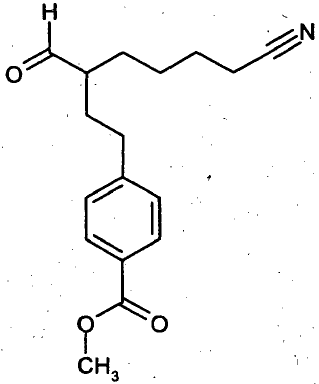

Eine Lösung von 2.23 g (7.71 mmol) 4-(7-Cyano-3-hydroxymethyl-heptyl)benzoesäuremethylester in 100 ml Dichlormethan wird mit 1.99 g (9.26 mmol) Pyridiniumchlorochromat (PCC) versetzt und 6 h bei Raumtemperatur gerührt. Nach vollständigem Umsatz wird das Lösungsmittel im Vakuum bis zur Trockene eingeengt. Der erhaltene Rückstand wird durch Flash-Chromatographie an Kieselgel (Laufmittel: Cyclohexan/Essigsäureethylester 10:1 → 4:1) gereinigt. Es werden 1.50 g (9.09 mmol, 68% d. Th.) eines Öls erhalten.
¹H-NMR (400 MHz, CDCl₃, δ/ppm): 9.62 (1H, d), 7.98-7.96 (2H, d), 7.25-7.23 (2H, d), 3.91 (3H, s), 2.77-2.62 (2H, m), 2.35 (2H, t), 2.38-2.29 (1H, m), 2.07-1.98 (1H, m), 1.82-1.63 (4H, m), 1.55-1.47 (3H, m).
MS (ESI): 310 (M+Na)⁺.

### Beispiel 51A

### E-4-{7-Cyano-3-[2-(2-hydroxyphenyl)vinyl]heptyl}benzoesäuremethylester

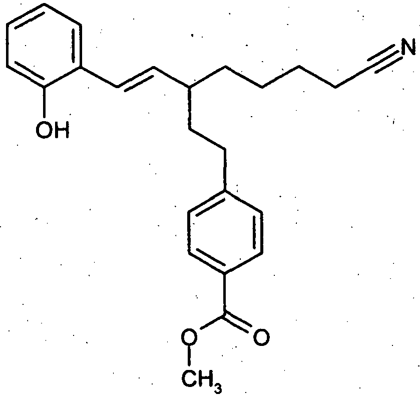

Zu einer Suspension von 3.26 g (7.26 mmol) 2-Hydroxyphenyl-triphenylphosphoniumbromid in 40 ml trockenem THF werden bei 0°C 9.07 ml (14.52 mmol) einer 1.6 M Lösung von n-Butyllithium in Hexan langsam zugetropft und die Mischung 5 min nachgerührt. Anschließend wird bei dieser Temperatur eine Lösung von 1.49 g (5.19 mmol) 4-(7-Cyano-3-formyl-heptyl)benzoesäuremethylester in 10 ml trockenem THF langsam zugetropft. Die Reaktionsmischung wird 10 min bei 0°C gerührt. Danach wird die Kühlung entfernt, die Reaktionslösung 10 min bei Raumtemperatur nachgerührt, dann mit Kieselgel versetzt und bis zur Trockene eingeengt. Der erhaltene Rückstand wird direkt durch Flash-Chromatographie an Kieselgel (Laufmittel: Cyclohexan/Essigsäureethylester 1:2 → 1:5) gereinigt. Es werden 1.71 g (75% Reinheit, 3.40 mmol, 65% d. Th.) eines Öls erhalten.
¹H-NMR (300 MHz, DMSO-d₆, δ/ppm): 9.50 (1H, s), 7.88-7.86 (2H, d), 7.40-7.34 (3H, m), 7.05-7.00 (1H, m), 6.84-6.68 (2H, m), 6.62-6.56 (1H, m), 6.05-5.97 (1H, dd), 3.83 (3H, s), 2.75-2.56 (2H, m), 2.52-2.44 (3H, m), 2.18-2.06 (1H, m), 1.81-1.28 (7H, m).
LC-MS (Methode 2): Rₜ 2.60 min; m/z 377 (M⁺).

### Beispiel 52A

### E-4-(3-{2-[2-(4-tert.-Butylbenzyloxy)phenyl]vinyl}-7-cyanoheptyl)benzoesäuremethylester

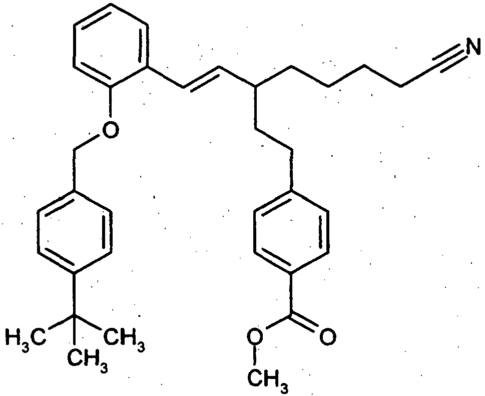

Eine Lösung von 1.70 g (3.38 mmol, 75%-ig) 4-{7-Cyano-3-[2-(2-hydroxyphenyl)vinyl]heptyl}-benzoesäuremethylester in 20 ml trockenem Acetonitril wird mit 1.53 g (7.76 mmol) 4-(*tert*.-Butyl)benzylbromid und 1.4 g (10.13 mmol) wasserfreiem Kaliumcarbonat versetzt und 12 h unter Rückfluss erhitzt. Anschließend wird der Ansatz filtriert und das Filtrat zur Trockene eingeengt. Das erhaltene Rohprodukt wird auf Kieselgel aufgenommen und durch Flash-Chromatographie (Laufmittel: Cyclohexan/Essigsäureethylester 10:1) gereinigt. Es werden 1700 mg (3.12 mmol, 96% Reinheit, 92% d. Th.) eines Öls erhalten.
¹H-NMR (400 MHz, CDCl₃, δ/ppm): 7.93-7.71 (2H, d), 7.46-7.35 (5H, m), 7.24-7.18 (3H, m), 6.97-6.93 (2H, m), 6.77-6.73 (1H, d), 5.98-5.92 (1H, dd), 5.11-5.05 (2H, m), 3.90 (3H, s), 2.79-2.70 (1H, m), 2.66-2.57 (1H, m), 3.36 (2H, t), 2.20-2.11 (1H, m), 1.82-1.73 (1H, m), 1.70-1.56 (3H, m), 1.56-1.35 (9H, s).
LC-MS (Methode 4): Rₜ 3.36 min; m/z 523 (M⁺).

### Beispiel 53A

### 4-[3-((E)-2-{2-[(4-tert.-Butylbenzyl)oxy]phenyl}vinyl)-7-(1H-tetrazol-5-yl)heptyl]benzoesäuremethylester

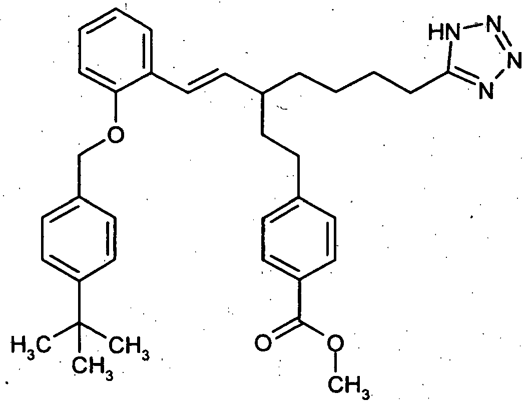

Eine Lösung von 200 mg (0.38 mmol) 4-[3-((*E*)-2-{2-[(4-*tert*.-Butylbenzyl)oxy]phenyl}vinyl)-7-cyanoheptyl]benzoesäuremethylester in 3 ml Toluol wird mit 660 mg (5.73 mmol) Trimethylsilylazid und 142.6 mg (0.57 mmol) Di-*n*-butylzinnoxid versetzt und 12 Stunden auf 80°C erhitzt. Nach Abkühlen auf Raumtemperatur wird der Ansatz mit gesättigter Natriumhydrogencarbonat-Lösung gewaschen. Die organische Phase wird abgetrennt, mit gesättigter Kochsalz-Lösung gewaschen und über Natriumsulfat getrocknet. Nach Filtration wird das Filtrat bis zur Trockene eingeengt. Das erhaltene Rohprodukt wird über eine Kieselgelsäule (Laufmittel: Cyclohexan/Essigsäureethyl-ester 1:1) chromatographisch gereinigt. Es werden 103 mg (0.18 mmol, 46% d. Th.) eines weißen Schaums erhalten.
¹H-NMR (300 MHz, CDCl₃, δ/ppm): 12.34 (1H, breit), 7.93 (2H, d), 7.47-7.33 (6H, m), 7.22 (3H, d), 7.06-6.93 (2H, m), 6.74 (1H, d), 5.91 (1H, dd), 5.18-5.02 (2H, m), 3.90 (3H, s), 2.89-2.54 (4H, m), 2.22-2.06 (1H, m), 1.83-1.53 (4H, m), 1.52-1.19 (13H, m).

### Beispiel 54A

### 2-Allyloxycarbonyl-heptandisäure-1-allylester-7-ethylester

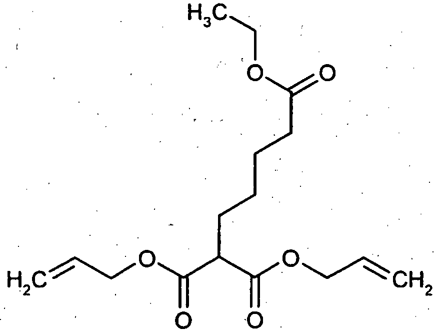

Zu einer Lösung von 100 g (542.92 mmol) Malonsäurediallylester in 900 ml trockenem Dioxan werden bei 5°C 16.29 g (407.19 mmol) Natriumhydrid portionweise zugegeben. Nach Beendigung der Gasentwicklung wird das Reaktionsgemisch auf 40°C erwärmt und 30 min nachgerührt. Anschließend werden 56.76 g (271.46 mmol) 5-Bromvaleriansäureethylester in 100 ml trockenem Dioxan zugetropft und das Gemisch 12 Stunden bei 110°C gerührt. Nach Beendigung der Reaktion wird der Ansatz auf Raumtemperatur abgekühlt und auf ca. 400 ml Eiswasser gegeben. Nach Neutralisation mit 1 N Salzsäure wird die organische Phase abgetrennt und die wässrige Phase dreimal mit je 250 ml Essigsäureethylester extrahiert. Nach Vereinigung der organischen Phasen wird mit gesättigter Natriumchlorid-Lösung gewaschen und über Natriumsulfat getrocknet. Nach Filtration wird die Lösung im Vakuum eingeengt. Darauffolgend wird durch Hochvakuum-Destillation überschüssiger Malonsäurediallylester entfernt (Siedepunkt: 57°C, 0.074 mbar): Der erhaltene Destillationsrückstand wird durch Flash-Chromatographie an Kieselgel (Laufmittel: Cyclohexan/Essigsäureethylester 2:1) gereinigt. Es werden 73.92 g (236.65 mmol, 44% d. Th.) einer farblosen Flüssigkeit erhalten.
¹H-NMR (400 MHz, DMSO-d₆, δ/ppm): 5.99-5.81 (2H, m), 5.38-5.16 (4H, m), 4.68-4.51 (4H, m), 4.04 (2H, q), 3.59 (1H, t), 2.28 (2H, t), 1.86-1.71 (2H, m), 1.61-1.45 (2H, m), 1.35-1.20 (2H, m), 1.17 (3H, t).
MS (DCI): 330 (M+NH₄⁺).

### Beispiel 55A

### 2-Allyloxycarbonyl-2-[2-(4-cyanophenyl)ethyl]heptandisäure-1-allylester-7-ethylester

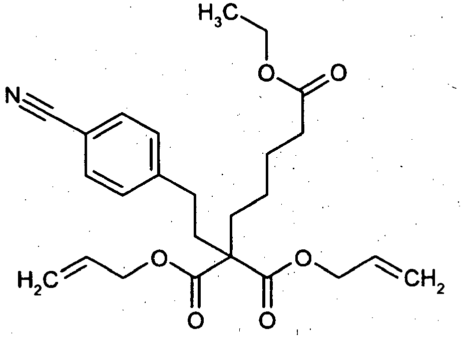

Eine Lösung von 45.23 g (144.79 mmol) 2-Allyloxycarbonyl-heptandisäure-1-allylester-7-ethylester in 250 ml DMF wird bei 0°C portionsweise mit 6.37 g (159.27 mmol, 60%-ig) Natriumhydrid versetzt. Anschließend lässt man die Reaktionslösung auf Raumtemperatur kommen und rührt 30 min nach. Danach wird die Reaktionslösung wieder auf 0°C abgekühlt, mit 36.50 g (173.75 mmol) 4-(2-Bromethyl)benzonitril in 250 ml DMF versetzt und 30 min bei dieser Temperatur nachgerührt. Der Ansatz wird anschließend bei Raumtemperatur über Nacht gerührt. Das Reaktionsgemisch wird tropfenweise mit Wasser versetzt, mit Essigsäureethylester dreimal extrahiert, die vereinigten organischen Phasen mit gesättigter Natriumchlorid-Lösung gewaschen und über Natriumsulfat getrocknet. Nach Filtration wird das Lösungsmittel im Vakuum bis zur Trockene eingeengt. Das erhaltene Rohprodukt wird durch Flash-Chromatographie an Kieselgel (Laufmittel: Cyclohexan/Essigsäureethylester 10:1 → 4:1 → 1:1) gereinigt. Es werden 17.85 g (40.43 mol, 28% d. Th.) eines farblosen Feststoffs erhalten.
¹H-NMR (400 MHz, DMSO-d₆, δ/ppm): 7.77 (2H, d), 7.42 (2H, d), 5.97-5.82 (2H, m), 5.37-5.18 (4H, m), 4.60 (4H, d), 4.04 (2H, q), 2.59-2.45 (2H, m), 2.30 (2H, t), 2.14-2.02 (2H, m), 1.96-1.83 (2H, m), 1.60-1.47 (2H, m), 1.24-1.07 (5H, m).
MS (DCI): 459 (M+NH₄⁺).

### Beispiel 56A

### 2-[2-(4-Cyanophenyl)ethyl]heptandisäure-7-ethylester

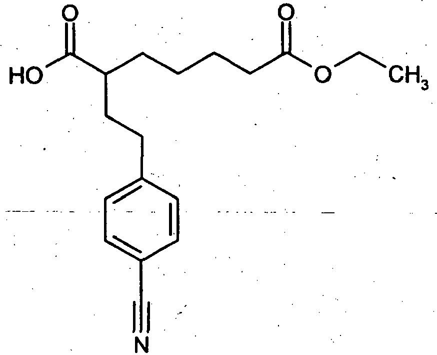

Eine Lösung von 21 g (40.43 mmol) 2-Allyloxycarbonyl-2-[2-(4-cyanophenyl)ethyl]heptandisäure-Eine Lösung von 21 g (40.43 mmol) 2-Allyloxycarbonyl-2-[2-(4-cyanophenyl)ethyl]heptandisäure-1-allylester-7-ethylester, 742 mg (2.83 mmol) Triphenylphosphin und 181 mg (0.81 mmol) Palladiumacetat in 175 ml Dioxan wird bei Raumtemperatur mit einer Lösung von 18.6 ml (133.4 mmol) Triethylamin und 3.81 ml (101.1 mmol) Ameisensäure in 175 ml Dioxan versetzt. Das Reaktionsgemisch wird anschließend bei 100°C 12 h lang gerührt. Nach vollständigem Umsatz wird die Reaktionslösung abgekühlt und das Lösungsmittel im Vakuum entfernt. Der Rückstand wird in Essigsäureethylester und Wasser aufgenommen, mit 1 N Salzsäure angesäuert und die organische Phase abgetrennt. Die wässrige Phase wird noch dreimal mit Essigsäureethylester extrahiert, die organischen Phasen anschließend vereinigt, mit gesättigter Natriumchlorid-Lösung gewaschen und über Natriumsulfat getrocknet. Nach Filtration wird die Lösung im Vakuum eingeengt. Das erhaltene Rohprodukt wird durch Flash-Chromatographie an Kieselgel (Laufmittel: Cyclohexan/Essigsäureethylester 4:1 → 1:1) gereinigt. Es werden 8.6 g (64% d. Th.) eines farblosen Feststoffs erhalten.
¹H-NMR (400 MHz, DMSO-d₆, δ/ppm): 12.25-12.09 (1H, breit), 7.76 (2H, d), 7.40 (2H, d), 4.04 (2H, q), 2.71-2.57 (2H, m), 2.30-2.14 (4H, m), 1.87-1.74 (1H, m), 1.73 (1H, m), 1.58-1.38 (3H, m), 1.31-1.19 (2H, m), 1.18 (3H, t).
MS (EI): 316 (M-H⁻).

### Beispiel 57A

### 8-(4-Cyanophenyl)-6-hydroxymethyl-octansäureethylester

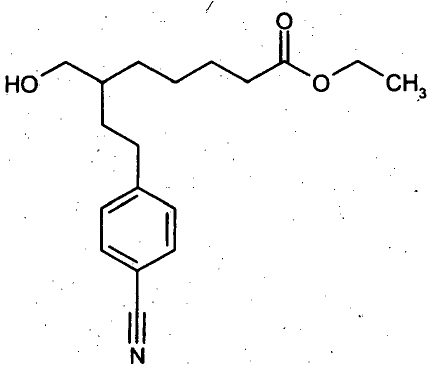

Zu einer Lösung von 8.6 g (27.1 mmol) 2-[2-(4-Cyanophenyl)ethyl]heptandisäure-7-ethylester in 200 ml THF werden 54.19 ml (54.19 mmol) einer 1 M Boran-THF-Komplex-Lösung bei -10°C zugetropft. Nach Erwärmen auf 0°C wird bei dieser Temperatur noch 2 Stunden nachgerührt. Nach vollständiger Umsetzung wird das Reaktionsgemisch mit gesättigter Natriumhydrogencarbonat-Lösung versetzt und das Lösungsmittel bis zur Trockene eingeengt. Der Rückstand wird in Dichlormethan aufgenommen, über Natriumsulfat getrocknet und wiederum vom Lösungsmittel befreit. Das erhaltene Rohprodukt wird. durch Flash-Chromatographie an Kieselgel (Laufmittel: Essigsäureethylester/Cyclohexan 1:10 → 1:4) gereinigt. Es werden 5.1 g (97%, 60% d. Th.) eines farblosen Feststoffs erhalten.
¹H-NMR (300 MHz, DMSO-d₆, δ/ppm): 7.74 (2H, d), 7.41 (2H, d), 4.41 (1H, t), 4.03 (2H, q), 3.41-3.29 (2H, m), 2.67 (2H, t), 2.28 (2H, t), 1.69-1.11 (9H, m), 1.18 (3H, t).
MS (DCI): 321 (M+NH₄⁺).

### Beispiel 58A

### 8-(4-Cyanophenyl)-6-formyl-octansäureethylester

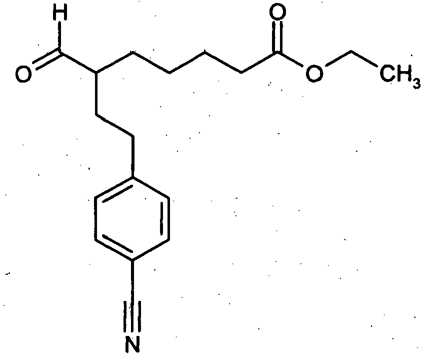

Eine Lösung von 4 g (13.18 mmol) 8-(4-Cyanophenyl)-6-hydroxymethyl-octansäureethylester in 100 ml Dichlormethan wird mit 3.41 g (15.82 mmol) Pyridiniumchlorochromat (PCC) versetzt und 12 Stunden bei Raumtemperatur gerührt. Nach vollständigem Umsatz wird das Lösungsmittel im Vakuum bis zur Trockene eingeengt. Der erhaltene Rückstand wird durch Flash-Chromatographie an Kieselgel (Laufmittel: Cyclohexan/Essigsäureethylester 10:1 → 4:1) gereinigt. Es werden 2.74 g (9.09 mmol, 69% d. Th.) eines farblosen Feststoffs erhalten.
LC-MS (Methode 2): Rₜ 2.38 min; m/z 302 (M+H⁺).

### Beispiel 59A

### E-6-[2-(4-Cyanophenyl)ethyl]-8-(2-hydroxyphenyl)-oct-7-ensäureethylester

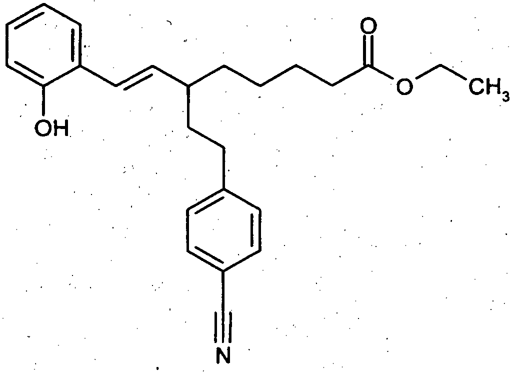

Zu einer Lösung von 1820 mg (4.05 mmol) (2-Hydroxybenzyl)-triphenylphosphoniumbromid in 15 ml wasserfreiem THF werden bei 0°C 5.91 ml (9.45 mmol) einer 1.6 M Lösung von n-Butyl-lithium in Hexan langsam hinzugefügt. Anschließend wird bei dieser Temperatur eine Lösung von 1085 mg (3.38 mmol) 8-(4-Cyanophenyl)-6-formyl-octansäureethylester in 15 ml THF langsam zudosiert. Nach Erwärmung auf Raumtemperatur wird die Reaktionslösung 12 Stunden nachgerührt, dann mit etwas Wasser versetzt und bis zur Trockene eingeengt. Der Rückstand wird in Essigsäureethylester aufgenommen, mit Wasser und gesättigter Natriumchlorid-Lösung gewaschen und über Natriumsulfat getrocknet. Nach Filtration wird das Lösungsmittel bis zur Trockene eingeengt. Das erhaltene Rohprodukt wird durch Flash-Chromatographie an Kieselgel (Laufmittel: Cyclohexan/Essigsäureethylester 4:1 → 2:1) gereinigt. Es werden 1150 mg (2.79 mmol, 83% d. Th.) eines farblosen Feststoffs erhalten.
¹H-NMR (400 MHz, DMSO-d₆, δ/ppm): 9.48 (1H, s), 7.73 (2H, d), 7.42 (2H, d), 7.38 (1H, d), 7.03 (1H, t), 6.80 (1H, d), 6.74 (1H, t), 6.57 (1H, d), 6.06-5.93 (1H, m), 4.03 (2H, q), 2.76-2.57 (2H, m), 2.26 (2H, t), 2.17-2.02 (1H, m), 1.80-1.68 (1H, m), 1.67-1.56 (1H, m), 1.56-1.39 (3H, m), 1.38-1.19 (3H, m), 1.13 (3H, t).
MS (DCI): 409 (M+NH₄⁺).

### Beispiel 60A

### E-8-[2-(4-tert.-Butylbenzyloxy)phenyl]-6-[2-(4-cyanophenyl)ethyl]-oct-7-ensäureethylester

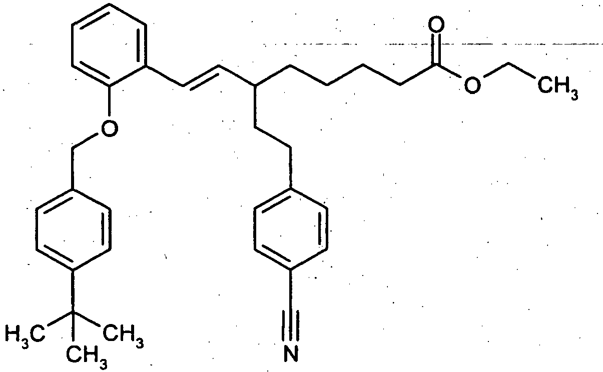

Eine Lösung von 2300 mg (5.87 mmol) *E*-6-[2-(4-Cyanophenyl)ethyl]-8-(2-hydroxyphenyl)-oct-7-ensäureethylester in 160 ml trockenem Acetonitril wird mit 1600 mg (7.05 mmol) 4-(*tert*.-Butyl)-benzylbromid und 1220 mg (8.81 mmol) wasserfreiem Kaliumcarbonat versetzt und 12 Stunden unter Rückfluss erhitzt. Anschließend wird der Ansatz zur Trockene eingeengt. Der Rückstand wird in Essigsäureethylester aufgenommen, mit Wasser und gesättigter Natriumchlorid-Lösung gewaschen, über Natriumsulfat getrocknet und die organische Phase eingeengt. Das erhaltene Rohprodukt wird durch Flash-Chromatographie an Kieselgel (Laufmittel: Cyclohexan/Essigsäureethylester 10:1) gereinigt. Es werden 2800 mg (5.21 mmol, 88% d. Th.) eines Feststoffs erhalten.
¹H-NMR (300 MHz, DMSO-d₆, δ/ppm): 7.68 (2H, d), 7.45 (1H, d), 7.41-7.32 (6H, m), 7.20 (1H, t), 7.09 (1H, d), 6.91 (1H, t), 6.61 (1H, d), 6.08-5.95 (1H, m), 5.10 (2H, s), 4.00 (2H, q), 2.77-2.45 (3H, m), 2.23 (2H, t), 2.12-1.98 (1H, m), 1.78-1.37 (5H, m), 1.32-1.19 (2H, m), 1.28 (9H, s), 1.13 (3H, t).
MS (DCI): 555 (M+NH₄⁺).

### Beispiel 61A

### (7E)-8-{2-[(4-tert.-Butylbenzyl)oxy]phenyl}-6-{2-[4-(1H-tetrazol-5-yl)phenyl]ethyl}oct-7-en-säureethylester

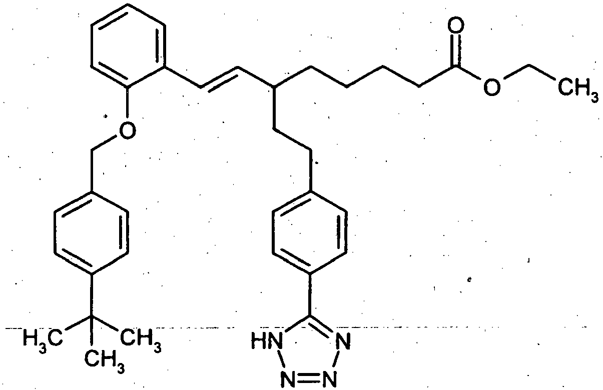

Eine Lösung von 1 g (1.86 mmol) (7*E*)-8-{2-[(4-*tert*.-Butylbenzyl)oxy]phenyl}-6-[2-(4-cyano-phenyl)ethyl]-oct-7-ensäureethylester in 70 ml Toluol wird mit 3.7 ml (27.9 mmol) Trimethylsilylazid und 694 mg (2.79 mmol) Di-*n*-butylzinnoxid versetzt und 12 Stunden auf 80°C erhitzt. Nach Abkühlen auf Raumtemperatur wird der Ansatz mit gesättigter Natriumhydrogencarbonat-Lösung gewaschen. Die organische Phase wird abgetrennt, mit gesättigter Kochsalz-Lösung gewaschen und über Natriumsulfat getrocknet. Nach Filtration wird das Filtrat bis zur Trockene eingeengt. Das erhaltene Rohprodukt wird durch Flash-Chromatographie an Kieselgel (Laufmittel: Cyclohexan/Essigsäureethylester 4:1 → 1:1) gereinigt. Es werden 622 mg (1.1 mmol, 34% d. Th.) eines weißen Schaums erhalten.
¹H-NMR (300 MHz, DMSO-d6, δ/ppm): 7.90 (2H, d), 7.48 (1H, d), 7.40-7.30 (6H, m), 7.22-7.10 (1H, m), 7.09 (1H, d), 6.90 (1H, t), 6.55 (1H, d), 6.06 (1H, dd), 5.10 (2H, s), 4.00 (2H, q), 2.77-2.52 (2H, m), 2.30-2.00 (2H, m), 1.80-1.38 (9H, m), 1.20 (9H, s), 1.10 (3H, t).

### Beispiel 62A

### (7E)-6-[2-(4-Cyanophenyl)ethyl]-8-{2-[(5-phenylpentyl)oxy]phenyl}oct-7-en-säureethylester

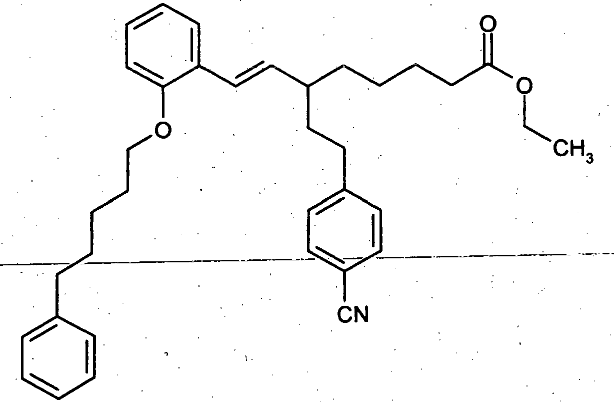

Eine Lösung von 127 mg (0.32 mmol) (7*E*)-6-[2-(4-Cyanophenyl)ethyl]-8-(2-hydroxyphenyl)-oct-7-ensäureethylester in 10 ml Acetonitril wird mit 88.42 mg (0.39 mmol) (5-Brompentyl)benzol und 67.25 mg (0.49 mmol) Kaliumcarbonat versetzt und 12 Stunden unter Rückfluss gerührt. Nach dem Abkühlen wird der Ansatz eingeengt. Der Rückstand wird in Essigsäureethylester aufgenommen und mit Wasser ausgeschüttelt. Die organische Phase wird mit gesättigter Natriumchlorid-Lösung gewaschen, über Natriumsulfat getrocknet und eingeengt. Der erhaltene Rückstand wird über präparative HPLC gereinigt. Es werden 128 mg (0.24 mmol, 73.4% d. Th.) der Titelverbindung erhalten.
LC-MS (Methode 2): Rₜ = 3.42 min.
MS (ESIpos): m/z= 538 (M+H)⁺.

### Beispiel 63A

### (7E)-8-{2-[(5-Phenylpentyl)oxy]phenyl}-6-{2-[4-(1H-tetrazol-5-yl)phenyl]ethyl}oct-7-ensäure-ethylester

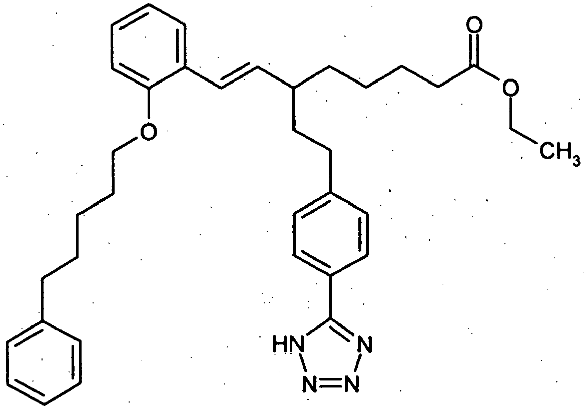

Eine Lösung von 128 mg (0.24 mmol) (7*E*)-6-[2-(4-Cyanophenyl)ethyl]-8-{2-[(5-phenylpentyl)-oxy]phenyl}oct-7-ensäureethylester in 10 ml Toluol wird mit 0.47 ml (3.57 mmol) Trimethylsilylazid und 88.9 mg (0.36 mmol) Di-*n*-butylzinnoxid versetzt und 12 Stunden auf 80°C erhitzt. Nach Abkühlen auf Raumtemperatur wird der Ansatz mit gesättigter Natriumhydrogencarbonat-Lösung gewaschen. Die organische Phase wird abgetrennt, mit gesättigter Kochsalz-Lösung gewaschen und über Natriumsulfat getrocknet. Nach Filtration wird das Filtrat bis zur Trockene eingeengt. Das erhaltene Rohprodukt wird durch präparative HPLC gereinigt. Es werden 62 mg (0.11 mmol, 44.8% d. Th.) eines farblosen Schaums erhalten.
LC-MS (Methode 2): Rₜ = 3.23 min.
MS (ESIpos): m/z = 581 (M+H)⁺.

### Beispiel 64A

### 2-Allyloxycarbonyl-2-(4-cyanobenzyl)-heptandisäure-1-allylester-7-ethylester

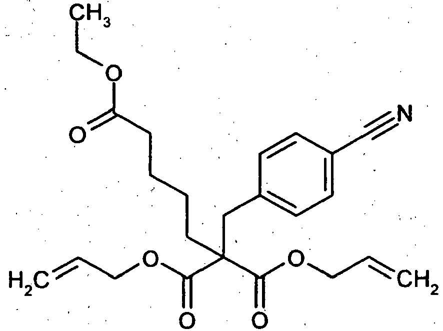

Eine Lösung von 20 g (64.03 mmol) 2-Allyloxycarbonyl-heptandisäure-1-allylester-7-ethylester in 140 ml DMF wird bei 0°C portionsweise mit 1.69 g (70.43 mmol) Natriumhydrid versetzt. Anschließend lässt man die Reaktionslösung auf Raumtemperatur kommen und rührt 30 min lang nach. Die Reaktionslösung wird wieder auf 0°C abgekühlt, mit 16.32 g (83.24 mmol) 4-Brommethylbenzonitril in 140 ml DMF versetzt und 30 min bei dieser Temperatur nachgerührt. Der Ansatz wird danach bei Raumtemperatur über Nacht gerührt. Das Reaktionsgemisch wird tropfenweise mit Wasser versetzt, mit Essigsäureethylester dreimal extrahiert, die vereinigten organischen Phasen mit gesättigter Kochsalz-Lösung gewaschen und über Natriumsulfat getrocknet. Nach der Filtration wird das Lösungsmittel im Vakuum bis zur Trockene eingeengt. Das erhaltene Rohprodukt wird durch Flash-Chromatographie an Kieselgel (Laufmittel: Cyclohexan/Essigsäureethylester 10:1 → 4:1 → 1:1) gereinigt. Es werden 20.08 g (46.97 mmol, 73% d. Th.) eines farblosen Feststoffs erhalten.
¹H-NMR (400 MHz, CDCl₃, δ/ppm): 7.56 (2H, d), 7.22 (2H, d), 5.91-5.78 (2H, m), 5.37-5.18 (4H, m), 4.66-4.54 (4H, m), 4.13 (2H, q), 3.29 (2H, s), 2.31 (2H, t), 1.87-1.77 (2H, m), 1.69-1.57 (2H, m), 1.39-1.28 (2H, m), 1.26 (3H, t).
MS (DCI): 445 (M+NH₄⁺).

### Beispiel 65A

### 2-(4-Cyanobenzyl)heptandisäure-7-ethylester

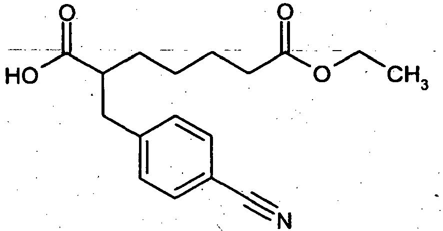

Eine Lösung von 22.5 g (52.63 mmol) 2-Allyloxycarbonyl-2-(4-cyanobenzyl)-heptandisäure-1-allylester-7-ethylester, 970 mg (3.68 mmol) Triphenylphosphin und 240 mg (1.05 mmol) Palladiumacetat in 500 ml Dioxan wird bei Raumtemperatur mit einer Lösung von 24.21 ml (173.69 mmol) Triethylamin und 4.96 ml (131.58 mmol) Ameisensäure in 500 ml Dioxan versetzt. Das Reaktionsgemisch wird anschließend 2 Stunden lang bei 100°C gerührt. Nach vollständigem Umsatz wird die Reaktionslösung abgekühlt und das Lösungsmittel im Vakuum entfernt. Der Rückstand wird in Essigsäureethylester und Wasser aufgenommen, mit 1 N Salzsäure angesäuert und die organische Phase abgetrennt. Die wässrige Phase wird noch dreimal mit Essigsäureethylester extrahiert, die organischen Phasen anschließend vereinigt, mit gesättigter Kochsalz-Lösung gewaschen und über Natriumsulfat getrocknet. Nach Filtration wird die Lösung im Vakuum eingeengt. Es werden 17.5 g (87% d. Th., 80% Reinheit) eines farblosen Feststoffs erhalten.
LC-MS (Methode 2): Rₜ = 1.97 min; m/z = 304 (M+H⁺).

### Beispiel 66A

### 6-(4-Cyanobenzyl)-7-hydroxyheptansäureethylester

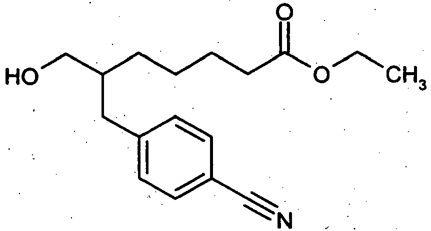

Zu einer Lösung von 6.64 g (22.94 mmol) 2-(4-Cyanobenzyl)-heptandisäure-7-ethylester in 260 ml THF werden 34.42 ml einer 1 M Boran-THF-Komptex-Lösung (34.42 mmol) bei -10°C zugetropft. Nach Erwärmen auf 0°C wird bei dieser Temperatur noch 2 Stunden nachgerührt. Nach vollständiger Umsetzung wird das Reaktionsgemisch mit gesättigter Natriumhydrogencarbonat-Lösung versetzt und das Lösungsmittel bis zur Trockene eingeengt. Der Rückstand wird in Dichlormethan aufgenommen, über Natriumsulfat getrocknet und wiederum vom Lösungsmittel befreit. Das erhaltene Rohprodukt wird durch Flash-Chromatographie an Kieselgel (Laufmittel: Essigsäureethylester/Cyclohexan 1:8 → 1:2) gereinigt. Es werden 5.84 g (88% d. Th., 20.19 mmol) eines farblosen Feststoffs erhalten.
¹H-NMR (400 MHz, DMSO-d₆, δ/ppm): 8.14 (2H, d), 7.80 (2H, d), 4.91 (1H, t), 4.44 (2H, q), 3.69-3.58 (2H, m), 3.16-3.06 (1H, m), 3.01-2.92 (1H, m), 2.62 (2H, t), 2.14-2.01 (1H, m), 1.92-1.78 (2H, m), 1.77-1.60 (3H, m), 1.59-1.48 (1H, m), 1.57 (3H, t).
MS (DCI): 307 (M+NH₄⁺).

### Beispiel 67A

### 6-(4-Cyanobenzyl)-7-oxo-heptansäureethylester

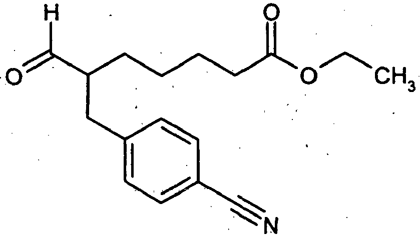

Eine Lösung von 4.6 g (15.90 mmol) 6-(4-Cyanobenzyl)-7-hydroxyheptansäureethylester in 250 ml Dichlormethan wird mit 4.11 g (19.08 mmol) Pyridiniumchlorochromat (PCC) versetzt und 12 Stunden bei Raumtemperatur gerührt. Nach vollständigem Umsatz werden 10 g Kieselgel zugegeben und das Lösungsmittel vorsichtig im Vakuum bis zur Trockene eingeengt. Der erhaltene Rückstand wird durch Flash-Chromatographie an Kieselgel (Laufmittel: Cyclohexan/Essigsäureethylester 1:1) gereinigt. Es werden 4.09 g (14.23 mmol, 89% d. Th.) eines farblosen Feststoffes erhalten.
¹H-NMR (400 MHz, DMSO-d₆, δ/ppm): 9.60 (1H, s), 7.75 (2H, d), 7.41 (2H, d), 4.03 (2H, q), 3.08-2.97 (1H, m), 2.82-2.64 (2H, m), 2.24 (2H, t), 1.63-1.19 (6H, m), 1.17 (3H, t).
MS (DCI): 305 (M+NH₄⁺).

### Beispiel 68A

### E-6-(4-Cyanobenzyl)-8-(2-hydroxyphenyl)-oct-7-ensäureethylester

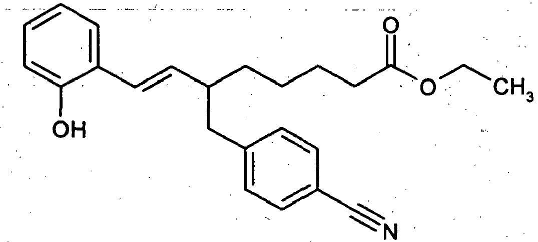

Zu einer Lösung von 6.411 g (14.27 mmol) (2-Hydroxybenzyl)-triphenylphosphoniumbromid in 300 ml wasserfreiem THF werden bei 0°C 15.98 ml (39.95 mmol) einer 2.5 M Lösung von n-Butyllithium in Hexan langsam hinzugefügt. Anschließend werden bei dieser Temperatur 4.1 g (14.27 mmol) 6-(4-Cyanobenzyl)-7-oxo-heptansäureethylester langsam zudosiert. Nach Erwärmung auf Raumtemperatur wird die Reaktionslösung 12 Stunden nachgerührt, dann mit gesättigter Ammoniumchlorid-Lösung versetzt und bis zur Trockene eingeengt. Der Rückstand wird in Essigsäureethylester aufgenommen, mit Wasser und gesättigter Kochsalz-Lösung gewaschen und über Natriumsulfat getrocknet. Nach Filtration wird das Lösungsmittel bis zur Trockene eingeengt. Das erhaltene Rohprodukt wird durch Flash-Chromatographie an Kieselgel (Laufmittel: Cyclohexan/ Essigsäureethylester 4:1) gereinigt. Es werden 1.75 g (4.64 mmol, 32% d. Th.) eines farblosen Feststoffs erhalten.
¹H-NMR (400 MHz, DMSO-d₆, δ/ppm): 9.42 (1H, s), 7.72 (2H, d), 7.40 (2H, d), 7.29 (1H, d), 7.00 (1H, t), 6.79-6.67 (2H, m), 6.39 (1H, d), 6.04-5.94 (1H, m), 4.01 (2H, q), 2.87-2.77 (1H, m), 2.76-2.66 (1H, m), 2.48-2.38 (1H, m), 2.25 (2H, t), 1.57-1.39 (3H, m), 1.38-1.19 (3H, m), 1.13 (3H, t).
MS (DCI): 395 (M+NH₄⁺).

### Beispiel 69A

### E-8-[2-(4-tert.-Butylbenzyloxy)phenyl]-6-(4-cyanobenzyl)-oct-7-ensäureethylester

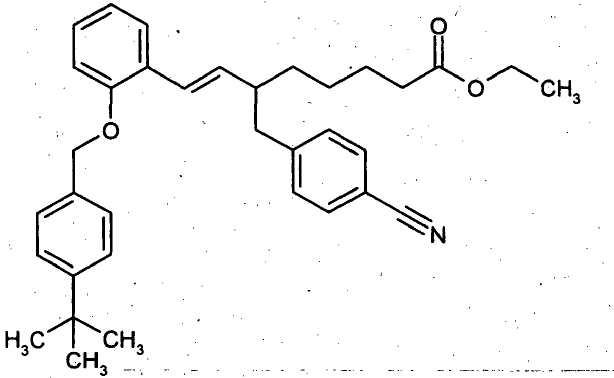

Eine Lösung von 1.75 g (4.64 mmol) *E*-6-(4-Cyanobenzyl)-8-(2-hydroxyphenyl)-oct-7-ensäureethylester in 50 ml trockenem Acetonitril wird mit 1579 mg (6.95 mmol) 4-(*tert*.-Butyl)benzylbromid und 961 mg (6.95 mmol) wasserfreiem Kaliumcarbonat versetzt und 12 Stunden unter Rückfluss erhitzt. Anschließend wird der Ansatz zur Trockene eingeengt. Der Rückstand wird in Essigsäureethylester aufgenommen, mit Wasser und gesättigter Kochsalz-Lösung gewaschen und über Natriumsulfat getrocknet. Die organische Phase wird eingeengt. Das erhaltene Rohprodukt wird durch Flash-Chromatographie an Kieselgel (Laufmittel: Cyclohexan/Essigsäureethylester 8:1 → 4:1) gereinigt. Es werden 2.24 g (4.28 mmol, 92% d. Th.) eines Feststoffs erhalten.
¹H-NMR (300 MHz, DMSO-d₆, δ/ppm): 7.68 (2H, d), 7.44-7.32 (5H, m), 7.28 (2H, d), 7.14 (1H, t), 7.01 (1H, d), 6.88 (1H, t), 6.42 (1H, d), 6.08-5.95 (1H, m), 5.04 (2H, s), 4.00 (2H, q), 2.89-2.78 (1H, m), 2.75-2.60 (2H, m), 2.54-2.40 (1H, m), 2.23 (2H, t), 1.60-1.21 (5H, m), 1.28 (9H, s), 1.13 (3H, t).
MS (DCI): 541 (M+NH₄⁺).

### Beispiel 70A

### (7E)-8-{2-[(4-tert.-Butylbenzyl)oxy]phenyl}-6-[4-(1H-tetrazol-5-yl)benzyl]oct-7-ensäureethylester

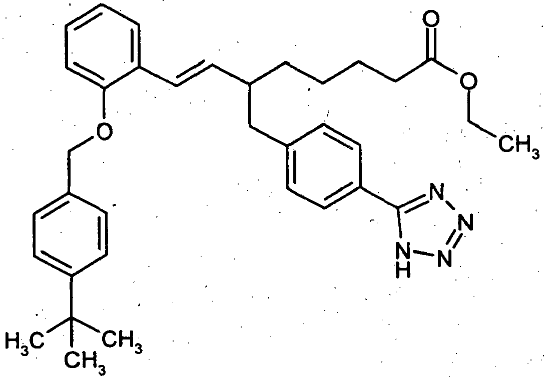

Eine Lösung von 1000 mg (1.91 mmol) (7*E*)-8-{2-[(4-*tert*.-Butylbenzyl)oxy]phenyl}-6-(4-cyanobenzyl)-oct-7-ensäureethylester in 70 ml Toluol wird mit 3.8 ml (28.6 mmol) Trimethylsilylazid und 713 mg (2.86 mmol) Di-*n*-butylzinnoxid versetzt und 12 Stunden auf 80°C erhitzt. Nach Abkühlen auf Raumtemperatur wird der Ansatz mit gesättigter Natriumhydrogencarbonat-Lösung gewaschen. Die organische Phase wird abgetrennt, mit gesättigter Kochsalz-Lösung gewaschen und über Natriumsulfat getrocknet. Nach Filtration wird das Filtrat bis zur Trockene eingeengt. Das erhaltene Rohprodukt wird durch Flash-Chromatographie an Kieselgel (Laufmittel: Dichlormethan → Dichlormethan/Methanol 10:1) gereinigt. Es werden 800 mg (1.4 mmol, 74% d. Th.) eines weißen Schaums erhalten.
MS (DCI): m/z = 541 (M+NH₄)⁺.

### Beispiel 71A

### (7E/Z)-6-(4-Cyanobenzyl)-8-{2-[(5-phenylpentyl)oxy]phenyl}oct-7-ensäureethylester

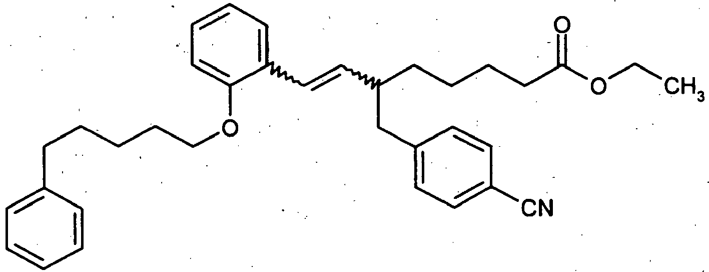

Zu einer Lösung von 1695 mg (2.85 mmol) Triphenyl-{2-[(5-phenylpentyl)oxy]benzyl}phosphoniumbromid in 15 ml THF werden bei 0°C 2.13 ml (3.42 mmol) einer 1.6 M Lösung von n-Butyllithium in Hexan langsam hinzugefügt. Anschließend werden bei dieser Temperatur 818 mg (2.85 mmol) 6-(4-Cyanobenzyl)-7-oxo-heptansäureethylester in 15 ml THF langsam zudosiert. Nach Erwärmung auf Raumtemperatur wird die Reaktionslösung 12 Stunden nachgerührt, dann mit etwas Wasser versetzt und bis zur Trockene eingeengt. Der Rückstand wird in Essigsäureethylester aufgenommen, mit Wasser und gesättigter Natriumchlorid-Lösung gewaschen und über Natriumsulfat getrocknet. Nach Filtration wird das Lösungsmittel bis zur Trockene eingeengt. Das erhaltene Rohprodukt wird durch Flash-Chromatographie an Kieselgel (Laufmittel: Cyclohexan/Essigsäureethylester 50:1 → 20:1) gereinigt. Es werden 730 mg (1.4 mmol, 48% d. Th.) eines weißen Schaums erhalten.
MS (DCI): m/z = 541 (M+NH₄)⁺.

### Beispiel 72A

### (7E/Z)-8-{2-[(5-Phenylpentyl)oxy]phenyl}-6-[4-(1H-tetrazol-5-yl)benzyl]oct-7-ensäureethylester

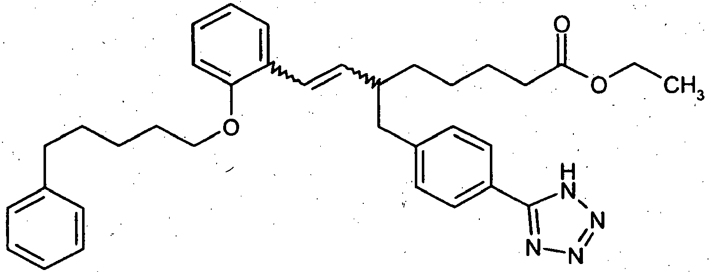

Eine Lösung von 100 mg (0.19 mmol) (7*E*/*Z*)-6-(4-Cyanobenzyl)-8-{2-[(5-phenylpentyl)oxy]-phenyl}-oct-7-ensäureethylester in 10 ml Toluol wird mit 0.05 ml (0.38 mmol) Trimethylsilylazid und 4.75 mg (0.02 mmol) Di-n-butylzinnoxid versetzt und 12 Stunden auf 80°C erhitzt. Nach Abkühlen auf Raumtemperatur wird der Ansatz mit gesättigter Natriumhydrogencarbonat-Lösung gewaschen. Die organische Phase wird abgetrennt, mit gesättigter Kochsalz-Lösung gewaschen und über Natriumsulfat getrocknet. Nach Filtration wird das Filtrat bis zur Trockene eingeengt. Das erhaltene Rohprodukt wird durch präparative HPLC gereinigt. Es werden 19 mg (0.03 mmol, 16.9% d. Th.) eines farblosen Schaums erhalten.
MS (ESIpos): m/z = 584 (M+NH₄)⁺.

### Ausführungsbeispiele:

### Beispiel 1

### 4-((3E)-4-{2-[(4-tert.-Butyl-2-chlorbenzyl)oxy]phenyl}-2-{2-[4-(1H-tetrazol-5-yl)phenyl]ethyl}but-3-en-1-yl)benzoesäure

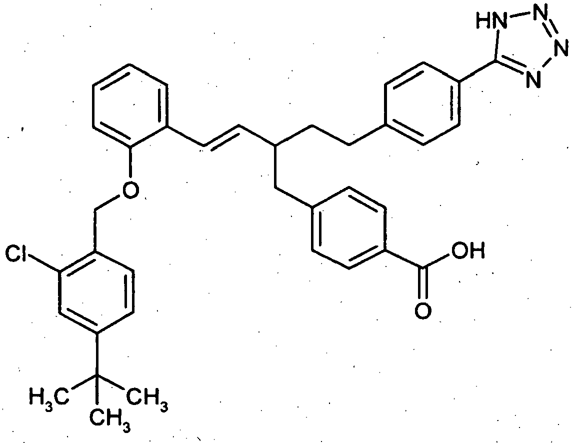

Eine Lösung von 500 mg (0.79 mmol) 4-((3*E*)-4-{2-[(4-tert.-Butyl-2-chlorbenzyl)oxy]phenyl}-2{2-[4-(1*H*-tetrazol-5-yl)phenyl]ethyl}but-3-en-1-yl)benzoesäuremethylester in 3 ml THF und 1.5 ml Wasser wird mit 75.4 mg (3.15 mmol) Lithiumhydroxid versetzt und 12 Stunden bei 60°C gerührt. Nach dem Abkühlen wird das THF abgezogen und die wässrige Phase mit 1 M Salzsäure auf pH 2 gestellt. Anschließend wird mit Essigsäureethylester dreimal extrahiert und die vereinigten organischen Phasen über Natriumsulfat getrocknet. Nach Filtration und Einengen wird der erhaltene Rückstand über eine Kieselgelsäule chromatographisch gereinigt (Laufmittel: Essigsäureethylester/Cyclohexan 1:1). Es werden 372 mg (0.6 mmol, 76% d. Th.) eines weißen Schaums erhalten.
¹H-NMR (300 MHz, DMSO-d₆, δ/ppm): 12.76 (1H, breit), 7.9 (2H, d), 7.79 (2H, d), 7.48-7.15 (9H, m),7.06(1H,d),6.93(1H,t),6.49(1H,d),6.12(1H,dd), 5.11 (2H, s), 2.91-2.8 (1H, m), 2.79-2.57 (3H, m), 2.57-2.42 (1H, m), 1.88-1.58 (2H, m), 1.38 (9H, s).

350 mg (0.56 mmol) 4-((3*E*)-4-{2-[(4-*tert*.-Butyl-2-chlorbenzyl)oxy]phenyl}-2-{2-[4-(1*H*-tetrazol-5-yl)phenyl]ethyl}but-3-en-1-yl)benzoesäure werden mittels präparativer HPLC an chiraler Phase weiter aufgetrennt. Es werden jeweils enantiomerenrein 156 mg bzw. 132 mg der beiden E-Isomere als farblose Feststoffe erhalten (siehe Beispiele 2 und 3).

### Beispiel 2

### 4-((3E)-4-{2-[(4-tert.-Butyl-2-chlorbenzyl)oxy]phenyl}-2-{2-[4-(1H-tetrazol-5-yl)phenyl]ethyl}-but-3-en-1-yl)benzoesäure (Enantiomer 1)

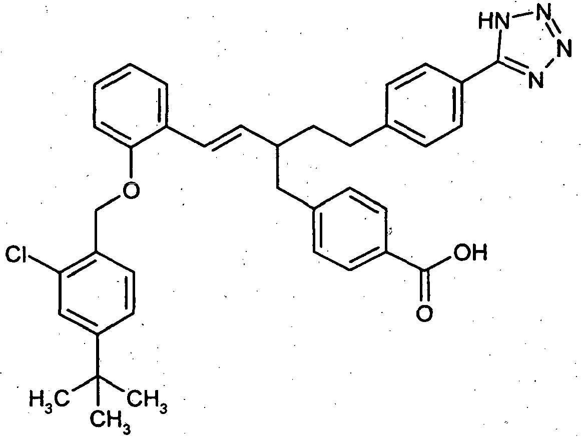

### Methode Enantiomerentrennung:

Säule: Daicel Chiralpak AD-H 250 x 20 mm; Elutionsmittel: Isohexan (mit 1% Wasser und 0.2% Essigsäure) / Isopropanol 70:30 (v/v); Fluss: 15 ml/min; UV-Detektion: 220 nm; Temperatur: 27°C.
Rₜ 6.73 min; Reinheit 99%; >99% ee
Ausbeute: 156 mg.

### Beispiel 3

### 4-((3E)-4-{2-[(4-tert.-Butyl-2-chlorbenzyl)oxy]phenyl}-2-{2-[4-(1H-tetrazol-5-yl)phenyl]ethyl}-but-3-en-1-yl)benzoesäure (Enantiomer 2)

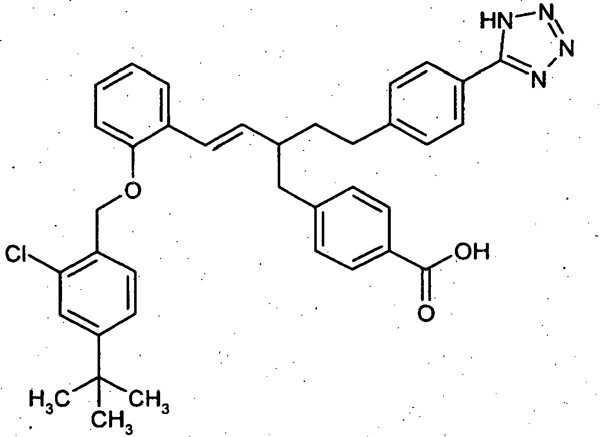

Methode Enantiomerentrennung: siehe Beispiel 2.
Rₜ 7.4 min; Reinheit 99%; >99% ee
Ausbeute: 132 mg.

### Beispiel 4

### 4-((3E)-4-{2-[(4-tert.-Butylbenzyl)oxy]phenyl}-2-{2-[4-(1H-tetrazol-5-yl)phenyl]ethyl}but-3-en-1-yl)benzoesäure

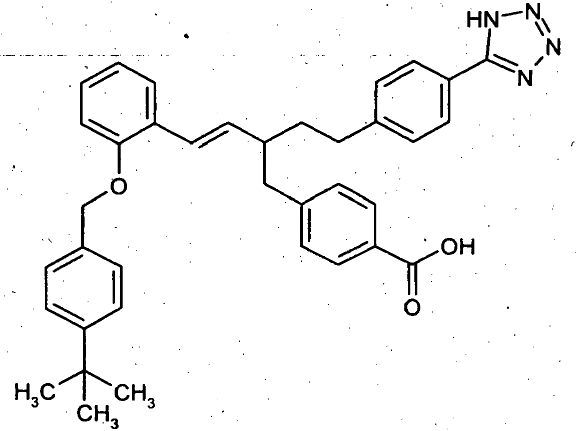

Eine Lösung von 150 mg (0.25 mmol) 4-((3*E*)-4-{2-[(4-tert.-Butylbenzyl)oxy]phenyl}-2-{2-[4-(1*H*-tetrazol-5-yl)phenyl]ethyl}but-3-en-1-yl)benzoesäuremethylester in 1.5 ml THF und 0.75 ml Wasser wird mit 24 mg (1 mmol) Lithiumhydroxid versetzt und 12 Stunden bei 60°C gerührt. Nach dem Abkühlen wird das THF abgezogen und die wässrige Phase mit 1 M Salzsäure auf pH 2 gestellt. Anschließend wird mit Essigsäureethylester dreimal extrahiert und die vereinigten organischen Phasen über Natriumsulfat getrocknet. Nach Filtration und Einengen wird der erhaltene Rückstand über eine Kieselgelsäule chromatographisch gereinigt (Laufmittel: Cyclohexan/Essig-säureethylester 2.5:1). Es werden 108 mg (0.18 mmol, 68.6% d. Th.) eines weißen Schaums erhal-ten.
¹H-NMR (400 MHz, DMSO-d₆, δ/ppm): 12.75 (1H, breit), 7.91 (2H, d), 7.81 (2H, d), 7.45-7.25 (9H, m), 7.21-7.13 (1H, m), 7.02 (1H, d), 6.89 (1H, t), 6.5 (1H, d), 6.13 (1H, dd), 5.05 (2H, s), 2.92-2.83 (1H, m), 2.8-2.53 (4H, m), 1.9-1.76 (1H, m), 1.76-1.62 (1H, m), 1.23 (9H, s).

880 mg (1.5 mmol) 4-((3*E*)-4-{2-[(4-*tert*.-Butylbenzyl)oxy]phenyl}-2-{2-[4-(1*H-*tetrazol-5-yl)-phenyl]ethyl}but-3-en-1-yl)benzoesäure werden mittels präparativer HPLC an chiraler Phase weiter aufgetrennt. Es werden jeweils enantiomerenrein 208 mg bzw. 236 mg der beiden *E*-Isomere als farblose Feststoffe erhalten (siehe Beispiele 5 und 6).

### Beispiel 5

### 4-((3E)-4-{2-[(4-tert.-Butylbenzyl)oxy]phenyl}-2-{2-[4-(1H-tetrazol-5-yl)phenyl]ethyl}but-3-en-1-yl)benzoesäure (Enantiomer 1)

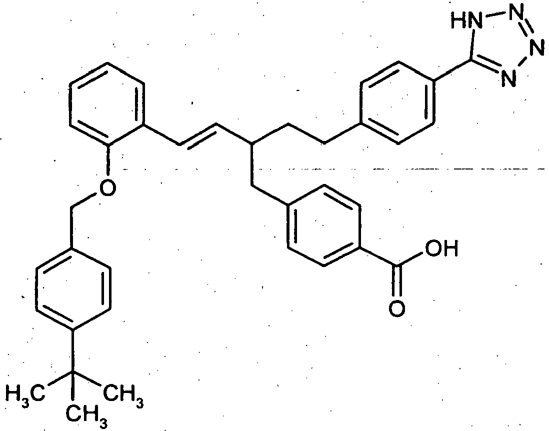

### Methode Enantiomerentrennung:

Säule: Daicel Chiralpak AD-H 250 x 20 mm; Elutionsmittel: Isohexan (mit 1% Wasser und 1% Essigsäure) / Isopropanol 70:30 (v/v); Fluss: 15 ml/min; UV-Detektion: 215 nm; Temperatur: 25°C.
Rₜ 8.27 min; Reinheit 99%; >99% ee
Ausbeute: 208 mg.

### Beispiel 6

### 4-((3E)-4-{2-[(4-tert.-Butylbenzyl)oxy]phenyl}-2-{2-[4-(1H-tetrazol-5-yl)phenyl]ethyl}but-3-en-1-yl)benzoesäure (Enantiomer 2)

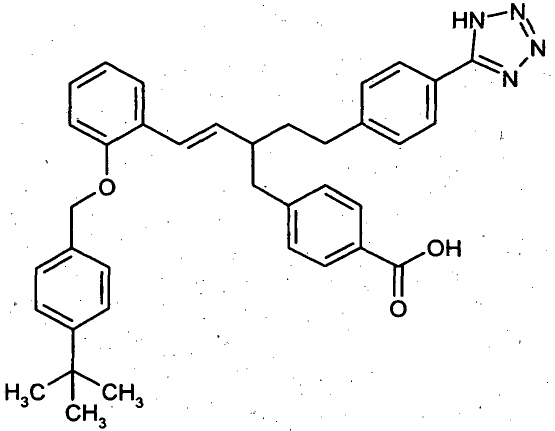

Methode Enantiomerentrennung: siehe Beispiel 5.
Rₜ 9.16 min; Reinheit 99%; >98% ee
Ausbeute: 236 mg
LC-MS (Methode 1): Rₜ = 3.11 min.
MS (ESIneg): m/z = 585 (M-H)⁻.

### Beispiel 7

### 4-((3E)-4-{2-[(2-Chlorbenzyl)oxy]phenyl}-2-{2-[4-(1H-tetrazol-5-yl)phenyl]ethyl}but-3-en-1-yl)-benzoesäure

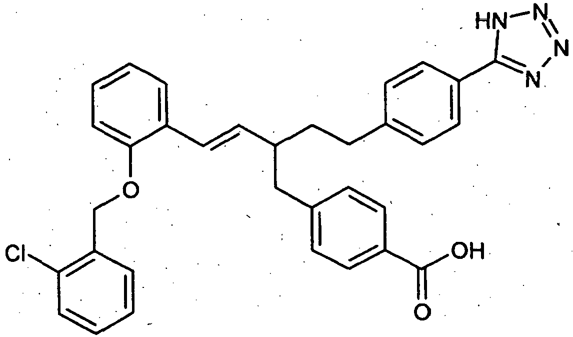

Eine Lösung von 95 mg (0.16 mmol) 4-((3*E*)-4-{2-[(2-Chlorbenzyl)oxy]phenyl}-2-{2-[4-(1*H*-tetra-zol-5-yl)phenyl]ethyl}but-3-en-1-yl)benzoesäuremethylester in 1 ml THF und 0.5 ml Wasser wird mit 15.7 mg (0.66 mmol) Lithiumhydroxid versetzt und 12 Stunden bei 60°C gerührt. Nach dem Abkühlen wird das THF abgezogen und die wässrige Phase mit 1 M Salzsäure auf pH 2 gestellt. Anschließend wird mit Essigsäureethylester dreimal extrahiert und die vereinigten organischen Phasen über Natriumsulfat getrocknet. Nach Filtration und Einengen wird der erhaltene Rückstand über eine Kieselgelsäule chromatographisch gereinigt (Laufmittel: Dichlormethan/Methanol 10:1). Es werden 42.6 mg (0.08 mmol, 41.4% d. Th.) eines weißen Schaums erhalten.
¹H-NMR (300 MHz, DMSO-d₆, δ/ppm): 12.75 (1H, breit), 7.9 (2H, d), 7.8 (2H, d), 7.55-7.48 (2H, m), 7.44 (1H, d), 7.4-7.3 (5H, m), 7.29-7.17 (2H, m), 7.06 (1H, d), 6.94 (1H, t), 6.5 (1H, d), 6.12 (1H, dd), 5.16 (2H, s), 2.9-2.8 (1H, m), 2.82-2.58 (3H, m), 2.58-2.42 (1H, m), 1.88-1.59 (2H, m).

85 mg (0.15 mmol) 4-((3*E*)-4-{2-[(2-Chlorbenzyl)oxy]phenyl}-2-{2-[4-(1*H*-tetrazol-5-yl)phenyl]-ethyl}but-3-en-1-yl)benzoesäure werden mittels präparativer HPLC an chiraler Phase weiter aufgetrennt. Es werden jeweils enantiomerenrein 39.1 mg bzw. 38.4 mg der beiden *E*-Isomere als farblose Feststoffe erhalten (siehe Beispiele 8 und 9).

### Beispiel 8

### 4-((3E)-4-{2-[(2-Chlorbenzyl)oxy]phenyl}-2-{2-[4-(1H-tetrazol-5-yl)phenyl]ethyl}but-3-en-1-yl)-benzoesäure (Enantiomer 1)

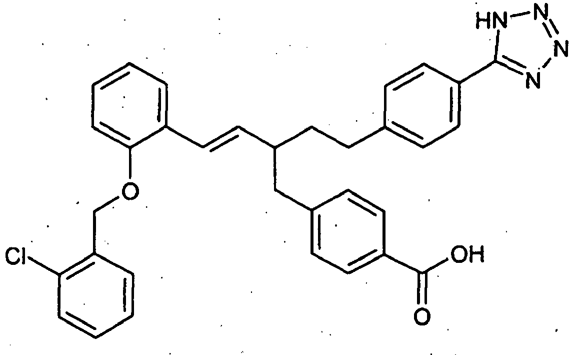

### Methode Enantiomerentrennung:

Säule: Daicel Chiralpak AD-H 250 x 20 mm; Elutionsmittel: Isohexan (mit 1% Wasser und 0.2% Trifluoressigsäure) / Ethanol 85:15 (v/v); Fluss: 15 ml/min; UV-Detektion: 220 nm; Temperatur: 30°C.
1;4 20.47 min.
Ausbeute: 39.1 mg
LC-MS (Methode 3): Rₜ = 2.97 min.
MS (ESIpos): m/z = 565 (M+H)⁺.

### Beispiel 9

### 4-((3E)-4-{2-[(2-Chlorbenzyl)oxy]phenyl}-2-{2-[4-(1H-tetrazol-5-yl)phenyl]ethyl}but-3-en-1-yl)-benzoesäure (Enantiomer 2)

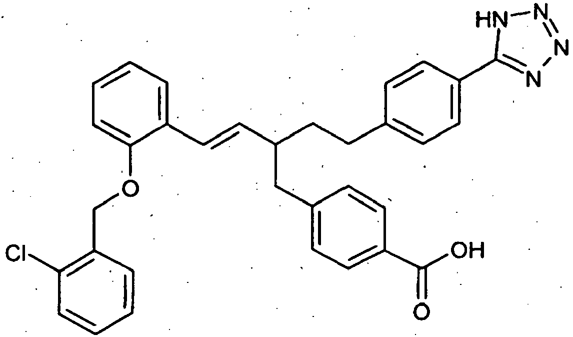

Methode Enantiomerentrennung: siehe Beispiel 8.
Rₜ 23.38 min.
Ausbeute: 38.4 mg.

### Beispiel 10

### 4-[(3E)-2-{2-[4-(1H-Tetrazol-5-yl)phenyl]ethyl}-4-(2-{[2-(trifluormethyl)benzyl]oxy}phenyl)but-3-en-1-yl]benzoesäure

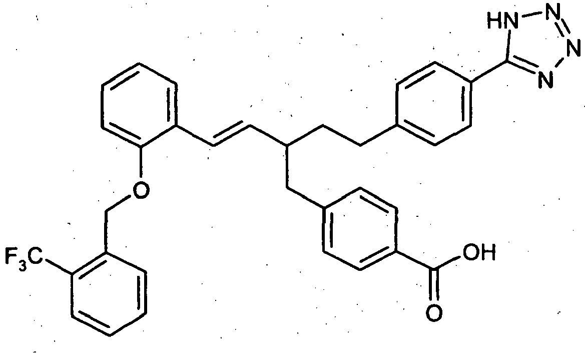

Eine Lösung von 120 mg (0.2 mmol) 4-[(3*E*)-2-{2-[4-(1*H*-Tetrazol-5-yl)phenyl]ethyl}-4-(2-{[2-(trifluormethyl)benzyl]oxy}phenyl)but-3-en-l-yl]benzoesäuremethylester in 1 ml THF und 0.5 ml Wasser wird mit 18.8 mg (0.78 mmol) Lithiumhydroxid versetzt und 12 Stunden bei 60°C gerührt. Nach dem Abkühlen wird das THF abgezogen und die wässrige Phase mit 1 M Salzsäure auf pH 2 gestellt. Anschließend wird mit Essigsäureethylester dreimal extrahiert und die vereinigten organischen Phasen über Natriumsulfat getrocknet. Nach Filtration und Einengen wird der erhaltene Rückstand über eine Kieselgelsäule chromatographisch gereinigt (Laufmittel: Cyclohexan/Essig-säureethylester 3:1 → 100% Essigsäureethylester). Es werden 109 mg (0.18 mmol, 91.5% d. Th.) eines weißen Schaums erhalten.
¹H-NMR (400 MHz, DMSO-d₆, δ/ppm): 16.72 (1H, breit), 12.72 (1H, breit), 7.88 (2H, d), 7.8 (3H, d), 7.69-7.62 (2H, m), 7.59-7.52 (1H, m), 7.45 (1H, d), 7.35 (2H, d), 7.26 (1H, d), 7.22-7.16 (1H, m), 6.95 (1H, t), 6.49 (1H, d), 6.1 (1H, dd), 5.22 (2H, s), 2.9-2.8 (1H, m), 2.76-2.65 (2H, m), 2.65-2.55 (1H, m), 1.87-1.72 (1H, m), 1.72-1.58 (1H, m), 1.37-1.2 (1H, m).

90 mg (0.15 mmol) 4-[(3*E*)-2-{2-[4-(1*H*-Tetrazol-5-yl)phenyl]ethyl}-4-(2-{[2-(trifluormethyl)-benzyl]oxy}phenyl)but-3-en-1-yl]benzoesäure werden mittels präparativer HPLC an chiraler Phase weiter aufgetrennt. Es werden jeweils enantiomerenrein 44.3 mg bzw. 41.8 mg der beiden *E*-Isomere als farblose Feststoffe erhalten (siehe Beispiele 11 und 12).

### Beispiel 11

### 4-[(3E)-2-{2-[4-(1H-Tetrazol-5-yl)phenyl]ethyl}-4-(2-{[2-(trifluormethyl)benzyl]oxy}phenyl)but-3-en-1-yl]benzoesäure (Enantiomer 1)

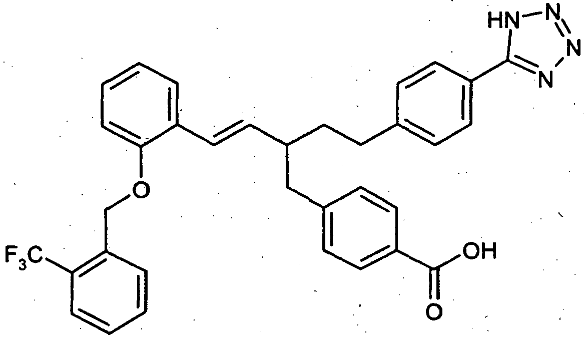

### Methode Enantiomerentrennung:

Säule: Daicel Chiralpak AD-H 250 x 20 mm; Elutionsmittel: Isohexan (mit 1% Wasser und 0.2% Trifluoressigsäure) / Ethanol 85:15 (v/v); Fluss: 15 ml/min; UV-Detektion: 220 nm; Temperatur: 30°C.
Rₜ 13.14 min.
Ausbeute: 44.3 mg
LC-MS (Methode 3): Rₜ = 2.98 min.
MS (ESIpos): m/z = 599 (M+H)⁺.

### Beispiel 12

### 4-[(3E)-2-{2-[4-(1H-Tetrazol-5-yl)phenyl]ethyl}-4-(2-{[2-(trifluormethyl)benzyl]oxy}phenyl)but-3-en-1-yl]benzoesäure (Enantiomer 2)

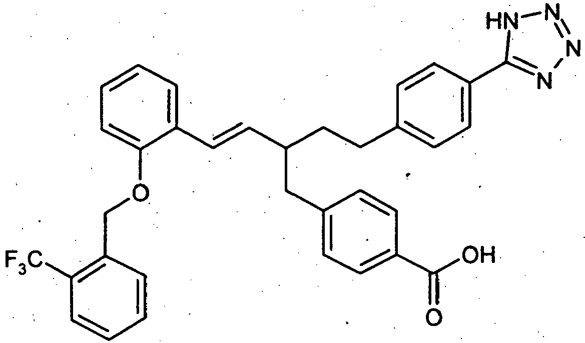

Methode Enantiomerentrennung: siehe Beispiel 11.
Rₜ 14.63 min.
Ausbeute: 41.8 mg.

### Beispiel 13

### 4-{(4E)-5-{2-[(5-Phenylpentyl)oxy]phenyl}-3-[4-(1H-tetrazol-5-yl)benzyl]pent-4-en-1-yl}benzoe-säure

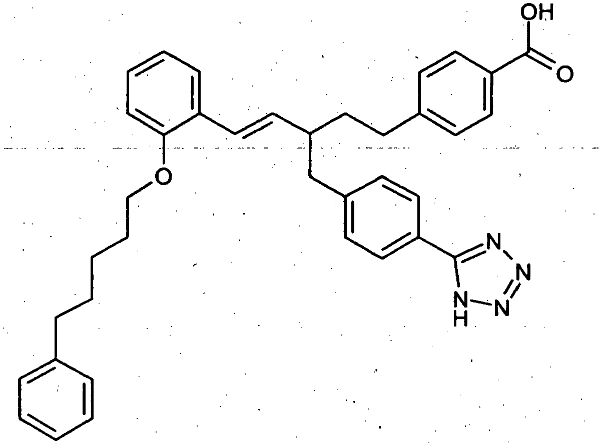

Eine Lösung von 284 mg (0.47 mmol) 4-{(4*E*)-5-{2-[(5-Phenylpentyl)oxy]phenyl}-3-[4-(1*H*-tetrazol-5-yl)benryl]pent-4-en-1-yl}benzoesäuremethylester in 10 ml THF und 10 ml Wasser wird mit 22.6 mg (0.95 mmol) Lithiumhydroxid versetzt und 12 Stunden bei 50°C gerührt. Nach dem Abkühlen wird das THF abgezogen und die wässrige Phase mit 1 M Salzsäure auf pH 3 gestellt. Anschließend wird mit Essigsäureethylester dreimal extrahiert und die vereinigten organischen Phasen über Natriumsulfat getrocknet. Nach Filtration und Einengen wird der erhaltene Rückstand über präparative HPLC gereinigt. Es werden 78 mg (0.13 mmol, 26% d. Th.) eines weißen Schaums erhalten.
MS (ESIpos): m/z = 587 (M+H)⁺.

70 mg (0.12 mmol) 4-{(4*E*)-5-{2-[(5-Phenylpentyl)oxy]phenyl}-3-[4-(1*H*-tetrazol-5-yl)benzyl]-pent-4-en-1-yl}benzoesäure werden mittels präparativer HPLC an chiraler Phase weiter aufgetrennt. Es werden jeweils enantiomerenrein 26 mg bzw. 17 mg der beiden E-Isomere als farblose Feststoffe erhalten (siehe Beispiele 14 und 15).

### Beispiel 14

### 4-{(4E)-5-{2-[(5-Phenylpentyl)oxy]phenyl}-3-[4-(1H-tetrazol-5-yl)benzyl]pent-4-en-1-yl}benzoesäure (Enantiomer 1)

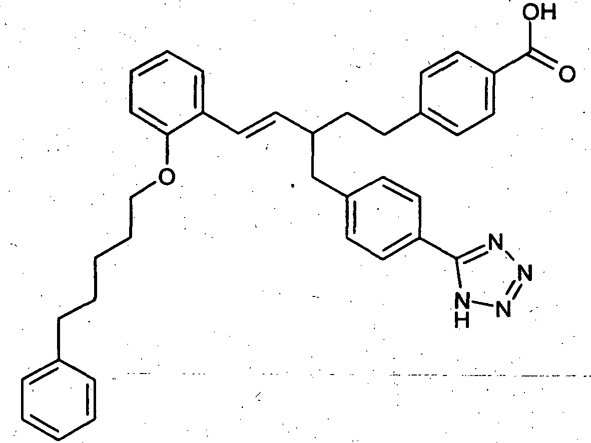

### Methode Enantiomerentrennung:

Säule: Daicel Chiralpak AD-H 250 x 20 mm; Elutionsmittel: Isohexan (mit 1% Wasser und 0.2% Trifluoressigsäure) / Isopropanol 70:30 (v/v); Fluss: 15 ml/min; UV-Detektion: 215 nm; Temperatur: 25°C.
R, 6.27 min; Reinheit 98.5%; >99% ee
Ausbeute: 26 mg
¹H-NMR (300 MHz, DMSO-d₆, δ/ppm): 12.77 (1H, breit), 7.91 (2H, d), 7.85 (2H, d), 7.42-7.33 (4H, m), 7.3 (2H, d), 7.26-7.17 (2H, m), 7.17-7.1 (4H, m), 6.95-6.83 (2H, m), 6.44 (1H, d), 6.17-6.05 (1H, m), 3.96-3.82 (2H, m), 2.93-2.82 (1H, m), 2.81-2.69 (2H, m), 2.68-2.56 (2H, m), 1.90-1.76 (1H, m), 1.76-1.62 (3H, m), 1.62-1.48 (2H, m), 1.46-1.28 (2H, m), 1.27-1.19 (1H, s).
LC-MS (Methode 1): Rₜ = 3.20 min.
MS (ESIpos): m/z = 587 (M+H)⁺.

### Beispiel 15

### 4-{(4E)-5-{2-[(5-Phenylpentyl)oxy]phenyl}-3-[4-(1H-tetrazol-5-yl)benzyl]pent-4-en-1-yl}benzoesäure (Enantiomer 2)

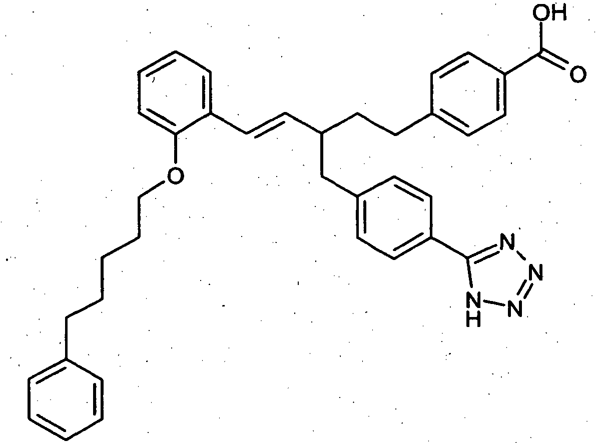

Methode Enantiomerentrennung: siehe Beispiel 14.
Rₜ 6.60 min; Reinheit 99%; >98% ee
Ausbeute: 17 mg
¹H-NMR (300 MHz, DMSO-d₆, δ/ppm): 12.77 (1H, breit), 7.91 (2H, d), 7.85 (2H, d), 7.42-7.33 (4H, m), 7.3 (2H, d), 7.26-7.17 (2H, m), 7.17-7.1 (4H, m), 6.95-6.83 (2H, m), 6.44 (1H, d), 6.17-6.06 (1H, m), 3.96-3.82 (2H, m), 2.93-2.82 (1H, m), 2.81-2.69 (2H, m), 2.68-2.56 (2H, m), 1.90-1.76 (1H, m), 1.76-1.62 (3H, m), 1.62-1.48 (2H, m), 1.46-1.28 (2H, m), 1.27-1.19 (1H, s).
LC-MS (Methode 1): Rₜ = 3.20 min.
MS (ESIpos): m/z = 587 (M+H)⁺.

### Beispiel 16

### 4-{(4E/Z)-5-{5-Fluor-2-[2-(4-methoxyphenyl)ethyl]phenyl}-3-[4-(1H-tetrazol-5-yl)benzyl]pent-4-en-1-yl}benzoesäure

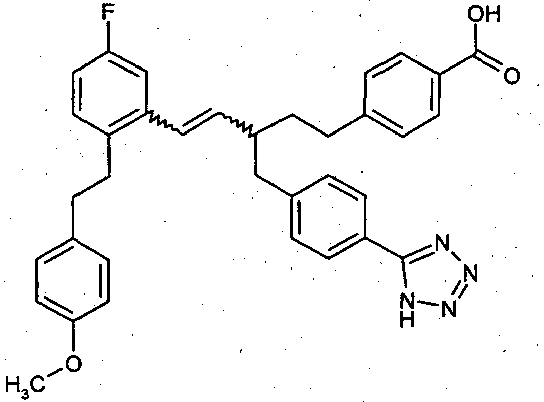

Eine Lösung von 92 mg (0.16 mmol) 4-{(4*E*/*Z*)-5-{5-Fluor-2-[2-(methoxyphenyl)ethyl]phenyl}-3-[4-(1*H*-tetrazol-5-yl)benzyl]pent-4-en-1-yl}benzoesäuremethylester in 5 ml THF und 5 ml Wasser wird mit 7.5 mg (0.31 mmol) Lithiumhydroxid versetzt und 12 Stunden bei 50°C gerührt. Nach dem Abkühlen wird das THF abgezogen und die wässrige Phase mit 1 M Salzsäure auf pH 3 gestellt. Anschließend wird mit Essigsäureethylester dreimal extrahiert und die vereinigten organischen Phasen über Natriumsulfat getrocknet. Nach Filtrieren und Einengen werden 79 mg (0.14 mmol, 88% d. Th.) eines weißen Schaums erhalten.
MS (ESIpos): m/z = 577 (M+H)⁺.

70 mg (0.12 mmol) 4-{(4*E*/*Z*)-5-{5-Fluor-2-[2-(4-methoxyphenyl)ethyl]phenyl}-3-[4-(1*H*-tetrazol-5-yl)benzyl]pent-4-en-1-yl}benzoesäure werden mittels präparativer HPLC an chiraler Phase weiter aufgetrennt. Es werden 23 mg bzw. 44 mg des Z- und *E*-Isomeren als farblose Feststoffe erhalten (siehe Beispiele 17 und 18).

### Beispiel 17

### 4-{(4Z)-5-{5-Fluor-2-[2-(4-methoxyphenyl)ethyl]phenyl}-3-(4-(1H-tetrazol-5-yl)benzyl]pent-4-en-1-yl} benzoesäure

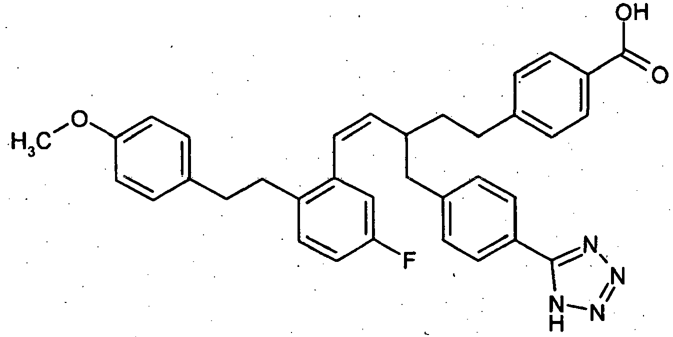

### Methode E/Z-Isomerentrennung:

Säule: Daicel Chiralpak AD-H 250 x 20 mm; Elutionsmittel: Isohexan (mit 1% Wasser und 0.2% Trifluoressigsäure) / Ethanol 60:40 (v/v); Fluss: 15 ml/min; UV-Detektion: 215 nm; Temperatur: 25°C.
Rₜ 5.276 min; Reinheit 98.5%
Ausbeute nach Trennung: 23 mg
¹H-NMR (400 MHz, DMSO-d₆, δ/ppm): 16.9-16.5 (1H, breit), 13-12.5 (1H, breit), 7.87 (2H, d), 7.77 (2H, d), 7.26 (2H, d), 7.16 (2H, d), 7.14-7.07 (1H, m), 6.97-6.89 (3H, m), 6.73 (2H, d), 6.47 (1H, d), 6.22 (1H, d), 5.74-5.64 (1H, m), 3.68 (3H, s), 2.94-2.85 (1H, m), 2.76-2.61 (4H, m), 1.85-1.61 (2H, m).

### Beispiel 18

### 4-{(4E)-5-{5-Fluor-2-[2-(4-methoxyphenyl)ethyl]phenyl}-3-[4-(1H-tetrazol-5-yl)benzyl]pent-4-en-1-yl}benzoesäure

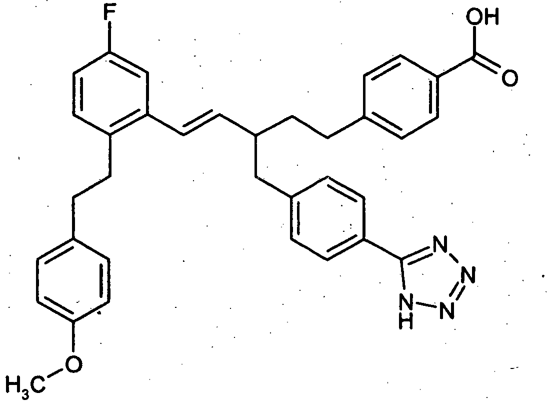

Methode *E*/*Z*-Isomerentrennung: siehe Beispiel 17.
Rₜ 5.286 min; Reinheit 99%
Ausbeute: 44 mg
¹H-NMR (400 MHz, DMSO-d₆, δ/ppm): 16.65 (1H, breit), 12.75 (1H, breit), 7.88 (2H, d), 7.84 (2H, d), 7.39 (2H, d), 7.31 (2H, d), 7.25-7.19 (1H, m), 7.12-7.06 (1H, m), 6.97-6.9 (1H, m), 6.88 (2H, d), 6.69 (2H, d), 6.37 (1H, d), 6.15-6.06 (1H, m), 3.67 (3H, s), 2.97-2.9 (1H, m), 2.8-2.6 (4H, m), 1.9-1.71 (2H, m).

### Beispiel 19

### 4-[(4E)-3-[4-(1H-Tetrazol-5-yl)benzyl]-5-(2-{[4'-(trifluormethyl)biphenyl-4-yl]methoxy}phenyl)-pent-4-en-1-yl]benzoesäure

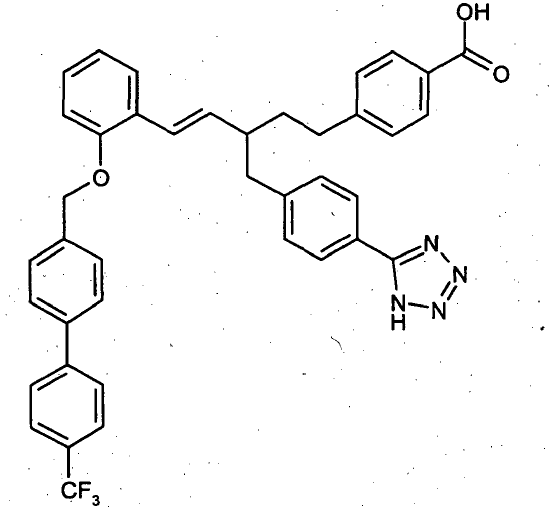

Eine Lösung von 384 mg (0.42 mmol) 4-[(4*E*)-3-[4-(1*H*-Tetrazol-5-yl)benzyl]-5-(2-{[4'-(trifluor-methyl)biphenyl-4-yl]methoxy}phenyl)pent-4-en-1-yl]benzoesäuremethylester in 10 ml THF und 10 ml Wasser wird mit 20.3 mg (0.85 mmol) Lithiumhydroxid versetzt und 12 Stunden bei 50°C gerührt. Nach dem Abkühlen wird das THF abgezogen und die wässrige Phase mit 1 M Salzsäure auf pH 3 gestellt. Anschließen wird mit Essigsäureethylester dreimal extrahiert und die vereinigten organischen Phasen über Natriumsulfat getrocknet. Nach Filtrieren und Einengen wird der erhaltene Rückstand über präparative HPLC gereinigt. Es werden 141 mg (0.21 mmol, 46% d. Th.) eines weißen Schaums erhalten.
MS (ESIpos): m/z = 675 (M+H)⁺.

120 mg (0.12 mmol) 4-[(4*E*)-3-[4-(1H-Tetrazol-5-yl)benzyl)-5-(2-{[4'-(trifluormethyl)biphenyl-4-yl]methoxy}phenyl)pent-4-en-1-yl]benzoesäure werden mittels präparativer HPLC an chiraler Phase weiter aufgetrennt. Es werden jeweils enantiomerenrein 50 mg bzw. 35 mg der beiden *E*-Iso-mere als farblose Feststoffe erhalten (siehe Beispiele 20 und 21).

### Beispiel 20

### 4-[(4E)-3-[4-(1H-Tetrazol-5-yl)benzyl]-5-(2-{[4'-(trifluormethyl)biphenyl-4-yl]methoxy}phenyl)-pent-4-en-1-yl]benzoesäure (Enantiomer 1)

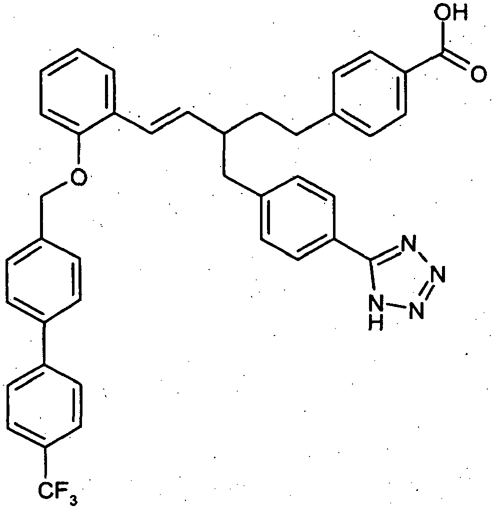

### Methode Enantiomerentrennung:

Säule: Daicel Chiralpak AD-H 250 x 20 mm; Elutionsmittel: Isohexan (mit 1% Wasser und 0.2% Trifluoressigsäure) / Isopropanol 60:40 (v/v); Fluss: 15 ml/min; UV-Detektion: 215 nm; Temperatur: 25°C.
R, 6.65 min; Reinheit 89%; >97.5% ee
Ausbeute: 50 mg
¹H-NMR (400 MHz, DMSO-d₆, δ/ppm): 16.7 (1H, breit), 12.8 (1H, s), 7.91 (2H, d), 7.86-7.77 (6H, m), 7.66 (2H, d), 7.52-7.43 (3H, m), 7.39 (2H, d), 7.27 (2H, d), 7.23-7.16 (1H, m), 7.06 (1H, d), 6.95-6.89 (1H, m), 6.54 (1H, d), 6.21-6.12 (1H, m), 5.2-5.1 (2H, m), 2.92-2.85 (1H, m), 2.8-2.7 (2H, m), 2.69 (1H, m), 2.58 (1H, m), 1.88-1.77 (1H, m), 1.77-1.64 (1H, m).

### Beispiel 21

### 4-[(4E)-3-[4-(1H-Tetrazol-5-yl)benzyl]-5-(2-{[4'-(trifluormethyl)biphenyl-4-yl]methoxy}phenyl)-pent-4-en-1-yl]benzoesäure (Enantiomer 2)

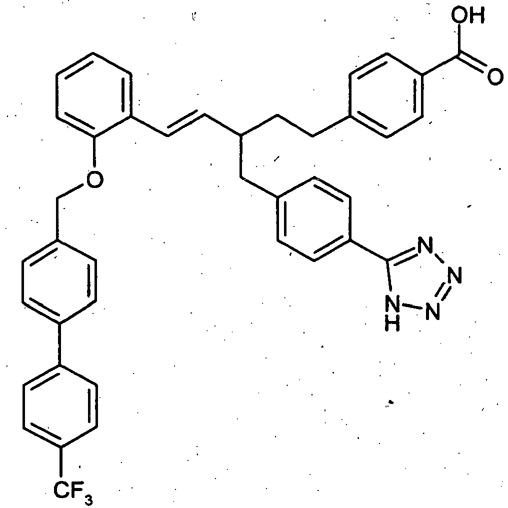

Methode Enantiomerentrennung: siehe Beispiel 20.
Rₜ 7.37 min; Reinheit 99%; >98% ee
Ausbeute: 35 mg
¹H-NMR (400 MHz, DMSO-d₆, δ/ppm): 16.7 (1H, breit), 12.8 (1H, s), 7.91 (2H, d), 7.86-7.77 (6H, m), 7.66 (2H, d), 7.52-7.43 (3H, m), 7.39 (2H, d), 7.27 (2H, d), 7.23-7.16 (1H, m), 7.06 (1H, d), 6.95-6.89 (1H, m), 6.54 (1H, d), 6.21-6.12 (1H, m), 5.2-5.1 (2H, m), 2.92-2.85 (1H, m), 2.8-2.7 (2H, m), 2.69 (1H, m), 2.69-2.6 (1H, m), 1.88-1.77 (1H, m), 1.77-1.64 (1H, m).

### Beispiel 22

### 4-{(4E)-5-{2-[(4-tert.-Butylbenzyl)oxy]phenyl}-3-[4-(1H-tetrazol-5-yl)benzyl]pent-4-en-1-yl}-benzoesäure

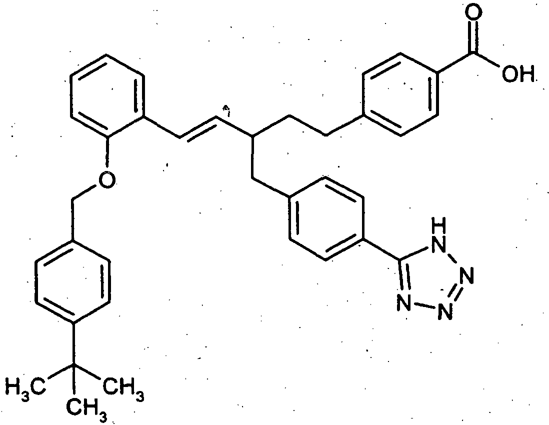

Eine Lösung von 70 mg (0.12 mmol) 4-{(4*E*)-5-{2-[(4-*tert*.-Butylbenzyl)oxy]phenyl}-3-[4-(1*H-*tetrazol-5-yl)benzyl]pent-4-en-1-yl}benzoesäuremethylester in 0.5 ml THF und 0.25 ml Wasser wird mit 11.16 mg (0.47 mmol) Lithiumhydroxid versetzt und 12 Stunden bei 60°C gerührt. Nach dem Abkühlen wird das THF abgezogen und die wässrige Phase mit 1 M Salzsäure auf pH 2 gestellt Anschließend wird mit Essigsäureethylester dreimal extrahiert und die vereinigten organischen Phasen über Natriumsulfat getrocknet. Nach Filtration und Einengen wird der erhaltene Rückstand über präparative HPLC gereinigt. Es werden 45 mg (0.076 mmol, 66% d. Th.) eines weißen Schaums erhalten.
¹H-NMR (400 MHz, DMSO-d₆, δ/ppm): 16.72 (1H, breit), 12.77 (1H, breit), 7.89 (2H, d), 7.82 (2H, d), 7.42 (1H, d), 7.37-7.23 (8H, m), 7.17 (1H, t), 7.04 (1H, d), 6.9 (1H, t), 6.52 (1H, d), 6.14 (1H, dd), 5.09-4.99 (2H, m), 2.90-2.81 (1H, m), 2.79-2.69 (2H, m), 2.69-2.57 (2H, m), 1.87-1.76-(2H, m), 1.74-1.61 (1H, m), 1.23 (9H, s).

45 mg (0.076 mmol) 4-{(4*E*)-5-{2-[(4-*tert*.-Butylbenzyl)oxy]phenyl}-3-[4-(1*H*-tetrazol-5-yl)-benzyl]pent-4-en-1-yl}benzoesäure werden mittels präparativer HPLC an chiraler Phase weiter aufgetrennt. Es werden jeweils enantiomerenrein 16 mg bzw. 18 mg der beiden *E*-Isomere als farblose Feststoffe erhalten (siehe Beispiele 23 und 24).

### Beispiel 23

### 4-{(4E)-5-{2-[(4-tert.-Butylbenzyl)oxy]phenyl}-3-[4-(1H-tetrazol-5-yl)benzyl]pent-4-en-1-yl}-benzoesäure (Enantiomer 1)

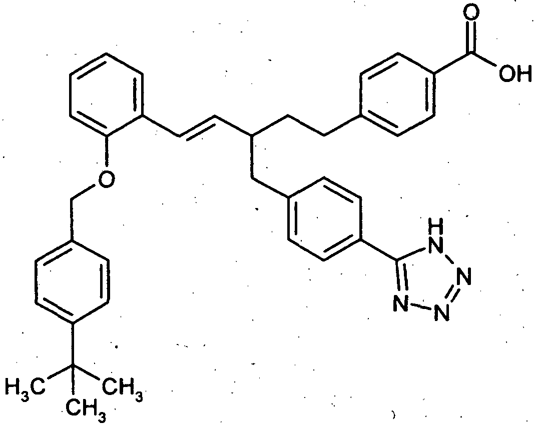

### Methode Enantiomerentrennung:

Säule: Daicel Chiralpak AD-H 250 x 20 mm; Elutionsmittel: Isohexan (mit 1% Wasser und 1% Essigsäure) / Isopropanol 50:50 (v/v); Fluss: 15 ml/min; UV-Detektion: 220 nm; Temperatur: 25°C.
Rₜ 5.92 min; Reinheit 99%; >99% ee
Ausbeute: 16 mg.

### Beispiel 24

### 4-{(4E)-5-{2-[(4-tert.-Butylbenzyl)oxy]phenyl}-3-[4-(1H-tetrazol-5-yl)benzyl]pent-4-en-1-yl}-benzoesäure (Enantiomer 2)

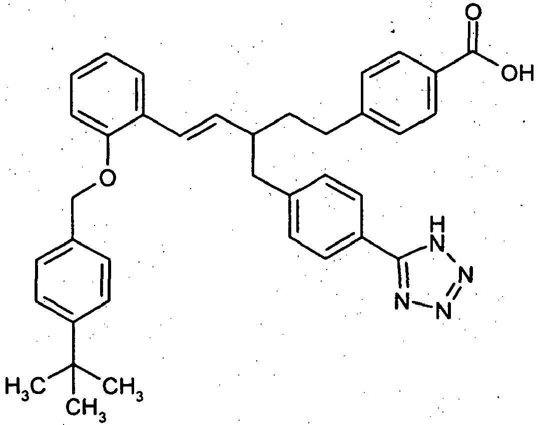

Methode Enantiomerentrennung: siehe Beispiel 23.
Rₜ 6.58 min; Reinheit 99%; >96% ee
Ausbeute: 18 mg.

### Beispiel 25

### 4-[3-((E)-2-{2-[(4-tert.-Butylbenzyl)oxy]phenyl}vinyl)-7-(1H-tetrazol-5-yl)heptyl]benzoesäure

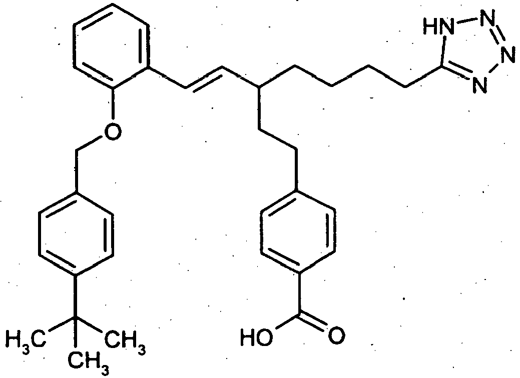

Eine Lösung von 181 mg (0.32 mmol) 4-[3-((*E*)-2-{2-[(4-*tert*.-Butylbenzyl)oxy]phenyl}vinyl)-7-(1*H*-tetrazol-5-yl)heptyl]benzoesäuremethylester in 5 ml THF und 5 ml Wasser wird mit 61.18 mg (2.55 mmol) Lithiumhydroxid versetzt und 12 Stunden bei 60°C gerührt. Nach dem Abkühlen wird das THF abgezogen und die wässrige Phase mit 1 M Salzsäure auf pH 2 gestellt. Anschließend wird mit Essigsäureethylester dreimal extrahiert und die vereinigten organischen Phasen über Natriumsulfat getrocknet. Nach Filtration und Einengen wird der erhaltene Rückstand über präparative HPLC gereinigt. Es werden 65 mg (0.12 mmol, 36.8% d. Th.) eines weißen Schaums erhalten.
¹H-NMR (400 MHz, DMSO-d₆, δ/ppm): 15.89 (1H, breit), 12.77 (1H, breit), 7.83 (2H, d), 7.46 (1H, d), 7.42-7.32 (4H, m), 7.28 (2H, d), 7.24-7.16 (1H, m), 7.16-7.0 (1H, m), 6.92 (1H, t), 6.64 (1H, d), 6.05 (1H, dd), 5.11 (2H, s), 2.84 (2H, t), 2.73-2.64 (1H, m), 2.63-2.56 (1H, m), 2.17-2.05 (1H, m), 1.79-1.54 (4H, m), 1.53-1.42 (1H, m), 1.42-1.14 (12H, m).
LC-MS (Methode 2): Rₜ = 2.83 min.
MS (ESIpos): m/z = 553 (M+H)⁺.

73 mg (0.13 mmol) 4-[3-((*E*)-2-{2-[(4-*tert.*-Butylbenzyl)oxy]phenyl}vinyl)-7-(1*H*-tetrazol-5-yl)-heptyl]benzoesäure werden mittels präparativer HPLC an chiraler Phase weiter aufgetrennt. Es werden enantiomerenrein jeweils 31 mg der beiden E-Isomere als farblose Feststoffe erhalten (siehe Beispiele 26 und 27).

### Beispiel 26

### 4-[3-((E)-2-{2-[(4-tert.-Butylbenzyl)oxy]phenyl}vinyl)-7-(1H-tetrazol-5-yl)heptyl]benzoesäure (Enantiomer 1)

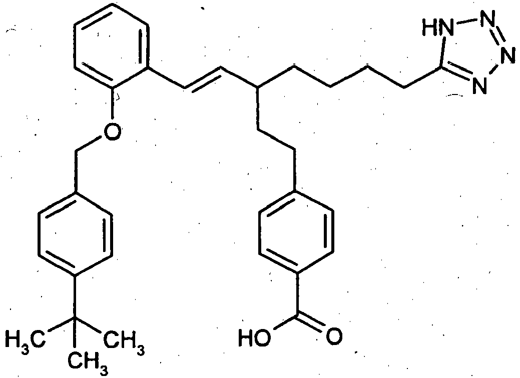

### Methode Enantiomerentrennung:

Säule: Daicel Chiralpak AD-H 250 x 20 mm; Elutionsmittel: Isohexan (mit 1% Wasser und 02% Essigsäure) / Isopropanol 50:50 (v/v); Fluss: 15 ml/min; UV-Detektion: 220 nm; Temperatur: 35°C.
Rₜ 4.19 min; Reinheit 99%; >99.5% ee
Ausbeute: 31 mg
LC-MS (Methode 2): Rₜ = 2.85 min.
MS (ESIpos): m/z = 553 (M+H)⁺.

### Beispiel 27

### 4-[3-((E)-2-{2-[(4-tert.-Butylbenzyl)oxy]phenyl}vinyl)-7-(1H-tetrazol-5-yl)heptyl]benzoesäure (Enantiomer 2)

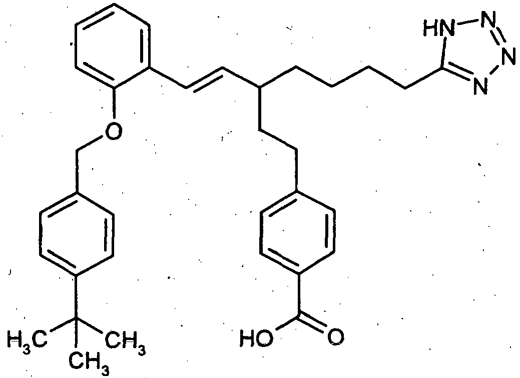

Methode Enantiomerentrennung: siehe Beispiel 26.
Rₜ 4.92 min; Reinheit 99%; >98.8% ee
Ausbeute: 31 mg.

### Beispiel 28

### (7E)-8-{2-[(4-tert.-Butylbenzyl)oxy]phenyl}-6-{2-[4-(1H-tetrazol-5-yl)phenyl]ethyl}oct-7-ensäure

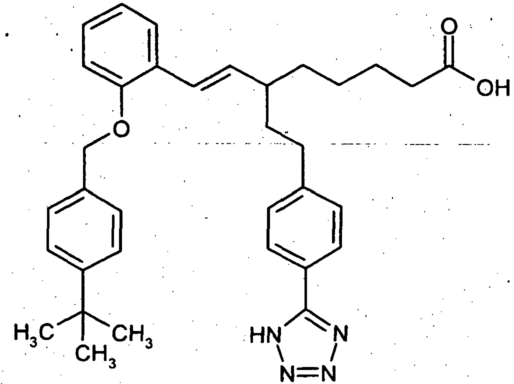

Eine Lösung von 600 mg (0.61 mmol) (7*E*)-8-{2-[(4-*tert*.-Butylbenzyl)oxy]phenyl}-6-{2-[4-(1*H-*tetrazol-5-yl)phenyl]ethyl}oct-7-ensäureethylester in 20 ml THF und 20 ml Wasser wird mit 29.2 mg (1.22 mmol) Lithiumhydroxid versetzt und 12 Stunden bei 50°C gerührt. Nach dem Abkühlen wird das THF abgezogen und die wässrige Phase mit 1 M Salzsäure auf pH 3 gestellt. Anschließend wird mit Essigsäureethylester dreimal extrahiert und die vereinigten organischen Phasen über Natriumsulfat getrocknet. Nach Filtration und Einengen wird der erhaltene Rückstand über präparative HPLC gereinigt. Es werden 205 mg (0.37 mmol, 59.6% d. Th.) eines weißen Schaums erhalten.
MS (ESIpos): m/z = 553 (M+H)⁺.

200 mg (0.36 mmol) (7*E*)-8-{2-[(4-*tert*.-Butylbenzyl)oxy]phenyl}-6-{2-[4-(1*H-*tetrazol-5-yl)-phenyl]ethyl}oct-7-ensäure werden mittels präparativer HPLC an chiraler Phase weiter aufgetrennt. Es werden enantiomerenrein jeweils 69 mg der beiden *E*-Isomere als farblose Feststoffe erhalten (siehe Beispiele 29 und 30).

### Beispiel 29

### (7E)-8-{2-[(4-tert.-Butylbenzyl)oxy]phenyl}-6-{2-[4-(1H-tetrazol-5-yl)phenyl]ethyl}oct-7-ensäure (Enantiomer 1)

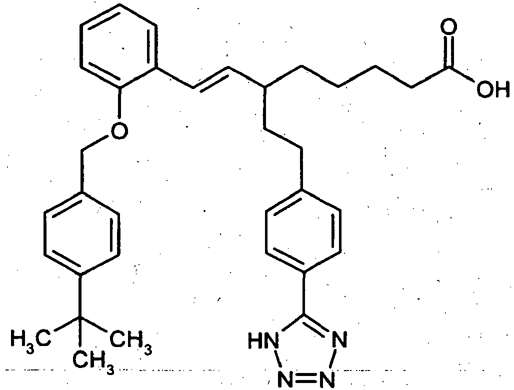

### Methode Enantiomerentrennung:

Säule: Daicel Chiralpak AD-H 250 x 20 mm; Elutionsmittel: Isohexan (mit 1% Wasser und 0.2% Essigsäure) / Isopropanol 80:20 (v/v); Fluss: 15 ml/min; UV-Detektion: 220 nm; Temperatur: 35°C.
R, 7.03 min; Reinheit 99.5%; >97.5% ee
Ausbeute: 69 mg
¹H-NMR (300 MHz, DMSO-d₆, δ/ppm): 11.96 (1H, breit), 7.92 (2H, d), 7.47 (1H, d), 7.44-7.33 (6H, m), 7.2 (1H, t), 7.09 (1H, d), 6.91 (1H, t), 6.64 (1H, d), 6.13-6.01 (1H, m), 5.11 (2H, s), 2.78-2.54 (2H, m), 2.22-2.05 (2H, m), 1.84-1.69 (1H, m), 1.69-1.56 (1H, m), 1.54-1.38 (4H, m), 1.38-1.16 (3H, m).
LC-MS (Methode 2): Rₜ = 2.92 min.
MS (ESIpos): m/z = 553 (M+H)⁺.

### Beispiel 30

### (7E)-8-{2-[(4-tert.-Butylbenzyl)oxy]phenyl}-6-{2-[4-(1H-tetrazol-5-yl)phenyl]ethyl}oct-7-ensäure (Enantiomer 2)

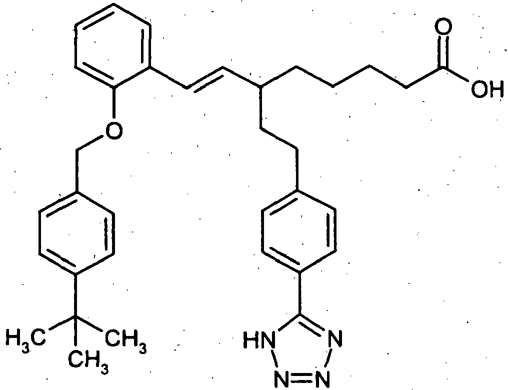

Methode Enantiomerentrennung: siehe Beispiel 29.
Rₜ 7.89 min; Reinheit 99.5%; >98.5% ee
Ausbeute: 69 mg
¹H-NMR (300 MHz, DMSO-d₆, δ/ppm): 11.95 (1H, breit), 7.91 (2H, d), 7.48 (1H, d), 7.44-7.33 (6H, m), 7.2 (1H, t), 7.09 (1H, d), 6.91 (1H, t), 6.64 (1H, d), 6.13-6.01 (1H, m), 5.11 (2H, s), 2.78-2.54 (2H, m), 2.22-2.05 (2H, m), 1.84-1.69 (1H, m), 1.69-1.56 (1H, m), 1.54-1.38 (4H, m), 1.38-1.16 (3H, m).
LC-MS (Methode 2): Rₜ = 2.93 min.
MS (ESIpos): m/z = 553 (M+H)⁺.

### Beispiel 31

### (7E)-8-{2-[(5-Phenylpentyl)oxy]phenyl}-6-{2-[4-(1H-tetrazol-5-yl)phenyl]ethyl}oct-7-ensäure

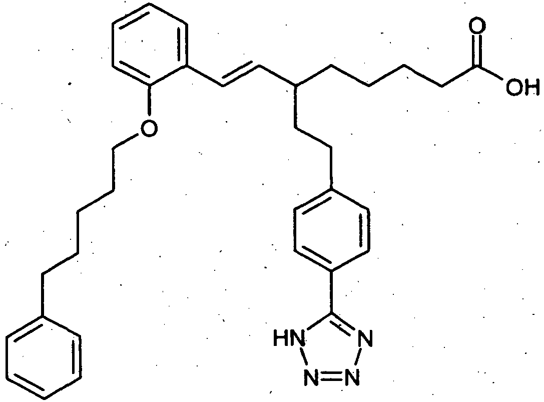

Eine Lösung von 62 mg (0.11 mmol) (7*E*)-8-{2-[(5-Phenylpentyl)oxy]phenyl}-6-{2-[4-(1*H*-tetra-zol-5-yl)phenyl]ethyl}oct-7-ensäureethylester in 5 ml THF und 5 ml Wasser wird mit 5.11 mg (0.21 mmol) Lithiumhydroxid versetzt und 12 Stunden bei 50°C gerührt. Nach dem Abkühlen wird das THF abgezogen und die wässrige Phase mit 1 M Salzsäure auf pH 3 gestellt. Anschließend wird mit Essigsäureethylester dreimal extrahiert und die vereinigten organischen Phasen über Natriumsulfat getrocknet. Nach Filtration und Einengen werden 34 mg (0.06 mmol, 57.6% d. Th.) eines weißen Schaums erhalten.
¹H-NMR (400 MHz, DMSO-d₆, δ/ppm): 11.95 (1H, breit), 7.93 (2H, d), 7.43 (1H, d), 7.4 (2H, d), 7.3-7.1 (6H, m), 6.97 (1H, d), 6.38 (1H, t), 6.58 (1H, d), 6.05 (1H, dd), 4.0 (2H, t), 2.75-2.65 (1H, m), 2.22-2.05 (3H, m), 1.83-1.7 (3H, m), 1.7-1.57 (3H, m), 1.57-1.4 (6H, m), 1.4-1.15 (5H, m).

30 mg (0.054 mmol) (7*E*)-8-{2-[(5-Phenylpentyl)oxy]phenyl}-6-{2-[4-(1*H*-tetrazol-5-yl)phenyl]-ethyl}oct-7-ensäure werden mittels präparativer HPLC an chiraler Phase weiter aufgetrennt. Es werden enantiomerenrein jeweils 10 mg der beiden *E*-Isomere als farblose Feststoffe erhalten (siehe Beispiele 32 und 33).

### Beispiel 32

### (7E)-8-{2-[(5-Phenylpentyl)oxy]phenyl}-6-{2-[4-(1H-tetrazol-5-yl)phenyl]ethyl}oct-7-ensäure (Enantiomer 1)

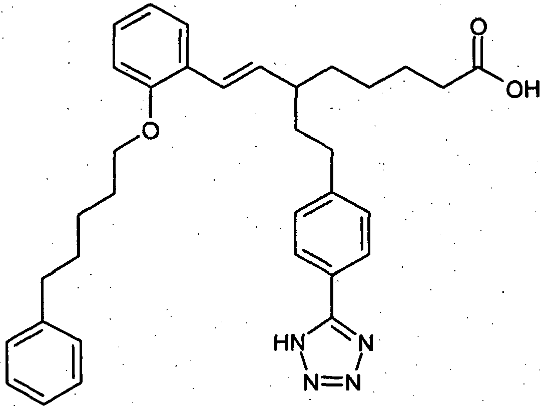

### Methode Enantiomerentrennung:

Säule: Daicel Chiralpak AD-H 250 x 20 mm; Elutionsmittel: Isohexan (mit 1% Wasser und 0.2% Essigsäure) / Isopropanol 80:20 (v/v); Fluss: 15 ml/min; UV-Detektion: 215 nm; Temperatur: 25°C.
Rₜ 6.07 min; Reinheit 99%; >99% ee
Ausbeute: 10 mg
¹H-NMR (300 MHz DMSOd₆, δ/ppm): 11.95 (1H, breit), 8.96 (2H, d), 7.45 (3H, d), 7.27-7.07 (6H, m), 6.97 (1H, d), 6.39 (1H, t), 6.57 (1H, d), 6.07 (1H, dd), 4.0 (2H, t), 2.78-2.5 (3H, m), 2.22-2.01 (3H, m), 1.85-1.16 (16H, m).

### Beispiel 33

### (7E)-8-{2-[(5-Phenylpentyl)oxy]phenyl}-6-{2-[4-(1H tetrazol-5-yl)phenyl]ethyl}oct-7-ensäure (Enantiomer 2)

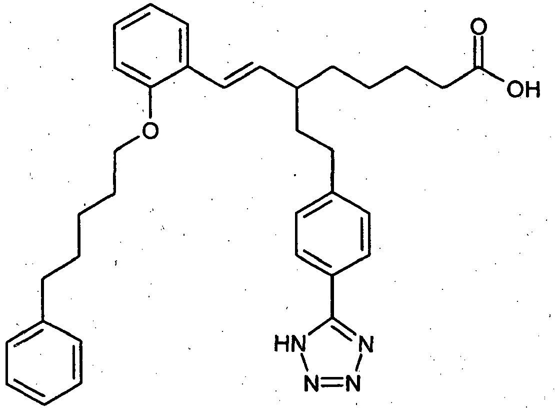

Methode Enantiomerentrennung: siehe Beispiel 32.
Rₜ 7.05 min; Reinheit 99%; >98.5% ee
Ausbeute: 10 mg
¹H-NMR (300 MHz, DMSO-d₆, δ/ppm): 11.95 (1H; breit), 8.96 (2H, d), 7.45 (3H, d), 7.27-7.07 (6H, m), 6.97 (1H, d), 6.39 (1H, t), 6.5 7 (1H, d), 6.07 (1H, dd), 4.0 (2H, t), 2.78-2.5 (3H, m), 2.22-2.01 (3H, m), 1.85-1.16 (16H, m).

### Beispiel 34

### (7E)-8-{2-[(4-tert.-Butylbenzyl)oxy]phenyl}-6-[4-(1H-tetrazol-5-yl)benzyl]oct-7-ensäure

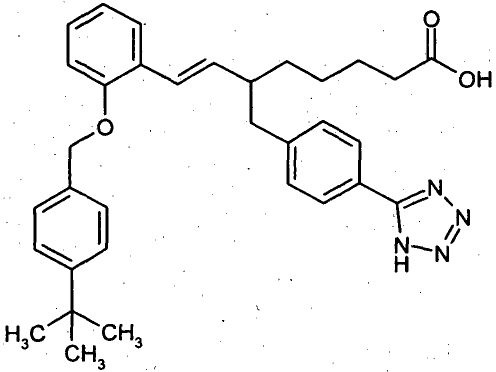

Eine Lösung von 800 mg (1.41 mmol) (7*E*)-8-{2-[(4-*tert*.-Butylbenzyl)oxy]phenyl}-6-[4-(1*H* tetrazol-5-yl)benzyl]oct-7-ensäureethylester in 10 ml THF und 10 mol Wasser wird mit 67.6 mg (2.82 mmol) Lithiumhydroxid versetzt und 12 Stunden bei 50°C gerührt. Nach dem Abkühlen wird das THF abgezogen und die wässrige Phase mit 1 M Salzsäure auf pH 3 gestellt. Anschließend wird mit Essigsäureethylester dreimal extrahiert und die vereinigten organischen Phasen über Natriumsulfat getrocknet. Nach Filtration und Einengen wird der erhaltene Rückstand über präparative HPLC gereinigt. Es werden 660 mg (1.2 mmol, 87% d. Th.) eines weißen Schaums erhalten.
LC-MS (Methode 4): Rₜ = 2.51 min.
MS (ESIpos): m/z = 539 (M+H)⁺.

700 mg (1.3 mmol) (7*E*)-8-{2-[(4-*tert*.-Butylbenzyl)oxy]phenyl}-6-[4-(1*H*-tetrazol-5-yl)benzyl]oct-7-ensäure werden mittels präparativer HPLC an chiraler Phase weiter aufgetrennt. Es werden jeweils enantiomerenrein 318 mg bzw. 257 mg der beiden E-Isomere als farblose Feststoffe erhalten (siehe Beispiele 35 und 36).

### Beispiel 35

### (7E)-8-{2-[(4-tert.-Butylbenzyl)oxy]phenyl}-6-[4-(1H-tetrazol-5-yl)benzyl]oct-7-ensäure (Enantiomer 1)

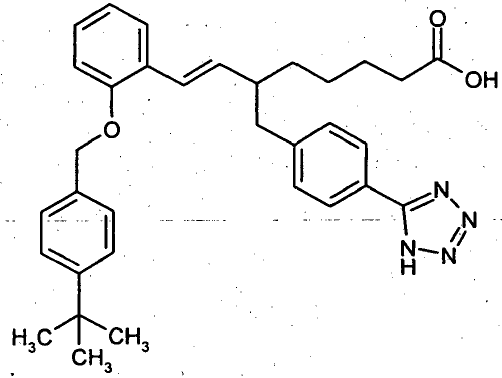

### Methode Enantiomerentrennung:

Säule: Daicel Chiralpak AD-H 250 x 20 mm; Elutionsmittel: Isohexan (mit 1% Wasser und 0.2% Essigsäure) / Isopropanol 65:35 (v/v); Fluss: 15 ml/min; UV-Detektion: 220 nm; Temperatur: 35°C.
R, 4.18 min; Reinheit 99%; >99.5% ee
Ausbeute: 318 mg
¹H-NMR (400 MHz DMSO-d₆, δ/ppm): 12.85 (1H, breit), 11.98 (1H, breit), 7.7 (2H, d), 7.38 (5H, d), 7.25 (2H, d), 7.15 (1H, t), 7.0 (1H, d), 6.88 (1H, t), 6.43 (1H, d), 6.07 (1H, dd), 5.03 (2H, s), 2.88 (1H, m), 2.69 (1H, t), 1.54-1.3 (6H, m), 1.28 (10H, m).

### Beispiel 36

### (7E)-8-{2-[(4-tert.-Butylbenzyl)oxy]phenyl}-6-[4-(1H-tetrazol-5-yl)benzyl]oct-7-ensäure (Enantiomer 2)

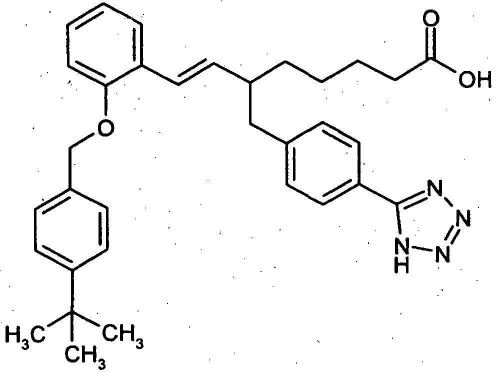

Methode Enantiomerentrennung: siehe Beispiel 35.
Rₜ 5.00 min; Reinheit 99%; >99.4% ee
Ausbeute: 257 mg
¹H-NMR (400 MHz, DMSO-d₆, δ/ppm): 11.98 (1H, breit), 7.9 (2H, d), 7.4 (3H, d), 7.32 (2H, d), 7.26 (2H, d), 7.16 (1H, t), 7.0 (1H, d), 6.89 (1H, t), 6.46 (1H, d), 6.08 (1H, dd), 2.88-2.8 (1H, m), 2.72-2.63 (1H, m), 2.18 (2H, t), 1.56-1.3 (6H, m), 1.3-1.22 (10H, m). ,

### Beispiel 37

### (7E/Z)-8-{2-[(5-Phenylpentyl)oxy]phenyl}-6-[4-(1H-tetrazol-5-yl)benzyl]oct-7-ensäure

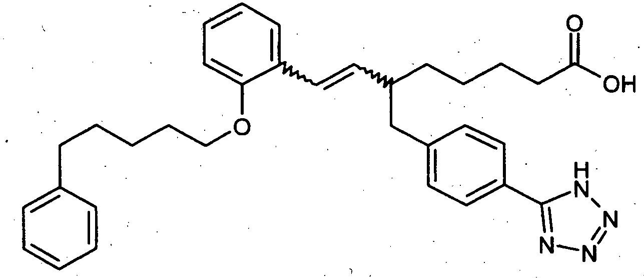

Eine Lösung von 36 mg (0.06 mmol) (7*E*/*Z*)-8-{2-[(5-Phenylpentyl)oxy]phenyl}-6-[4-(1*H*-tetrazol-5-yl)benzyl]oct-7-ensäureethylester in 5 ml Methanol wird mit 0.5 ml 45%-iger Natronlauge versetzt und 2 Stunden bei Raumtemperatur gerührt. Anschließend wird das Methanol abgezogen und der Rückstand in Wasser/Essigsäureethylester aufgenommen. Man stellt mit 1 M Salzsäure auf pH 3. Die Phasen werden getrennt und die wässrige Phase noch zweimal mit Essigsäureethylester extrahiert. Die vereinigten organischen Phasen werden mit gesättigter Natriumchlorid-Lösung gewaschen und über Natriumsulfat getrocknet. Nach Filtration und Einengen wird der erhaltene Rückstand über präparative HPLC gereinigt. Es werden 14.7 mg (0.03 mmol, 43% d. Th.) eines farblosen Schaums erhalten.
¹H-NMR (300 MHz, CDCl₃, δ/ppm): (*E*/*Z* = 2:1) 7.92 (1.06H, d), 7.87 (0.53H, d), 7.38-7.21 (4H, m), 7.21-7.09 (4H, m), 6.93-6.72 (3H, m), 6.59 (0.53H; d), 6.49 (0.26H, d), 6.07-5.95 (0.53H, m), 5.44-5.36 (0.26H, t), 3.99-3.89 (1.06H, t), 3.89-3.79 (0.53H, m), 2.86-2.73 (2H, m), 2.65-2.57 (2H, t), 2.54-2.43 (1H, m), 2.34-2.26 (1.06H, t), 2.26-2.18 (0.53H, m), 1.85-1.73 (2H, m), 1.73-1.55 (2H, m), 1.55-1.23 (6H, m).

Auf analoge Weise werden die in der folgenden Tabelle aufgeführten Beispiele erhalten:

| **Beispiel Nr.** | **Beispiel-Struktur** ***[Edukte]*** | **Analytische Daten** |
|---|---|---|
| **38** | 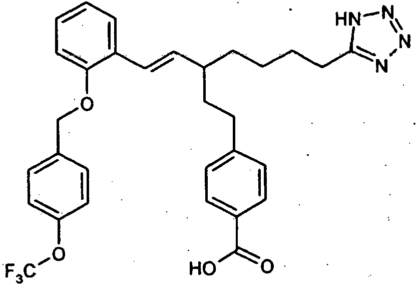 | ¹H-NMR (400 MHz, DMSO-d₆, δ/ppm): 15.89 (1H, br. s), 12.76 (1H, br. s), 7.82 (2H, d), 7.59 (2H, d), 7.47 (1H, d), 7.37 (2H, d), 7.28 (2H, d), 7.20 (1H, t), 7.08 (1H, d), 6.92 (1H, t), 6.62 (1H, d), 6.11-5.98 (1H, m), 5.18 (2H, s), 2.84 (2H, t), 2.73-2.41 (2H, m), 2.18-2.03 (1H, m), 1.80-1.53 (4H, m), 1.52-1.41 (1H, m), 1.40-1.20 (3H, m). |
| | (Racemat) | |
| | *[ausgehend von Bsp. 51A und 1-(Brommethyl)-4-(trifluormethoxy)benzol]* | |
| | | LC-MS (Methode 4): Rₜ = 2.84 min; m/z = 581 (M+H⁺). |
| **39** | 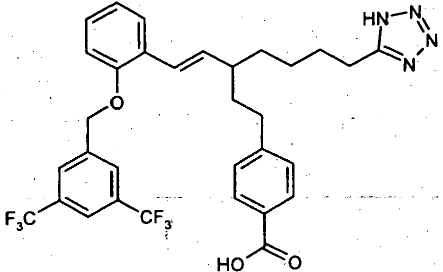 | LC-MS (Methode 6): Rₜ = 2.99 min; m/z = 633 (M+H⁺). |
| | (Racemat) | |
| | *[ausgehend von Bsp. 51A und 1-(Brommethyl)-3,5-bis-(trifluormethoxy)benzol]* | |

### B. Bewertung der pharmakologischen Wirksamkeit

Die pharmakologische Wirkung der erfindungsgemäßen Verbindungen kann in folgenden Assays gezeigt werden:

### B-1. GefäBrelaxierende Wirkung in vitro:

Kaninchen werden durch intravenöse Injektion von Thiopental-Natrium narkotisiert bzw. getötet (ca. 50 mg/kg) und entblutet. Die Arteria Saphena wird entnommen und in 3 mm breite Ringe geteilt. Die Ringe werden einzeln auf je einem triangelförmigen, am Ende offenen Häkchenpaar aus 0.3 mm starkem Spezialdraht (Remanium^{®}) montiert. Jeder Ring wird unter Vorspannung in 5 ml-Organbäder mit 37°C warmer, carbogenbegaster Krebs-Henseleit-Lösung folgender Zusammensetzung gebracht: NaCl 119 mM; KCl 4.8 mM; CaCl₂ x 2 H₂O 1 mM; MgSO₄ x 7 H₂O 1.4 mM; KH₂PO₄ 1.2 mM; NaHCO₃ 25 mM; Glucose 10 mM; Rinderserumalbumin 0.001%. Die Kontraktionskraft wird mit Statham UC2-Zellen erfasst, verstärkt und über A/D-Wandler (DAS-1802 HC, Keithley Instruments, München) digitalisiert sowie parallel auf Linienschreibern registriert. Kontraktionen werden durch Zugabe von Phenylephrin induziert.

Nach mehreren (allgemein 4) Kontrollzyklen wird die zu untersuchende Substanz in jedem weiteren Durchgang in steigender Dosierung zugesetzt und die Höhe der unter dem Einfluss der Testsubstanz erzielten Kontraktion mit der Höhe der im letzten Vordurchgang erreichten Kontraktion verglichen. Daraus wird die Konzentration errechnet, die erforderlich ist, um die in der Vorkontrolle erreichte Kontraktion auf 50% zu reduzieren (IC₅₀-Wert). Das Standard-Applikationsvolumen beträgt 5 µl. Der DMSO-Anteil in der Badlösung entspricht 0.1%.

Repräsentative Ergebnisse zu den erfindungsgemäßen Verbindungen sind in Tabelle 1 aufgeführt:

**Tabelle 1: Gefäßrelaxierende Wirkung in vitro**

| **Beispiel Nr.** | **IC₅₀ [nM]** |
|---|---|
| 3 | 682 |
| 5 | 2510 |
| 6 | 119 |
| 14 | 1324 |
| 17 | 5800 |
| 18 | 890 |
| 21 | 372 |
| 23 | 3888 |
| 24 | 102 |
| 26 | 559 |
| 27 | 9.7 |
| 29 | 1990 |
| 36 | 760 |
| 38 | 567 |
| 39 | 150 |

### B-2. Stimulation der rekombinanten löslichen Guanylatcyclase (sGC in vitro:

Die Untersuchungen zur Stimulation der rekombinanten löslichen Guanylatcyclase (sGC) durch die erfindungsgemäßen Verbindungen mit und ohne Natriumnitroprussid sowie mit und ohne den Häm-abhängigen sGC-Inhibitor 1*H*-1,2,4-Oxadiazol-(4,3a)-chinoxalin-1-on (ODQ) werden nach der in folgender Literaturstelle im Detail beschriebenen Methode durchgeführt: M. Hoenicka, E.M. Becker, H. Apeler, T. Sirichoke, H. Schroeder, R. Gerzer und J.-P. Stasch, "Purified soluble guanylyl cyclase expressed in a baculovirus/Sf9 system: Stimulation by YC-1, nitric oxide, and carbon oxide", J. Mol. Med. 77 (1999), 14-23. Die Häm-freie Guanylatcyclase wird durch Zugabe von Tween 20 zum Probenpuffer (0.5% in der Endkonzentration) erhalten.

Die Aktivierung der sGC durch eine Prüfsubstanz wird als n-fache Stimulation der Basalaktivität angegeben. Das Ergebnis für Beispiel 6 ist in Tabelle 2 gezeigt:

**Tabelle 2: Stimulation (n-fach) der rekombinanten löslichen Guanylatcyclase (sGC) in vitro durch Beispiel 6**

| **Konzentration Beispiel 6 [µM]** | **Häm-haltige sGC** | | | **Häm-freie sGC** | |
|---|---|---|---|---|---|
| | **Basal** | **+ 0.1 µM DEA/NO** | **+ 10 µM ODQ** | **Basal** | **+ 10 µM ODQ** |
| 0.0 | 1.0 | 64.1 | 2.4 | 1.0 | 1.2 |
| 0.001 | 1.9 | 62.0 | 4.8 | 3.2 | 3.0 |
| 0.01 | 5.1 | 66.0 | 25.7 | 20.4 | 18.9 |
| 0.1 | 20.2 | 80.0 | 102.7 | 90.9 | 89.7 |
| 1 | 29.5 | 96.2 | 125.8 | 143.8 | 143.8 |
| 10 | 42.1 | 99.9 | 135.0 | 145.9 | 149.3 |
| [DEA/NO = 2-(*N,N*-Diethylamino)diazenolat-2-oxid; ODQ = 1*H*-1,2,4-Oxadiazol-(4,3a)-chinoxalin-1-on]. | | | | | |

Aus Tabelle 2 ist ersichtlich, dass eine Stimulation sowohl des Häm-haltigen als auch des Hämfreien Enzyms erreicht wird. Weiterhin zeigt die Kombination aus Beispiel 6 und 2-(*N*,*N*-Diethyl-amino)diazenolat-2-oxid (DEA/NO), einem NO-Donor, keinen synergistischen Effekt, d.h. die Wirkung von DEA/NO wird nicht potenziert, wie dies bei einem über einen Häm-abhängigen Mechanismus wirkenden sGC-Aktivator zu erwarten wäre. Darüber hinaus wird die Wirkung des erfindungsgemäßen sGC-Aktivators durch den Häm-abhängigen Inhibitor der löslichen Guanylatcyclase ODQ nicht blockiert, sondern sogar gesteigert. Die Ergebnisse aus Tabelle 2 belegen somit den Wirkmechanismus der erfindungsgemäßen Verbindungen als Aktivatoren der löslichen Guanylatcyclase.

### B-3. Radiotelemetrische Messung von Blutdruck und Herzfrequenz an wachen SH-Ratten

Für die im Folgenden beschriebenen Messungen an wachen SH-Ratten wird ein im Handel erhältliches Telemetriesystem der Firma Data Sciences International DSI, USA, eingesetzt.

Das System besteht aus 3 Hauptkomponenten: (*1*) Implantierbare Sender, (*2*) Empfänger, die über einen Multiplexer mit einem (*3*) Datenakquisitionscomputer verbunden sind. Die Telemetrieanlage ermöglicht eine kontinuierliche Erfassung von Blutdruck und Herzfrequenz an wachen Tieren in ihrem gewohnten Lebensraum.

Die Untersuchungen werden an ausgewachsenen weiblichen, spontan-hypertensiven Ratten (SH-Ratten) mit einem Körpergewicht von >200 g durchgeführt. Die Versuchstiere werden nach Senderimplantation einzeln in Makrolon-Käfigen Typ 3 gehalten. Sie haben freien Zugang zu Standardfutter und Wasser. Der Tag/Nacht-Rhythmus im Versuchslabor wird per Raumbeleuchtung um 6:00 Uhr morgens und um 19:00 Uhr abends gewechselt.

Die eingesetzten Telemetriesender (TAM PA-C40, DSI) werden den Versuchstieren mindestens 14 Tage vor dem ersten Versuchseinsatz unter aseptischen Bedingungen chirurgisch implantiert. Die so instrumentierten Tiere sind nach Abheilen der Wunde und Einwachsen des Implantats wiederholt einsetzbar.

Zur Implantation werden die nüchternen Tiere mit Pentobarbital (Nembutal, Sanofi, 50 mg/kg i.p.) narkotisiert und an der Bauchseite weiträumig rasiert und desinfiziert. Nach Eröffnung des Bauchraumes entlang der Linea alba wird der flüssigkeitsgefüllte Messkatheter des Systems oberhalb der Bifurcation nach cranial in die Aorta descendens eingesetzt und mit Gewebekleber (VetBonD™, 3M) befestigt. Das Sendergehäuse wird intraperitoneal an der Bauchwandmuskulatur fixiert und die Wunde schichtweise verschlossen. Postoperativ wird zur Infektionsprophylaxe ein Antibiotikum verabreicht (Tardomyocel COMP, Bayer, 1 ml/kg s.c.).

### Versuchsablauf:

Die zu untersuchenden Substanzen werden jeweils einer Gruppe von Tieren (n = 6) per Schlundsonde oral verabreicht. Entsprechend einem Applikationsvolumen von 5 ml/kg Körpergewicht werden die Testsubstanzen in geeigneten Lösungsmittelgemischen gelöst oder in 0.5%-iger Tylose suspendiert. Eine Lösungsmittel-behandelte Gruppe von Tieren wird als Kontrolle eingesetzt.

Die Telemetrie-Messeinrichtung ist für 24 Tiere konfiguriert. Jeder Versuch wird unter einer Versuchsnummer registriert.

Den in der Anlage lebenden instrumentierten Ratten ist jeweils eine eigene Empfangsantenne zugeordnet (1010 Receiver, DSI). Die implantierten Sender sind über einen eingebauten Magnetschalter von außen aktivierbar und werden bei Versuchsvorlauf auf Sendung geschaltet. Die ausgestrahlten Signale können durch ein Datenakquisitionssystem (Dataques™ A.R.T. for Windows, DSI) online erfasst und entsprechend aufgearbeitet werden. Die Ablage der Daten erfolgt jeweils in einem hierfür eröffneten Ordner, der die Versuchsnummer trägt.

Im Standardablauf werden über je 10 Sekunden Dauer gemessen: (*1*) systolischer Blutdruck (SBP), (*2*) diastolischer Blutdruck (DBP), (*3*) arterieller Mitteldruck (MAP) und (*4*) Herzfrequenz (HR).

Die Messwerterfassung wird rechnergesteuert in 5 Minuten-Abständen wiederholt. Die als Absolutwert erhobenen Quelldaten werden im Diagramm mit dem aktuell gemessenen Barometerdruck korrigiert und in Einzeldaten abgelegt. Weitere technische Details sind in der Dokumentation der Herstellerfirma (DSI) aufgeführt.

Die Verabreichung der Prüfsubstanzen erfolgt am Versuchstag um 9:00 Uhr. Im Anschluss an die Applikation werden die oben beschriebenen Parameter über 24 Stunden gemessen. Nach Versuchsende werden die erhobenen Einzeldaten mit der Analysis-Software (Dataquest™ A.R.T. Analysis) sortiert. Als Leerwert wird der Zeitpunkt 2 Stunden vor Substanz-Applikation angenommen, so dass der selektierte Datensatz den Zeitraum von 7:00 Uhr am Versuchstag bis 9:00 Uhr am Folgetag umfasst.

Die Daten werden über eine voreinstellbare Zeit durch Mittelwertbestimmung geglättet (15 Minuten-Mittelwert, 30 Minuten-Mittelwert) und als Textdatei auf einen Datenträger übertragen. Die so vorsortierten und komprimierten Messwerte werden in Excel-Vorlagen übertragen und tabellarisch dargestellt.

Die Verbindung aus Beispiel 6 zeigt in diesem Test nach oraler Gabe von 10 mg/kg eine deutliche Blutdrucksenkung über einen Zeitraum von 11 Stunden.

### C. Ausführunesbeispiele für pharmazeutisch Zusammensetzungen

Die erfindungsgemäßen Verbindungen können folgendermaßen in pharmazeutische Zubereitungen überführt werden:

### Tablette:

### Zusammensetzung:

100 mg der erfindungsgemäßen Verbindung, 50 mg Lactose (Monohydrat), 50 mg Maisstärke (nativ), 10 mg Polyvinylpyrrolidon (PVP 25) (Fa. BASF, Ludwigshafen, Deutschland) und 2 mg Magnesiumstearat.

Tablettengewicht 212 mg. Durchmesser 8 mm, Wölbungsradius 12 mm.

### Herstellung:

Die Mischung aus erfindungsgemäßer Verbindung, Lactose und Stärke wird mit einer 5%-igen Lösung (m/m) des PVPs in Wasser granuliert. Das Granulat wird nach dem Trocknen mit dem Magnesiumstearat 5 Minuten gemischt. Diese Mischung wird mit einer üblichen Tablettenpresse verpresst (Format der Tablette siehe oben). Als Richtwert für die Verpressung wird eine Presskraft von 15 kN verwendet.

### Oral applizierbare Suspension:

### Zusammensetzung:

1000 mg der erfindungsgemäßen Verbindung, 1000 mg Ethanol (96%), 400 mg Rhodigel^{®} (Xanthan gum der Firma FMC, Pennsylvania, USA) und 99 g Wasser.

Einer Einzeldosis von 100 mg der erfindungsgemäßen Verbindung entsprechen 10 ml orale Suspension.

### Herstellung:

Das Rhodigel wird in Ethanol suspendiert, die erfindungsgemäße Verbindung wird der Suspension zugefügt. Unter Rühren erfolgt die Zugabe des Wassers. Bis zum Abschluß der Quellung des Rhodigels wird ca. 6 h gerührt.

### Oral applizierbare Lösung:

### Zusammensetzung:

500 mg der erfindungsgemäßen Verbindung, 2.5 g Polysorbat und 97 g Polyethylenglycol 400. Einer Einzeldosis von 100 mg der erfindungsgemäßen Verbindung entsprechen 20 g orale Lösung.

### Herstellung:

Die erfindungsgemäße Verbindung wird in der Mischung aus Polyethylenglycol und Polysorbat unter Rühren suspendiert. Der Rührvorgang wird bis zur vollständigen Auflösung der erfindungsgemäßen Verbindung fortgesetzt.

### i.v. Lösung:

Die erfindungsgemäße Verbindung wird in einer Konzentration unterhalb der Sättigungslöslichkeit in einem physiologisch verträglichen Lösungsmittel (z.B. isotonische Kochsalzlösung, Glucoselösung 5% und/oder PEG 400-Lösung 30%) gelöst. Die Lösung wird steril filtriert und in sterile und pyrogenfreie Injektionsbehältnisse abgefüllt.

## Patentansprüche

1. Verbindung der Formel (I) 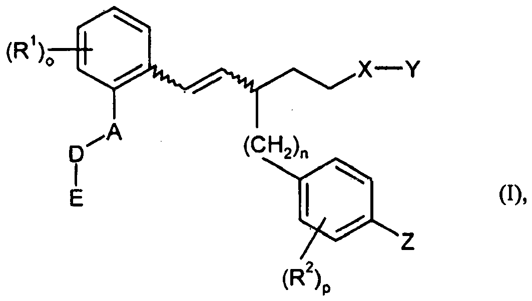 in welcher
A für O oder CH₂ steht,
D für eine Bindung oder für (C₁-C₇)-Alkandiyl, (C₂-C₇)-Alkendiyl oder (C₂-C₇)-Alkindiyl steht,
E für Wasserstoff, Trifluormethyl oder für eine Gruppe der Formel 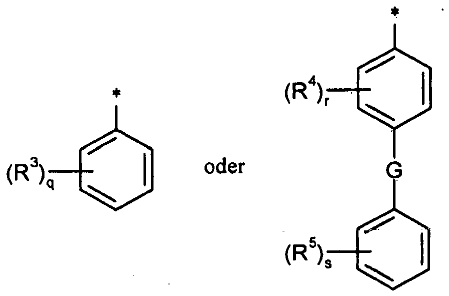
steht, worin * die Verknüpfungsstelle mit der Gruppe D und
G eine Bindung, CH₂, -CH₂-CH₂- oder -CH=CH- bedeutet,
X für -CH₂-CH₂- oder eine Gruppe der Formel 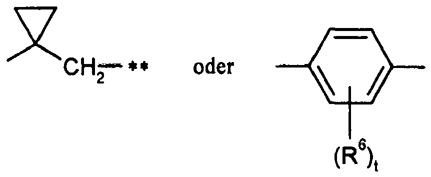 steht, worin ** die Verknüpfungsstelle mit der Gruppe Y bedeutet,
Y für Carboxyl
und
Z für eine Gruppe der Formel 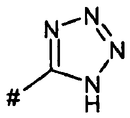
oder
Y für eine Gruppe der Formel 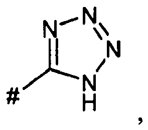
worin # jeweils die Verknüpfungsstelle bedeutet,
und
Z für Carboxyl stehen,
n für die Zahl 1 oder 2 steht,
R¹, R², R³, R⁴, R⁵ und R⁶ unabhängig voneinander für einen Substituenten ausgewählt aus der Reihe Halogen, (C₁-C₆)-Alkyl, Trifluormethyl, (C₁-C₆)-Alkoxy, Trifluormethoxy, Cyano und Nitro stehen,
und
o, p, q, r, s und t unabhängig voneinander jeweils für die Zahl 0, 1, 2, 3 oder 4 stehen,
wobei für den Fall, dass R¹, R², R³, R⁴, R⁵ oder R⁶ mehrfach auftreten, ihre Bedeutungen jeweils gleich oder verschieden sein können,
sowie ihre Salze, Solvate und Solvate der Salze.

2. Verbindung der Formel (I) nach Anspruch 1, in welcher
A für O steht,
D für (C₁-C₇)-Alkandiyl steht,
E für Wasserstoff, Trifluormethyl oder für eine Gruppe der Formel 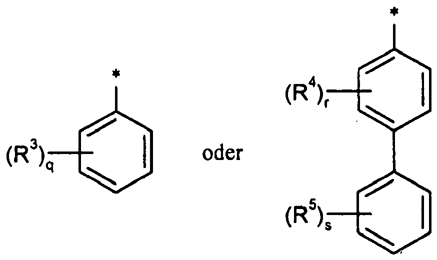
steht, worin * die Verknüpfungsstelle mit der Gruppe D bedeutet,
X für -CH₂-CH₂- oder eine Gruppe der Formel 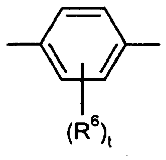
steht,
Y für Carboxyl
und
Z für eine Gruppe der Formel 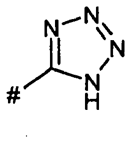
oder
Y für eine Gruppe der Formel 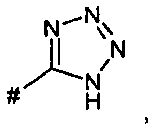
worin # jeweils die verknüpfungsstelle bedeutet,
und
Z für Carboxyl stehen,
n für die Zahl 1 oder 2 steht,
R¹, R³, R⁴ und R⁵ unabhängig voneinander für einen Substituenten ausgewählt aus der Reihe Fluor, Chlor, Brom, (C₁-C₄)-Alkyl, Trifluormethyl, (C₁-C₄)-Alkoxy und Trifluormethoxy stehen,
o, q, r und s unabhängig voneinander jeweils für die Zahl 0, 1 oder 2 stehen, wobei für den Fall, dass R¹, R³, R⁴ oder R⁵ mehrfach auftreten, ihre Bedeutungen jeweils gleich oder verschieden sein können,
R² und R⁶ jeweils für Fluor stehen
und
p und t unabhängig voneinander jeweils für die Zahl 0 oder 1 stehen,
sowie ihre Salze, Solvate und Solvate der Salze.

3. Verbindung nach Anspruch 1 der Formel (I-A) 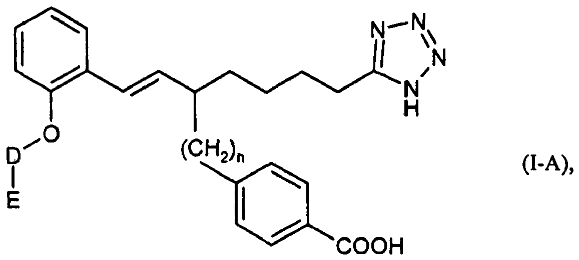 in welcher
D für (C₁-C₇)-Alkandiyl,
E für Wasserstoff oder eine Gruppe der Formel 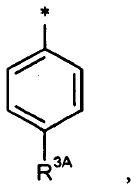
worin * die Verknüpfungsstelle mit der Gruppe D und
R^{3A} Wasserstoff, Fluor, Chlor, Methyl, *tert*.-Butyl, Trifluormethyl, Methoxy oder Trifluormethoxy bedeuten,
und
n für die Zahl 1 oder 2
stehen,
sowie ihre Salze, Solvate und Solvate der Salze.

4. Verfahren zur Herstellung einer Verbindung der Formel (I) bzw. (I-A), wie in den Ansprüchen 1 bis 3 definiert, **dadurch gekennzeichnet, dass** man entweder
[A] Verbindungen der Formel (II-1) 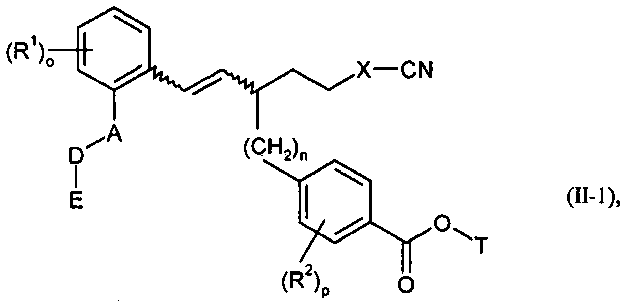 in welcher R¹, R², A, D, E, X, n, o und p jeweils die in den Ansprüchen 1 bis 3 angegebenen Bedeutungen haben und
T für (C₁-C₄)-Alkyl steht,
in einem inerten Lösungsmittel mit einem Alkali-Azid oder mit Trimethylsilylazid zu Verbindungen der Formel (III-1) 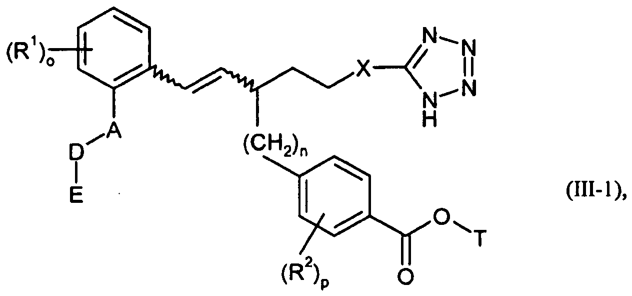 in welcher R¹, R², A, D, E, X, n, o, p und T jeweils die oben angegebenen Bedeutungen haben,
oder
[B] Verbindungen der Formel (II-2) 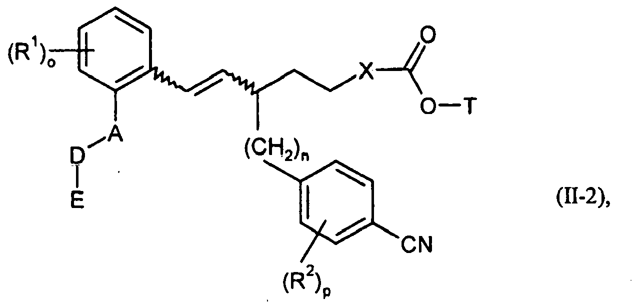 in welcher R¹, R², A, D, E, X, n, o, p und T jeweils die oben angegebenen Bedeutungen haben,
in einem inerten Lösungsmittel mit einem Alkali-Azid oder mit Trimethylsilylazid zu Verbindungen der Formel (III-2) 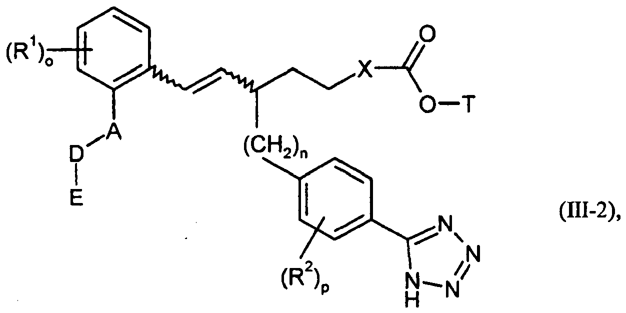 in welcher R¹, R², A, D, E, X, n, o, p und T jeweils die oben angegebenen Bedeutungen haben,
umsetzt
und die resultierenden Verbindungen der Formel (III-1) bzw. (III-2) durch Hydrolyse der Ester-Gruppierung -C(O)OT in die entsprechenden Carbonsäuren der Formel (I) überführt
und die Verbindungen der Formel (I) gegebenenfalls mit den entsprechenden (i) Lösungsmitteln und/oder (ii) Basen oder Säuren zu ihren Solvaten, Salzen und/oder Solvaten der Salze umsetzt.

5. Verbindung, wie in einem der Ansprüche 1 bis 3 definiert, zur Behandlung und/oder Prävention von Krankheiten.

6. Verwendung einer Verbindung, wie in einem der Ansprüche 1 bis 3 definiert, zur Herstellung eines Arzneimittels zur Behandlung und/oder Prävention von Herzinsuffizienz, Angina pectoris, Hypertonie, pulmonaler Hypertonie, Ischämien, Gefäßerkrankungen, thromboembolischen Erkrankungen und Arteriosklerose.

7. Arzneimittel enthaltend eine Verbindung, wie in einem der Ansprüche 1 bis 3 definiert, in Kombination mit einem inerten, nicht-toxischen, pharmazeutisch geeigneten Hilfsstoff.

8. Arzneimittel enthaltend eine Verbindung, wie in einem der Ansprüche 1 bis 3 definiert, in Kombination mit einem weiteren Wirkstoff ausgewählt aus der Gruppe bestehend aus organischen Nitraten, NO-Donatoren, cGMP-PDE-Inhibitoren, Stimulatoren der Guanylatcyclase, antithrombotisch wirkenden Mitteln, den Blutdruck senkenden Mitteln sowie den Fettstoffwechsel verändernden Mitteln.

9. Arzneimittel nach Anspruch 7 oder 8 zur Behandlung und/oder Prävention von Herzinsuffizienz, Angina pectoris, Hypertonie, pulmonaler Hypertonie, Ischämien, Gefäßerkrankungen, thromboembolischen Erkrankungen und Arteriosklerose.

## Claims

1. A compound of the formula (I) 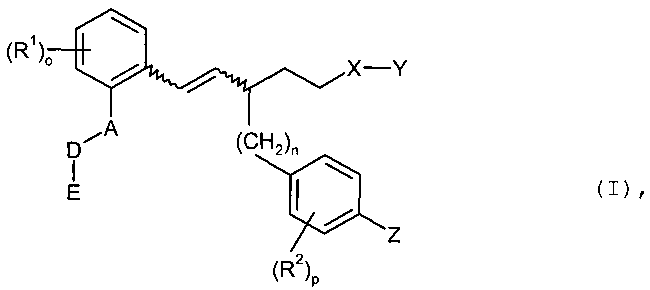 in which
A is O or CH₂,
D is a bond or is (C₁-C₇) -alkanediyl, (C₂-C₇)-alkenediyl or (C₂-C₇)-alkynediyl,
E is hydrogen, trifluoromethyl or a group of the formula 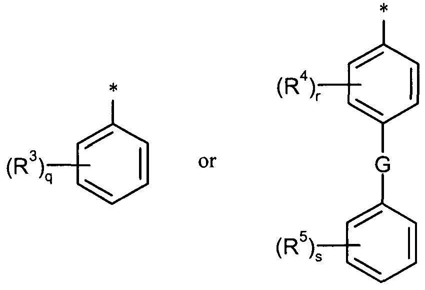
in which * means the point of linkage to the group D and
G is a bond, CH₂, -CH₂-CH₂- or -CH=CH-,
X is -CH₂-CH₂- or a group of the formula 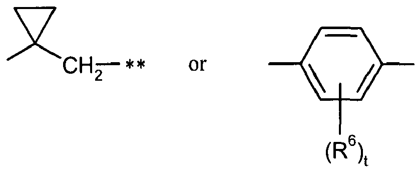
in which ** means the point of linkage to the group Y,
Y is carboxyl
and
Z is a group of the formula 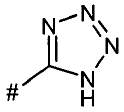
or
Y is a group of the formula 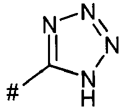
in which # means the respective point of linkage,
and
Z is carboxyl,
n is the number 1 or 2,
R¹, R², R³, R⁴, R⁵ and R⁶ are independently of one another a substituent selected from the series halogen, (C₁-C₆)-alkyl, trifluoromethyl, (C₁-C₆) -alkoxy, trifluoromethoxy, cyano and nitro,
and
o, p, q, r, s and t are independently of one
another each the number 0, 1, 2, 3 or 4, where in the case where R¹, R², R³, R⁴, R⁵ or R⁶ occur more than once, their meanings may in each case be identical or different,
and the salts, solvates and solvates of the salts thereof.

2. The compound of the formula (I) as claimed in claim 1, in which
A is O,
D is (C₁-C₇)-alkanediyl,
E is hydrogen, trifluoromethyl or is a group of the formula 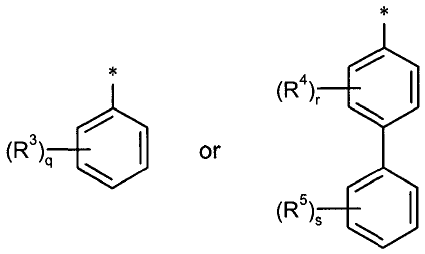
in which * means the point of linkage to the group D,
X is -CH₂-CH₂- or a group of the formula 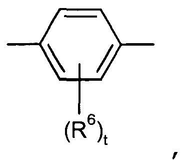
Y is carboxyl
and
Z is a group of the formula 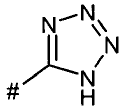
or
Y is a group of the formula 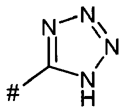
in which # means the respective point of linkage,
and
Z is carboxyl,
n is the number 1 or 2,
R¹, R³, R⁴ and R⁵ are independently of one another a substituent selected from the series fluorine, chlorine, bromine, (C₁-C₄)-alkyl, trifluoromethyl, (C₁-C₄)-alkoxy and trifluoromethoxy,
o, q, r and s are independently of one another each the number 0, 1 or 2, where in the case where R¹, R³, R⁴ or R⁵ occur more than once, their meanings may in each case be identical or different,
R² and R⁶ are each fluorine,
and
p and t are independently of one another each the number 0 or 1,
and the salts, solvates and solvates of the salts thereof.

3. The compound as claimed in claim 1 of the formula (I-A) 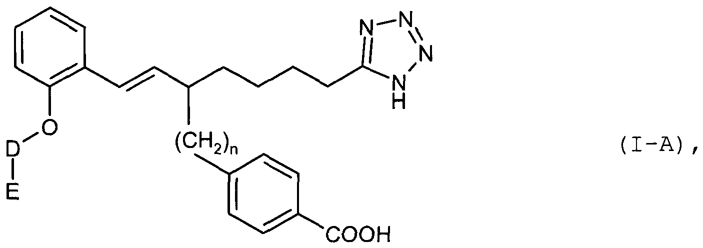 in which
D is (C₁-C₇)-alkanediyl,
E is hydrogen or a group of the formula 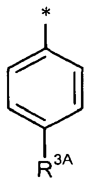
in which * means the point of linkage to the group D, and
R^{3A} is hydrogen, fluorine, chlorine, methyl, tert-butyl, trifluoromethyl, methoxy or trifluoromethoxy,
and
n is the number 1 or 2,
and the salts, solvates and solvates of the salts thereof.

4. A process for preparing a compound of the formula (I) or (I-A) as defined in claims 1 to 3, **characterized in that** either
[A] compounds of the formula (II-1) 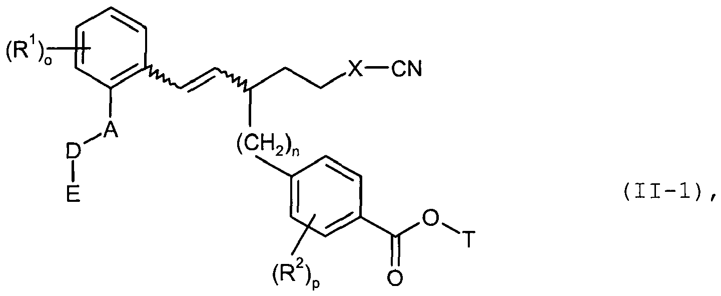 in which R¹, R², A, D, E, X, n, o and p each have the meanings indicated in claims 1 to 3,
and
T is (C₁-C₄) -alkyl,
are reacted with an alkali metal azide or with trimethylsilyl azide in an inert solvent to give compounds of the formula (III-1) 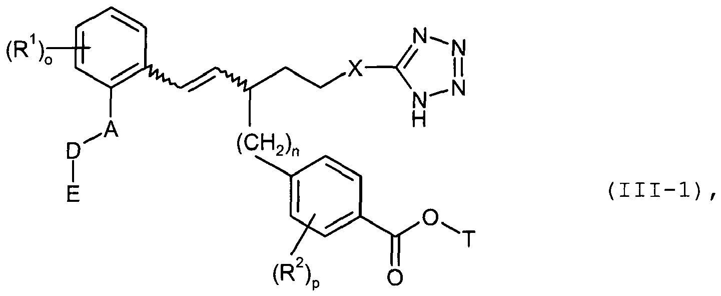 in which R¹, R², A, D, E, X, n, o, p and T each have the meanings indicated above,
or
[B] compounds of the formula (II-2) 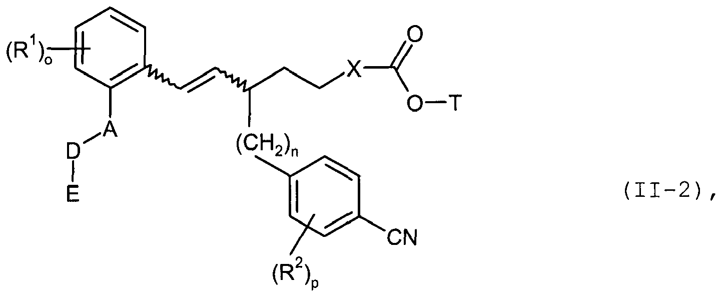 in which R¹, R², A, D, E, X, n, o, p and T each have the meanings indicated above,
are reacted with an alkali metal azide or with trimethylsilyl azide in an inert solvent to give compounds of the formula (III-2) 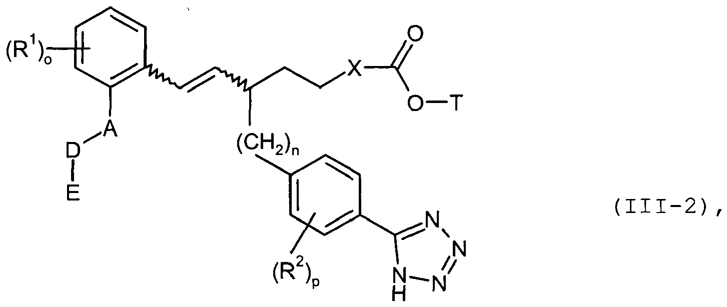 in which R¹, R² ,A, D, E, X, n, o, p and T each have the meanings indicated above,
and the resulting compounds of the formula (III-1) or (III-2) are converted by hydrolysis of the ester group -C(O)OT into the corresponding carboxylic acids of the formula (I),
and the compounds of the formula (I) are reacted where appropriate with the appropriate (i) solvents and/or (ii) bases or acids to give the solvates, salts and/or solvates of the salts thereof.

5. The compound as defined in any of claims 1 to 3 for the treatment and/or prevention of diseases.

6. The use of a compound as defined in any of claims 1 to 3 for producing a medicament for the treatment and/or prevention of heart failure, angina pectoris, hypertension, pulmonary hypertension, ischemias, vascular disorders, thromboembolic disorders and arteriosclerosis.

7. A medicament comprising a compound as defined in any of claims 1 to 3 in combination with an inert, non-toxic, pharmaceutically suitable excipient.

8. A medicament comprising a compound as defined in any of claims 1 to 3 in combination with a further active ingredient selected from the group consisting of organic nitrates, NO donors, cGMP-PDE inhibitors, stimulators of guanylate cyclase, agents having antithrombotic activity, agents lowering blood pressure, and agents altering lipid metabolism.

9. The medicament as claimed in claim 7 or 8 for the treatment and/or prevention of heart failure, angina pectoris, hypertension, pulmonary hypertension, ischemias, vascular disorders, thromboembolic disorders and arteriosclerosis.

## Revendications

1. Composé de formule (I) 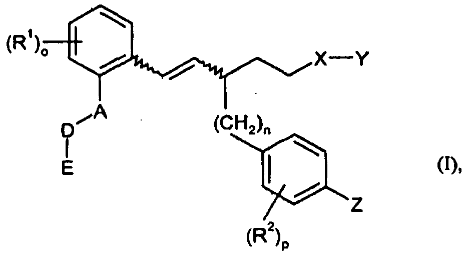 dans laquelle
A représente O ou CH₂,
D représente une liaison ou (C₁-C₇)-alcanediyle, (C₂-C₇)-alcènediyle ou (C₂-C₇)-alcynediyle,
E représente hydrogène, trifluorométhyle ou un groupe de formule 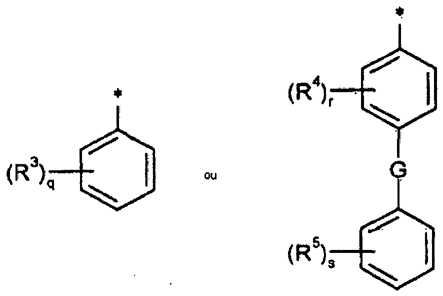
* signifie le site de liaison avec le groupe D et
G signifie une liaison, CH₂, -CH₂-CH₂- ou -CH=CH-,
X représente--CH₂-CH₂- ou un groupe de formule 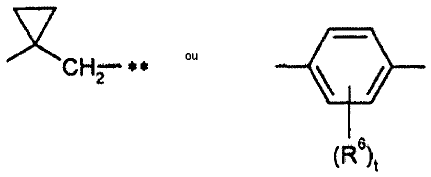
dans laquelle ** signifie le site de liaison avec le groupe Y,
Y représente carboxyle et
Z représente un groupe de formule 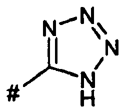
ou
Y représente un groupe de formule 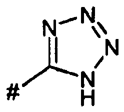
dans laquelle # représente à chaque fois le site de liaison,
et
Z représente carboxyle,
n vaut le nombre 1 ou 2,
R¹, R², R³, R⁴, R⁵ et R⁶ représentent, indépendamment les uns des autres, un substituant choisi dans la série halogène, (C₁-C₆)-alkyle, trifluorométhyle, (C₁-C₆)-alcoxy, trifluorométhoxy, cyano et nitro, et
o, p, q, r, s et t représentent, indépendamment les uns des autres, à chaque fois le nombre 0, 1, 2, 3 ou 4, où, pour le cas où R^{1,} R², R³, R⁴_{,} R⁵ ou R⁶ apparaissent plusieurs fois, leurs significations peuvent à chaque fois être identiques ou différentes,
ainsi que ses sels, solvates et les solvates des sels.

2. Composé de formule (I) selon la revendication 1, dans laquelle
A représente O,
D représente (C₁-C₇) -alcanediyle,
E représente hydrogène, trifluorométhyle ou un groupe de formule 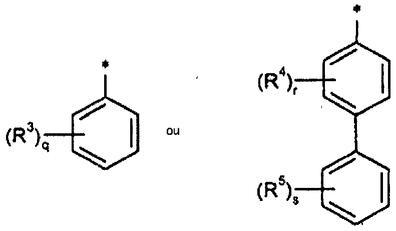
dans laquelle * signifie le site de liaison avec le groupe D,
X représente--CH₂-CH₂- ou un groupe de formule 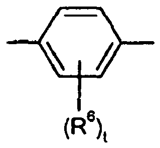
Y représente carboxyle et
Z représente un groupe de formule 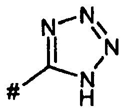
ou
Y représente un groupe de formule 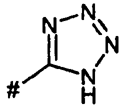
dans laquelle # représente à chaque fois le site de liaison,
et
Z représente carboxyle,
n vaut le nombre 1 ou 2,
R¹, R³, R⁴ et R⁵ représentent, indépendamment les uns des autres, un substituant choisi dans la série fluor, chlore, brome, (C₁-C₄)-alkyle, trifluorométhyle, (C₁-C₄)-alcoxy et trifluorométhoxy,
o, q, r et s représentent, indépendamment les uns des autres, à chaque fois le nombre 0, 1, ou 2, où, pour le cas où R¹, R², R³, R⁴ ou R⁵ apparaissent plusieurs fois, leurs significations peuvent à chaque fois être identiques ou différentes,
R² et R⁶ représentent à chaque fois fluor, et
p et t représentent, indépendamment l'un de l'autre, à chaque fois le nombre 0 ou 1,
ainsi que ses sels, solvates et les solvates des sels.

3. Composé selon la revendication 1 de formule (I-A) 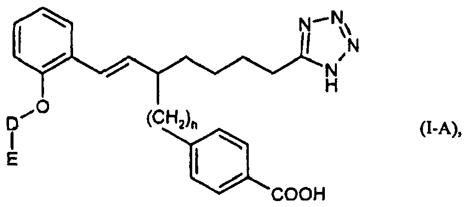 dans laquelle
D représente (C₁-C₇)-alcanediyle,
E représente hydrogène ou un groupe de formule 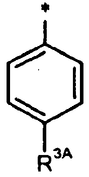
dans laquelle * signifie le site de liaison avec le groupe D et
R^{3A} signifie hydrogène, fluor, chlore, méthyle, tert-butyle, trifluorométhyle, méthoxy ou trifluorométhoxy,
et
n représente le nombre 1 ou 2,
ainsi que ses sels, solvates et les solvates des sels.

4. Procédé pour la préparation d'un composé de formule (I) ou (I-A), tel que défini dans les revendications 1 à 3, **caractérisé en ce qu'**on transforme soit
[A] des composés de formule (II-1) 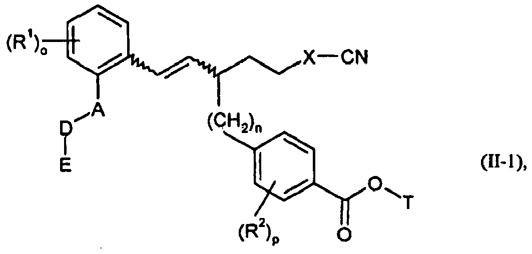 dans laquelle R¹, R², A, D, E, X, n, o et p présentent à chaque fois les significations indiquées dans les revendications 1 à 3,
T représente (C₁-C₄) -alkyle,
dans un solvant inerte avec un azide de métal alcalin ou avec un azide de triméthylsilyle en composés de formule (III-1) 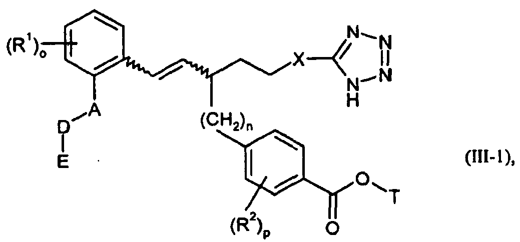 dans laquelle R¹, R², A, D, E, X, n, o, p et T présentent à chaque fois les significations indiquées ci-dessus, soit
[B] des composés de formule (II-2) 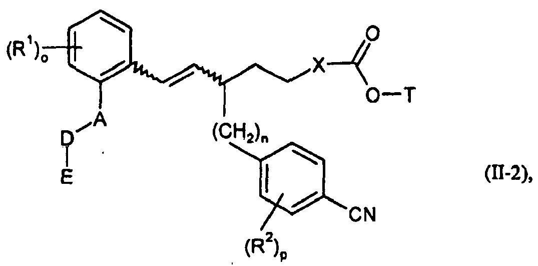 dans laquelle R¹, R², A, D, E, X, n, o, p et T présentent à chaque fois les significations indiquées ci-dessus,
dans un solvant inerte avec un azide de métal alcalin ou avec un azide de triméthylsilyle en composés de formule (III-2) 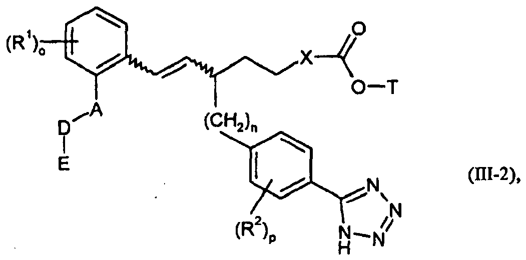 dans laquelle R¹, R², A, D, E, X, n, o, p et T présentent à chaque fois les significations indiquées ci-dessus,
et on transforme les composés résultants respectivement de formule (III-1) ou (III-2) par hydrolyse du groupement ester -C(O)OT en acides carboxyliques correspondants de formule (I)
et on transforme les composés de formule (I) le cas échéant avec (i) les solvants et/ou (ii) les bases ou acides correspondants en leurs solvates, sels et/ou les solvates des sels.

5. Composé, tel que défini dans l'une quelconque des revendications 1 à 3, destiné au traitement et/ou à la prévention de maladies.

6. Utilisation d'un composé tel que défini dans l'une quelconque des revendications 1 à 3 pour la préparation d'un médicament destiné au traitement et/ou à la prévention de l'insuffisance cardiaque, de l'angine de poitrine, de l'hypertonie, de l'hypertonie pulmonaire, des ischémies, des maladies vasculaires, des maladies thromboemboliques et de l'artériosclérose.

7. Médicament contenant un composé, tel que défini dans l'une quelconque des revendications 1 à 3, en combinaison avec un adjuvant inerte, non toxique, pharmaceutiquement approprié.

8. Médicament contenant un composé tel que défini dans l'une quelconque des revendications 1 à 3, en combinaison avec une autre substance active choisie dans le groupe constitué par les nitrates organiques, les donneurs de NO, les inhibiteurs de cGMP-PDE, les stimulateurs de la guanylate-cyclase, les agents à effet antithrombotique, les agents hypotenseurs ainsi que les agents modifiant le métabolisme des lipides.

9. Médicament selon la revendication 7 ou 8, destiné au traitement et/ou à la prévention de l'insuffisance cardiaque, de l'angine de poitrine, de l'hypertonie, de l'hypertonie pulmonaire, des ischémies, des maladies vasculaires, des maladies thromboemboliques et de l'artériosclérose.
